# EUROPÄISCHE PATENTSCHRIFT

(11) **EP 0 520 371 B1**
(45) Veröffentlichungstag und Bekanntmachung des Hinweises auf die Patenterteilung: **09.04.1997**
(21) Anmeldenummer: 92110562.3
(22) Anmeldetag: 23.06.1992
(51) Int. Cl.: A01N 25/32, C07D 261/04, C07D 275/03, C07D 275/02

(54) **Neue Isoxazoline und Isothiazoline, sie enthaltende pflanzenschützende Mittel sowie ein Nachweisverfahren zur Identifizieurung potentieller pflanzenschützender Mittel**
Isoxazolines and isothiazolines, plant-protecting agent containing them and method of analysis for the identification of potential plant-protecting agents
Isoxazolines et isothiazolines, agents phytoprotecteurs les contenant et méthode d'analyse pour l'identification d'agents phytoprotecteurs potentiels

(30) Priorität: 25.06.1991 DE 4120964
(43) Veröffentlichungstag der Anmeldung: 30.12.1992
(73) Patentinhaber: Hoechst Schering AgrEvo GmbH, 13342 Berlin (DE)
(72) Erfinder: Löher, Heinz-Josef, Dr., W-6237 Liederbach (DE); Trinks, K., Dr., W-6093 Flörsheim am Main (DE)

(56) Entgegenhaltungen:
- WO-A-91/08202
- US-A- 4 889 551
- BULLETIN OF THE CHEMICAL SOCIETY OF JAPAN Bd. 59, Nr. 9, September 1986, Tokyo, JP, Seiten 2827-2831, T. SHIMIZU et al.
- CHEMICAL ABSTRACTS, vol. 115, no. 15, 14. Oktober 1991, Columbus, Ohio, US, abstract no. 159012
- CHEMICAL ABSTRACTS, vol. 112, no. 13, 26. März 1990, Columbus, Ohio, US, abstract no. 118694p
- SYNTHESIS Nr. 6, Juni 1986, Stuttgart, DE, Seiten 488-490, T. SHIMIZU et al.
- BULLETIN OF THE CHEMICAL SOCIETY OF JAPAN Bd. 57, Nr. 9, September 1984, Tokyo, JP, Seiten 2531-2534, T. SHIMIZU et al.
- CHEMICAL ABSTRACTS, vol. 67, no. 21, 20. November 1967, Columbus, Ohio, US, abstract no. 100039w
- CHEMICAL ABSTRACTS, vol. 82, no. 7, 17. Februar 1975, Columbus, Ohio, US, abstract no. 43227q
- ANGEWANDTE CHEMIE, Intern. Ed., Bd. 18, Nr. 1, 1979, Weinheim, DE, Seiten 78-80, V. JÄGER et al.

## Beschreibung

Der Einsatz von Herbiziden kann dazu führen, daß auch an Kulturpflanzen Schäden auftreten. Als "Antidot" oder "Safener" werden solche Substanzen bezeichnet, die eine durch Herbizide verursachte Schädigung von Kulturpflanzen verhindern, ohne gleichzeitig die Wirkung des Herbizids auf Schadpflanzen zu beeinträchtigen.

Einige dieser Verbindungen wurden für diese Anwendung bereits beschrieben (vgl. z.B. EP-A-152 006 (US-A-4,668,276) oder EP-A-174 56 (US-A-4,639,266)). Aus WO 91/08202 sind pflanzenschützende Isoxazolin-3-carbonsäurederivate bekannt, welche in 5-Stellung einen Benzylrest aufweisen. US-A-4889551 beschreibt 3-Phenyl-isoxazolin-5-carbonsäurederivate als Pflanzenwachtumsregulatoren. Die Anwendung bestimmter Isoxazoline und Isothiazoline als Safener wurde in der deutschen Patentanmeldung P 40 26 018.6 (ZA 91/6500) vorgeschlagen. Weitere Isoxazolincarbonsäuren sind als Zwischenprodukte der interessante chemische Syntheseziele ohne Hinweis auf eine Safenerwirkung beschrieben (vgl. Literaturstellen auf Seiten 7 und 8).

Die Erfindung betrifft kulturpflanzenschützende Mittel, welche Isoxazoline oder Isothiazoline der allgemeinen Formel I oder deren Salze, 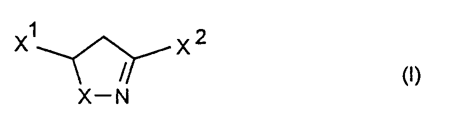 in welcher entweder
X¹ = R¹ und X² = CO-R bedeutet oder
X¹ = CO-R und X² = R¹ bedeutet,
worin
- X: ein Sauerstoff- oder Schwefelatom, insbesondere ein Sauerstoffatom bedeutet, und
- R: a) Hydroxy oder Mercapto,
b) Alkoxy, Alkenyloxy, Alkinyloxy, Alkylthio, Alkenylthio, Alkinylthio, Cycloalkyloxy oder Cycloalkylthio bedeutet, das jeweils unsubstituiert oder ein- oder mehrfach durch Reste aus der Gruppe enthaltend Phenyl, Alkoxy, Alkenyloxy, Alkinyloxy, Benzyloxy, Phenyloxy, Cycloalkyloxy, Alkylthio, Mono- und Dialkylamino, Cyano, Halogen und NO₂ substituiert ist,
c) Benzyloxy, Phenyloxy, Benzylthio oder Phenylthio, das jeweils unsubstituiert oder ein- oder mehrfach durch Reste aus der Gruppe enthaltend Alkyl, Alkenyl, Alkinyl, Halogen, Cyano, Nitro, Alkoxy, Alkenyloxy, Alkinyloxy, Alkylthio, Mono- und Dialkylamino, Phenyloxy und Benzyloxy substituiert ist,
d) Trialkylsilylalkoxy, Aryldialkylsilyloxy, Aralkyldialkylsilyloxy, Diarylalkylsilyloxy oder Diaralkylalkylsilyloxy bedeutet,
e) einen Rest der Formel NR'R' bedeutet, worin die Reste R' für gleiche oder verschiedene Reste aus der Gruppe Alkyl, Alkenyl, Alkinyl und Cycloalkyl stehen,
f) Pyridino, Morpholino, Alkylmorpholino, Dialkylmorpholino, Hydrazino, Alkylhydrazino oder Dialkylhydrazino bedeutet,
g) einen Rest der Formel 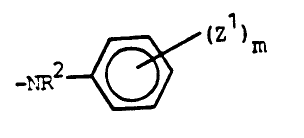 bedeutet, worin R² Wasserstoff, Alkyl, Alkenyl oder Alkinyl und die Reste Z¹ unabhängig voneinander Halogen, Nitro, Alkyl, Alkenyl, Alkoxy oder Phenoxy und m eine ganze Zahl von 0 bis 5 sind,
h) einen Rest der Formel 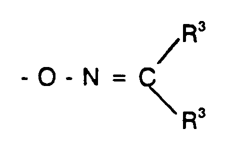 bedeutet, worin die Reste R³ jeweils unabhängig voneinander Alkyl oder gemeinsam mit dem sie verknüpfenden C-Atom Cycloalkyl bedeuten,
i) einen Rest der Formel

   - O - CR⁴R⁴ - CO - R⁵

   bedeutet, worin die R⁴ unabhängig voneinander Wasserstoff, Alkyl, Alkenyl, Alkinyl, Aryl, Benzyl, Alkoxy, Alkenyloxy, Alkinyloxy oder Phenoxy bedeuten und R⁵ Wasserstoff, Alkyl, Alkenyl, Alkinyl, Aryl oder Benzyl bedeutet,
k) einen Rest der Formel 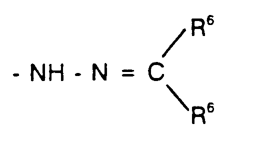 worin die R⁶ unabhängig voneinander Alkyl, Wasserstoff oder Aryl oder gemeinsam mit dem sie verknüpfenden C-Atom Cycloalkyl bedeuten, oder
l) einen Rest der Formel

   - O - CR⁷R⁷ - CO - R⁸

   bedeutet, worin die Reste R⁷ unabhängig voneinander Wasserstoff, Alkyl, Alkenyl, Alkinyl, Aryl, Benzyl, Alkoxy, Alkenyloxy, Alkinyloxy oder Phenoxy bedeuten und R⁸ die vorstehend unter a) bis k) für R angegebene Bedeutung hat, und
- R¹ =: Wasserstoff, Alkyl, Alkenyl, Alkinyl, Cycloalkyl, Cycloalkenyl, wobei die 4 letztgenannten Gruppen unsubstituiert oder ein- oder mehrfach durch Reste aus der Gruppe enthaltend Phenyl, Hydroxy, Alkoxy, Alkenyloxy, Alkinyloxy, Benzyloxy, Phenyloxy, Cycloalkyloxy, Alkylthio, Mono- und Dialkylamino, Cyano, Halogen und Nitro substitutiert sind,
bedeuten,
und übliche Formulierungshilfsmittel enthalten.

Gegenstand der Erfindung sind außerdem selektive herbizide Mittel, die einen Wirkstoff der genannten Formel (I) oder deren Salze in Kombination mit einem Herbizid und gegebenenfalls übliche Formulierungshilfsmittel enthalten.

In der Formel (I) können Alkyl-, Alkoxy- und Alkylthioreste sowie die entsprechenden ungesättigten Reste jeweils geradkettig oder verzweigt sein. Sie haben vorzugsweise bis zu fünf C-Atome. Entsprechendes gilt für die davon abgeleiteten Reste, wie Alkoxy, Alkylmercapto, Alkylamino, Dialkylamino und die entsprechenden ungesättigten Reste. Cycloalkylreste und davon abgeleitete Reste wie Cycloalkyloxy oder Cycloalkylmercapto haben vorzugsweise 3 bis 10 C-Atome und können mono-, bi- oder tricyclisch sein, wie z.B. Cyclopentyl, Cyclohexyl, Cycloheptyl, Decahydronaphtyl und Adamantyl.

Halogen bedeutet Fluor, Chlor, Brom oder Iod, vorzugsweise Fluor, Chlor oder Brom, insbesondere Fluor oder Chlor.

Arylreste haben vorzugsweise 6 bis 12 C-Atome; bevorzugt sind Phenyl, Naphthyl und Biphenylyl, insbesondere Phenyl. Entsprechendes gilt für davon abgeleitete Reste wie Aryloxy, Arylmercapto, Aroyl, Aralkyl, Aralkyloxy und Aralkylmercapto. Aralkyl ist vorzugsweise Benzyl.

Im Falle R = OH können die Verbindungen der Formel (I) Salze bilden. Erfindungsgemäß einsetzen lassen sich die in der Landwirtschaft verwendbaren Salze. Als solche kommen beispielsweise Metallsalze wie Alkali- oder Erdalkalisalze, insbesondere Natrium- oder Kaliumsalze, Salze mit Ammonium, Mono-, Di-, Tri-oder Tetra-(C₁-C₄)alkylammonium oder mit Mono-, Di-, Tri- oder Tetra-(C₁-C₄)alkanolammonium in Frage.

Gegenstand der Erfindung sind insbesondere auch alle Stereoisomeren und deren Gemische, die von der Formel (I) umfaßt, jedoch nicht spezifisch definiert sind. Stereoisomere können vor allem auftreten, wenn ein oder mehrere asymmetrische C-Atome und/oder geeignet substituierte Doppelbindungen in den Verbindungen der Formel (I) vorhanden sind. Die Stereoisomere lassen sich aus racemischen Gemischen nach üblichen Trennmethoden erhalten. Alternativ können Stereoisomere durch Einsatz stereoselektiver Reaktionen unter Verwendung optisch aktiver Ausgangsstoffe selektiv hergestellt werden.

Von besonderem Interesse sind erfindungsgemäße pflanzenschützende oder selektive herbizide Mittel, die eine Verbindung der genannten Formel (I) oder deren Salze enthalten, worin
- X: ein Sauerstoffatom bedeutet,
- R: a) Hydroxy oder Mercapto bedeutet,
b) (C₁-C₁₀)-Alkoxy, (C₂-C₄)-Alkenyloxy, (C₂-C₄)-Alkinyloxy, (C₁-C₄)-Alkylthio, (C₂-C₄)-Alkenylthio, (C₂-C₄)-Alkinylthio, (C₃-C₈)-Cycloalkoxy oder (C₃-C₈)-Cycloalkylthio bedeutet, das jeweils unsubstituiert oder ein- oder mehrfach durch Reste aus der Gruppe Phenyl, (C₁-C₄)-Alkoxy, (C₂-C₄)-Alkenyloxy, (C₂-C₄)-Alkinyloxy, Benzyloxy, Phenyloxy, (C₃-C₈)-Cycloalkyloxy, (C₁-C₄)-Alkylthio, Mono- und Di-(C₁-C₄)-Alkylamino, Cyano, Halogen und NO₂ substituiert ist,
c) Phenyloxy, Phenylthio, Benzyloxy oder Benzylthio bedeutet, das jeweils unsubstituiert oder durch 1 bis 3 Reste aus der Gruppe (C₁-C₄)-Alkyl, (C₂-C₄)-Alkenyl, (C₂-C₄)-Alkinyl, Halogen, Cyano, NO₂, (C₁-C₄)-Alkoxy, (C₂-C₄)-Alkenyloxy, (C₂-C₄)-Alkinyloxy, (C₁-C₄)-Alkylthio, Mono- und Di-(C₁-C₄)-Alkylamino, Phenyloxy und Benzyloxy substituiert ist,
d) einen Rest der Formel -NR'R', worin R' (C₁-C₄)-Alkyl bedeutet,
e) Pyridino, Morpholino, Methylmorpholino, Dimethylmorpholino, Hydrazino, Methylhydrazino oder Dimethylhydrazino bedeutet,
f) einen Rest der Formel 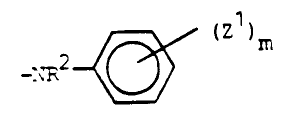 bedeutet, worin R², H oder (C₁-C₄)-Alkyl, die Reste Z¹ unabhängig voneinander Halogen, Nitro, (C₁-C₄)-Alkyl, (C₁-C₄)-Alkoxy oder Phenoxy und m 0 bis 3 bedeuten,
g) einen Rest der Formel -O-N=CR³R³ bedeutet, worin die R³ (C₁-C₄)-Alkyl, insbesondere Methyl, oder gemeinsam mit dem sie verknüpfenden C-Atom Cyclohexyl oder Cyclopentyl bedeuten,
h) einen Rest der Formel -O-CR⁴R⁴-CO-R⁵ bedeutet, worin die R⁴ unabhängig voneinander für H, (C₁-C₄)-Alkyl, (C₂-C₄)-Alkenyl, (C₂-C₄)-Alkinyl, Phenyl, Benzyl oder (C₁-C₄)-Alkoxy stehen und R⁵ für (C₁-C₄)-Alkyl, (C₂-C₄)-Alkenyl, (C₂-C₄)-Alkinyl, Phenyl oder Benzyl steht,
i) einen Rest der Formel -NH-N=CR⁶R⁶ bedeutet, worin die Reste R⁶ unabhängig voneinander H, (C₁-C₄)-Alkyl oder Phenyl oder gemeinsam mit dem sie verknüpfenden C-Atom Cyclohexyl oder Cyclopentyl bedeuten, oder
k) einen Rest der Formel -O-CR⁷R⁷-CO-R⁸ bedeutet, worin die Reste R⁷ unabhängig voneinander für H, (C₁-C₄)-Alkyl, (C₂-C₄)-Alkenyl, (C₂-C₄)-Alkinyl, Phenyl, Benzyl oder (C₁-C₄)-Alkoxy stehen und R⁸ die vorstehend unter a) bis i) für R genannten Bedeutung hat, und
- R¹: (C₁-C₁₈)-Alkyl, (C₁-C₁₈)-Alkenyl, (C₁-C₁₈)-Alkinyl, Cycloalkyl oder Cycloalkenyl bedeutet, wobei die 4 letztgenannten Gruppen unsubstituiert oder ein- oder mehrfach durch Reste aus der Gruppe enthaltend Phenyl, (C₁-C₄)-Alkoxy, (C₁-C₄)-Alkenyloxy, (C₁-C₄)-Alkyl, (C₁-C₄)-Alkinyloxy, Benzyloxy, Phenyloxy, Cycloalkyloxy, Alkylthio, Mono- und Dialkylamino, Cyano, Halogen und NO₂ substituiert sind.

Aus der Literatur sind Verbindungen der Formel (I) worin X¹= R¹ und X²= COR mit
- R =: OC₂H₅ und R¹ = n-C₆H₁₃ [Tetrahedron 43, 3983 (1987)] oder C₂H₃, n-C₄H₉ [Tetrahedron 41, 5569 (1985)] oder H, CH₃ [Chem. Ber. 97, 159 (1964)] oder C₃H₅ [Chem. Pharm. Bull. 20, 122 (1972)] oder CH=CH₂, C≡CH, C(CH₃)=CH₂ [Zh.Org.Chim. 3 (1967) 980 ff.] oder mit
- R =: OCH₃ und R¹ = n-C₆H₁₃ [J. Org. Chem 48, 2780 (1983)] oder n-C₁₂H₂₅, n-C₈H₁₇ [Bull. Chem.Soc.Jp. 59 (1986) 2827 ff. bzw. Bull.Chem.Soc.Jp. 57 (1984) 2531 ff.] oder mit
- R =: OH, R¹ = H, CH₃ [Chem. Ber. 97, 159 (1964)] oder mit
- R=: Phenylamino oder p-Chlorphenylamino, R¹ = n-C₁₂H₂₅ [Synthesis 1986, 488]

Verbindungen der Formel (II) worin X¹= COR und X²= R¹ mit

| | | |
|---|---|---|
| R¹ = | R = | |
| H | OCH₃ | Dokl.Akad.Nauk. SSSR 1974, 109 ff. |
| CH₃ | OCH₃ | Tetrahedron Lett. 3155 (1975) oder Dokl.Akad.Nauk. SSSR 1974, 109 ff. |
| C₂H₅ | OCH₃ | Bull. Chem.Soc.Jp. 59 (1986) 2827 ff. oder Dokl.Akad.Nauk. SSSR 1974, 109 ff. |
| C₂H₅ | OC₂H₅ | J. Org. Chem 41, 122 (1976) |
| t·C₄H₉ | OCH₃ | Chem. Ber. 106, 3345 (1973) |
| t·C₄H₉ | OC₂H₅ | US 205 189 |
| iso·C₃H₇ | OC₂H₅ | " |
| (C₁-C₇)alkyl | OC₂H₅ | Sci.Eng.Rev.'Doshisha Univ. 1991, 37 ff. |

bekannt, ihre Safenerwirkung wurde jedoch nicht erkannt.
Gegenstand der vorliegenden Erfindung sind daher auch die bisher nicht bekannten Verbindungen der Formel (I) mit Ausnahme der oben definierten Verbindungen.

Die bekannten und die bisher noch nicht bekannten Verbindungen der Formel (I) können nach oder analog den in der genannten Literatur beschriebenen Verfahren hergestellt werden. Beispielsweise erhält man Verbindungen der Formel (I) worin X¹ = R¹ und X² = COR, indem man ein Olefin der Formel (II)

H₂C = CH - R¹ (II)

mit einer Verbindung der Formel (III) umsetzt, 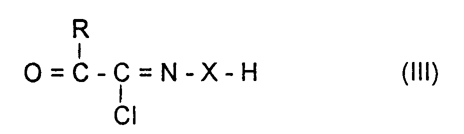 wobei R¹, R und X in den Formeln (II) und (III) wie in Formel (I) definiert sind. Verbindungen der Formel (I) worin X¹ = COR und X² = R¹, erhält man, indem man Acrylsäureester der allgemeinen Formel (IV) mit 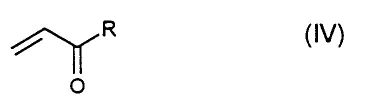 Verbindungen der Formel (V) 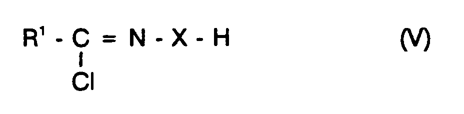 umsetzt, wobei in den Formeln (IV und V) R, R¹ und X wie in Formel (I) definiert sind.

Die Umsetzungen werden vorzugsweise in einem aprotischen, dipolaren organischen Lösungsmittel, einem aliphatischen Ether wie z.B. Diethylether, Tetrahydrofuran, Ethylenglykoldimethylether, Ethylenglykoldiethylether, Diethylenglykoldimethylether oder -diethylether, bei -10°C bis zum Siedepunkt des Lösungsmittels und in Gegenwart einer organischen Base wie Triethylamin und Pyridin oder einer anorganischen Base wie Kalium- und Natriumcarbonat oder Natriumbicarbonat durchgeführt.

Die Verbindungen der Formel (II), (III), (IV) und (V) sind bekannt oder lassen sich nach allgemein bekannten Verfahren herstellen (siehe beispielsweise J. Am. Chem. Soc. 46, 731 (1924); Angew. Chemie 75, 604 (1963).

Verbindungen der Formel I reduzieren oder unterbinden phytotoxische Nebenwirkungen von Herbiziden, die beim Einsatz der Herbizide in Nutzpflanzenkulturen auftreten können, und können deshalb in üblicher Weise als Antidote oder Safener bezeichnet werden.

Die erfindungsgemäßen Verbindungen der Formel (I) können zusammen mit herbiziden Wirkstoffen oder in beliebiger Reihenfolge ausgebracht werden und sind dann in der Lage, schädliche Nebenwirkungen dieser Herbizide bei Kulturpflanzen zu reduzieren oder völlig aufzuheben, ohne die Wirksamkeit dieser Herbizide gegen Schadpflanzen zu beeinträchtigen.

Hierdurch kann das Einsatzgebiet herkömmlicher Pflanzenschutzmittel ganz erheblich erweitert werden. Herbizide, deren phytotoxische Nebenwirkungen auf Kulturpflanzen mittels Verbindungen der Formel (I) herabgesetzt werden können, sind z.B. Carbamate, Thiocarbamate, Halogenacetanilide, substituierte Phenoxy-, Naphthoxy- und Phenoxy-phenoxycarbonsäurederivate sowie Heteroaryloxyphenoxyalkancarbonsäurederivate, wie Chinolyloxy-, Chinoxalyloxy-, Pyridyloxy-, Benzoxalyloxy- und Benzthiazolyloxy-phenoxyalkancarbonsäureester, Cyclohexandionabkömmlinge, Imidazolinone sowie Sulfonylharnstoffe. Bevorzugt sind dabei Phenoxyphenoxy- und Heteroaryloxy-phenoxycarbonsäureester und - salze, Sulfonylharnstoffe und Imidazolinone.

Geeignete Herbizide, die mit den erfindungsgemäßen Safenern kombiniert werden können sind beispielsweise:
A) Herbizide vom Typ der Phenoxyphenoxy- und Heteroarylphenoxycarbonsäure-(C₁-C₄)alkyl-, (C₂-C₄)alkenyl- und (C₃-C₄)alkinylester wie
A1) Phenoxy-phenoxy- und Benzyloxy-phenoxy-carbonsäure-derivate, z.B.
   2-(4-(2,4-Dichlorphenoxy)-phenoxy)-propionsäuremethylester (Diclofop-methyl),
   2-(4-(4-Brom-2-chlorphenoxy)-phenoxy)-propionsäuremethylester (s. DE-A-2601548),
   2-(4-(4-Brom-2-fluorphenoxy)-phenoxy)-propionsäuremethylester (s. US-A-4808750),
   2-(4-(2-Chlor-4-trifluormethylphenoxy)-phenoxy)-propionsäuremethylester (s. DE-A-2433067),
   2-(4-(2-Fluor-4-trifluormethylphenoxy)-phenoxy)-propionsäuremethylester (s. US-A-4808750),
   2-(4-(2,4-Dichlorbenzyl)-phenoxy)-propionsäuremethylester (s. DE-A-2417487),
   4-(4-(4-Trifluormethylphenoxy)-phenoxy)-pent-2-en-säureethylester,
   2-(4-(4-Trifluormethylphenoxy)-phenoxy)-propionsäuremethylester (s. DE-A-
   2433067),
A2) "Einkernige" Heteroaryloxy-phenoxy-alkancarbonsäurederivate z.B.
   2-(4-(3,5-Dichlorpyridyl-2-oxy)-phenoxy)-propionsäureethylester (s. EP-A-2925),
   2-(4-(3,5-Dichlorpyridyl-2-oxy)-phenoxy)-propionsäurepropargylester (EP-A-3114),
   2-(4-(3-Chlor-5-trifluormethyl-2-pyridyloxy)-phenoxy-propionsäure-methylester (s. EP-A-3890),
   2-(4-(3-Chlor-5-trifluormethyl-2-pyridyloxy)-phenoxy)-propionsäure-ethylester (s. EP-A-3890),
   2-(4-(5-Chlor-3-fluor-2-pyridyloxy)-phenoxy)-propionsäurepropargylester (EP-A-191736),
   2-(4-(5-Trifluormethyl-2-pyridyloxy)-phenoxy)-propionsäurebutylester, Fluazifopbutyl),
A3) "Zweikernige" Heteroaryloxy-phenoxy-alkancarbonsäurederivate, z.B.
   2-(4-(6-Chlor-2-chinoxalyloxy)-phenoxy)-propionsäuremethylester und - ethylester (Quizalofop-methyl und -ethyl),
   2-(4-(6-Fluor-2-chinoxalyloxy)-phenoxy)-propionsäuremethylester(s. J. Pest. Sci. Vol. 10, 61 (1985)),
   2-(4-(6-Chlor-2-chinoxalyloxy)-phenoxy)-propionsäure,-methylestertetrahydrofurfurylester und -2-isopropylidenaminooxyethylester (Propaquizafop u. verschiedene Ester),
   2-(4-(6-Chlorbenzoxazol-2-yl-oxy)-phenoxy)-propionsäureethylester(Fenoxapropethyl) und
   2-(4-(6-Chlorbenzthiazol-2-yloxy)-phenoxypropionsäureethylester(s. DE-A-2640730);
B) Herbizide aus der Sulfonylharnstoff-Reihe, wie z.B. Pyrimidin- oder Triazinylaminocarbonyl-[benzol-, pyridin-, pyrazol-, thiophen- und (alkylsulfonyl)alkylamino-]-sulfamide; bevorzugt als Substituenten am Pyrimidinring oder Triazinring sind Alkoxy, Alkyl, Haloalkoxy, Haloalkyl, Halogen oder Dimethylamino, wobei alle Substituenten unabhängig voneinander kombinierbar sind, bevorzugte Substituenten im Benzol-, Pyridin-, Pyrazol-, Thiophen- oder (Alkylsulfonyl)alkylamino-Teil sind Alkyl, Alkoxy, Halogen, Nitro, Alkoxycarbonyl, Aminocarbonyl, Alkylaminocarbonyl, Dialkylaminocarbonyl, Alkoxyaminocarbonyl, N-Alkyl-N-alkoxyaminocarbonyl, Haloalkoxy, Haloalkyl, Alkylcarbonyl, Alkoxyalkyl, (Alkansulfonyl)alkylamino; geeignete Sulfonylharnstoffe sind beispielsweise
B1) Phenyl- und Benzylsulfonylharnstoffe und verwandte Verbindungen, z.B.
   1-(2-Chlorphenylsulfonyl)-3-(4-methoxy-6-methyl-1,3,5-triazin-2-yl)-harnstoff (Chlorsulfuron),
   1-(2-Ethoxycarbonylphenylsulfonyl)-3-(4-chlor-6-methoxypyrimidin-2-yl)-harnstoff (Chlorimuron-ethyl),
   1-(2-Methoxyphenylsulfonyl)-3-(4-methoxy-6-methyl-1,3,5-triazin-2-yl)-harnstoff (Metsulfuron-methyl),
   1-(2-Chlorethoxy-phenylsulfonyl)-3-(4-methoxy-6-methyl-1,3,5-triazin-2-yl)-harnstoff (Triasulfuron),
   1-(2-Methoxycarbonyl-phenylsulfonyl)-3-(4,6-dimethyl-pyrimidin-2-yl)-harnstoff (Sulfometuron-methyl),
   1-(2-Methoxycarbonylphenylsulfonyl)-3-(4-methoxy-6-methyl-1,3,5-triazin-2-yl)-3-methylharnstoff (Tribenuron-methyl)
   1-(2-Methoxycarbonylbenzylsulfonyl)-3-(4,6-dimethoxy-pyrimidin-2-yl)-harnstoff (Bensulfuron-methyl)
   1-(2-Methoxycarbonylphenylsulfonyl)-3-(4,6-bis-(difluormethoxy)-pyrimidin-2-yl)-harnstoff (Primisulfuron-methyl),
   3-(4-Ethyl-6-methoxy-1,3,5-triazin-2-yl)-1-(2,3-dihydro-1,1-dioxo-2-methylbenzo[b]thiophen-7-sulfonyl)-harnstoff (s. EP-A-79683),
   3-(4-Ethoxy-6-ethyl-1,3,5-triazin-2-yl)-1-(2,3-dihydro-1,1-dioxo-2-methylbenzo[b]thiophen-7-sulfonyl)-harnstoff (s. EP-A-79683),
B2) Thienylsulfonylharnstoffe, z.B.
   1-(2-Methoxycarbonylthiophen-3-yl)-3-(4-methoxy-6-methyl-1,3,5-triazin-2-yl)-harnstoff (Thifensulfuron-methyl),
B3) Pyrazolylsulfonylharnstoffe, z.B.
   1-(4-Ethoxycarbonyl-1-methylpyrazol-5-yl-sulfonyl)-3-(4,6-dimethoxypyrimidin-2-yl)-harnstoff (Pyrazosulfuron-methyl),
   Methyl-3-chlor-5-(4,6-dimethoxypyrimidin-2-ylcarbamoylsulfamoyl)-1-methylpyrazol-4-carboxylat (s. EP-A-282613),
   5-(4,6-Dimethylpyrimidin-2-yl)-carbamoylsulfonyl)-1-(2-pyridyl)-pyrazol-4-carbonsäuremethylester (NC 330, siehe Brighton Crop Prot. Conf.Weeds 1991, Bd.1,S.45ff.)
B4) Sulfondiamid-Derivate, z.B.
   3-(4,6-Dimethoxypyrimidin-2-yl)-1-(N-methyl-N-methylsulfonylaminosulfonyl)-harnstoff (Amidosulfuron) und Strukturanaloge (s. EP-A-0131258 und Z. Pfl. Krankh. Pfl. Schutz, Sonderheft XII, 489-497 (1990)),
B5) Pyridylsulfonylharnstoffe, z.B.
   1-(3-N,N-Dimethylaminocarbonylpyridin-2-yl-sulfonyl)-3(4,6-dimethoxypyrimidin-2-yl)-harnstoff (Nicosulfuron),
   1-(3-Ethylsulfonylpyridin-2-yl-sulfonyl)-3-(4,6-dimethoxy-pyrimidin-2-yl)-harnstoff (DPX-E 9636, s. Brighton Crop Prot. Conf. - Weeds - 1989, S. 23 ff.), Pyridylsulfonylharnstoffe, wie sie in den deutschen Patentanmeldungen P 4000503.8 (ZA/0173) und P 4030577.5 beschrieben sind, vorzugsweise solche der Formel (VI) oder deren Salze, 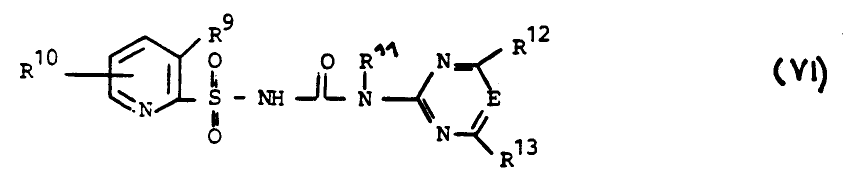 worin
   - E: CH oder N, vorzugsweise CH,
   - R⁹: Iod oder NR¹⁴R¹⁵,
   - R¹⁰: Wasserstoff, Halogen, Cyano, (C₁-C₃)-Alkyl, (C₁-C₃)-Alkoxy, (C₁-C₃)-Haloalkyl, (C₁-C₃)-Haloalkoxy, (C₁-C₃)-Alkylthio, (C₁-C₃)-Alkoxy-(C₁-C₃)-alkyl, (C₁-C₃)-Alkoxy-carbonyl, Mono- oder Di-(C₁-C₃)-alkyl-amino, (C₁-C₃)-Alkyl-sulfinyl oder -sulfonyl, SO₂-NR^{a}R^{b} oder CO-NR^{a}R^{b}, insbesondere Wasserstoff,
   - R^{a},R^{b}: unabhängig voneinander Wasserstoff, (C₁-C₃)-Alkyl, (C₁-C₃)-Alkenyl, (C₁-C₃)-Alkinyl oder zusammen -(CH₂)₄-, -(CH₂)₅- oder (CH₂)₂-O-(CH₂)₂-,
   - R¹¹: Wasserstoff oder CH₃,
   - R¹²: Halogen, (C₁-C₂)-Alkyl, (C₁-C₂)-Alkoxy, (C₁-C₂)-Haloalkyl, vorzugsweise CF₃, (C₁-C₂)-Haloalkoxy, vorzugsweise OCHF₂ oder OCH₂CF₃,
   - R¹³: (C₁-C₂)-Alkyl, (C₁-C₂)-Haloalkoxy, vorzugsweise OCHF₂, oder (C₁-C₂)-Alkoxy, und
   - R¹⁴: (C₁-C₄)-Alkyl und
   - R¹⁵: (C₁-C₄)-Alkylsulfonyl oder
   - R¹⁴ und R¹⁵: gemeinsam eine Kette der Formel -(CH₂)₃SO₂- oder - (CH₂)₄SO₂-bedeuten, z.B. 3-(4,6-Dimethoxypyrimidin-2-yl)-1-(3-N-methylsulfonyl-N-methylaminopyridin-2-yl)-sulfonylharnstoff,
B6) Alkoxyphenoxysulfonylharnstoffe, wie sie in EP-A-0342569 beschrieben sind, vorzugsweise solche der Formel (VII) oder deren Salze, 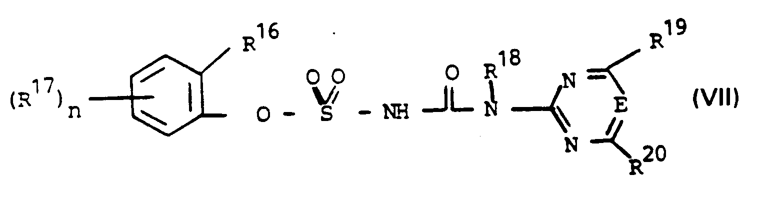 worin
   - E: CH oder N, vorzugsweise CH,
   - R¹⁶: Ethoxy, Propoxy oder Isopropoxy,
   - R¹⁷: Wasserstoff, Halogen, NO₂, CF₃, CN, (C₁-C₄)-Alkyl, (C₁-C₄)-Alkoxy, (C₁-C₄)-Alkylthio oder (C₁-C₃)-Alkoxy-carbonyl, vorzugsweise in 6-Position am Phenylring,
   - n: 1, 2 oder 3, vorzugsweise 1,
   - R¹⁸: Wasserstoff, (C₁-C₄)-Alkyl oder (C₃-C₄)-Alkenyl,
   - R¹⁹,R²⁰: unabhängig voneinander Halogen, (C₁-C₂)-Alkyl, (C₁-C₂)-Alkoxy, (C₁-C₂)-Haloalkyl, C₁-C₂-Haloalkoxy oder (C₁-C₂)-Alkoxy-(C₁-C₂)-alkyl, vorzugsweise OCH₃ oder CH₃, bedeuten, z.B. 3-(4,6-Dimethoxypyrimidin-2-yl)-1-(2-ethoxyphenoxy)-sulfonylharnstoff,
   und andere verwandte Sulfonylharnstoffderivate und Mischungen daraus;
C) Chloracetanilid-Herbizide wie
   N-Methoxymethyl-2,6-diethyl-chloracetanilid (Alachlor),
   N-(3'-Methoxyprop-2'-yl)-2-methyl-6-ethyl-chloracetanilid (Metolachlor),
   N-(3-Methyl-1,2,4-oxdiazol-5-yl-methyl)-chloressigsäure-2,6-dimethylanilid,
   N-(2,6-Dimethylphenyl)-N-(1-pyrazolylmethyl)-chloressigsäureamid (Metazachlor),
D) Thiocarbamate wie
   S-Ethyl-N,N-dipropylthiocarbamat (EPTC) oder
   S-Ethyl-N,N-diisobutylthiocarbamat (Butylate);
E) Cyclohexandion-Derivate wie
   Methyl-3-(1-allyloxyimino)butyl)-4-hydroxy-6,6-dimethyl-2-oxocyclohex-3-encarboxylat (Alloxydim),
   2-(N-Ethoxybutyrimidoyl)-5-(2-ethylmercaptopropyl)-3-hydroxy-2-cyclohexen-1-on (Sethoxydim),
   2-(N-Ethoxybutyrimidoyl)-5-(2-phenylmercaptopropyl)-3-hydroxy-2-cyclohexen-1-on (Cloproxydim),
   2-(1-(3-Chlorallyloxy)-iminobutyl)-5-(2-ethylmercapto)-propyl)-3-hydroxy-2-cyclohexen-1-on,
   2-(1-(3-Chlorallyloxy)-iminopropyl)-5-2-ethylmercapto)-propyl)-3-hydroxycyclohex-2-en-1-on (Clethodim),
   2-(1-Allyloxyiminobutyl)-4-methoxycarbonyl-5,5-dimethyl-3-oxocyclohexenol,
   2-(1-(Ethoxyimino)-butyl)-3-hydroxy-5-(thian-3-yl)-cyclohex-2-enon (Cycloxydim),
   oder
   2-(1-Ethoxyiminopropyl)-5-(2,4,6-trimethylphenyl)-3-hydroxy-2-cyclohexen-1-on (Tralkoxydim);
F) 2-Carboxyphenylimidazolinone oder 2-Carboxyheteroarylimidazolinone und deren Ester und Salze, z.B.
   2-(4-Isopropyl-4-methyl-5-oxo-2-imidazolin-2-yl)-5-methylbenzoesäuremethylester in Mischung mit
   2-(4-Isopropyl-4-methyl-5-oxo-2-imidazolin-2-yl)-4-methylbenzoesäuremethylester (Imazamethabenz),
   5-Ethyl-2-(4-isopropyl-4-methyl-5-oxo-2-imidazolin-2-yl)-pyridin-3-carbonsäure (Imazethapyr), z.B. das Ammoniumsalz,
   2-(4-Isopropyl-4-methyl-5-oxo-2-imidazolin-2-yl)-chinolin-3-carbonsäure (Imazaquin), z.B. das Ammoniumsalz, und 2-[4,5-dihydro-4-methyl-4-(1-methylethyl)-5-oxo-1H-imidazol-2-yl]-5-methyl-3-pyridin-carbonsäure (Imazethamethapyr), deren Ester und Salze.

Die obengenannten herbiziden Wirkstoffe der Gruppen A bis F sind dem Fachmann bekannt und in der Regel in "The Pesticide Manual", British Crop Protection Council, 8th Edition (1990-91) oder in "Agricultural Chemicals Book II-Herbicides-", by W.T. Thompson, Thompson Publications, Fresno CA, USA 1990 oder in "Farm Chemicals Handbook '90", Meister Publishing Company, Willoughby OH, USA 1990 beschrieben.

Die herbiziden Wirkstoffe und die erwähnten Safener können zusammen (als fertige Formulierung oder im Tank-mix-Verfahren) oder in beliebiger Reihenfolge nacheinander ausgebracht werden. Das Gewichtsverhältnis Safener:Herbizid kann innerhalb weiter Grenzen variieren und liegt vorzugsweise im Bereich von 1:10 bis 10:1, insbesondere von 1:10 bis 5:1. Die jeweils optimalen Mengen an Herbizid und Safener sind vom Typ des verwendeten Herbizids oder vom verwendeten Safener sowie von der Art des zu behandelnden Pflanzenbestandes abhängig und lassen sich von Fall zu Fall durch entsprechende Vorversuche ermitteln.

Haupteinsatzgebiete für die Anwendung der Safener sind vor allem Getreidekulturen (Weizen, Roggen, Gerste, Hafer), Reis, Mais, Sorghum, aber aber auch Baumwolle und Sojabohne, vorzugsweise Getreide und Mais.

Ein besonderer Vorteil der erfindungsgemäßen Safener der Formel I ist bei deren Kombination mit Wirkstoffen aus der Gruppe der Sulfonylharnstoffen und/oder Imidazolinone festzustellen. Herbizide der genannten Strukturklassen hemmen primär das Schlüsselenzym Acetolactatsynthase (ALS) in den Pflanzen und sind bezüglich des Wirkungsmechanismus daher zumindest partiell verwandt. Einige Herbizide dieser Strukturklassen können speziell in Getreidekulturen und/oder Mais nicht oder nicht genügend selektiv eingesetzt werden. Durch die Kombination mit den erfindungsgemäßen Safenern sind auch bei diesen Herbiziden in Getreide oder Mais hervorragende Selektivitäten zu erreichen.

Die Safener der Formel I werden je nach ihren Eigenschaften zur Vorbehandlung des Saatgutes der Kulturpflanze (Beizung der Samen) verwendet oder vor der Saat in die Saatfurchen eingebracht oder zusammen mit dem Herbizid vor oder nach dem Auflaufen der Pflanzen angewendet werden. Vorauflaufbehandlung schließt sowohl die Behandlung der Anbaufläche vor der Aussaat als auch die Behandlung der angesäten, aber noch nicht bewachsenen Anbauflächen ein. Bevorzugt ist die gemeinsame Anwendung mit dem Herbizid. Hierzu können Tankmischungen oder Fertigformulierungen eingesetzt werden.

Die benötigten Aufwandmengen der Safener können je nach Indikation und verwendetem Herbizid innerhalb weiter Grenzen schwanken und liegen in der Regel im Bereich von 0,001 bis 5 kg, vorzugsweise 0,005 bis 0,5 kg Wirkstoff je Hektar.

Gegenstand der vorliegenden Erfindung ist deshalb auch ein Verfahren zum Schutz von Kulturpflanzen vor phytotoxischen Nebenwirkungen von Herbiziden, das dadurch gekennzeichnet ist, daß eine wirksame Menge einer Verbindung der Formel I vor, nach oder gleichzeitig mit dem Herbizid auf die Pflanzen, Pflanzenteile, Pflanzensamen oder die Anbaufläche appliziert wird.

Gegenstand der Erfindung sind auch pflanzenschützende Mittel, die einen Wirkstoff der Formel I und übliche Formulierungshilfsmittel enthalten, sowie herbizide Mittel, die einen Wirkstoff der Formel I und einen herbiziden Wirkstoff sowie im Bereich des Pflanzenschutzes übliche Formulierungshilfsmittel enthalten.

Die Verbindungen der Formel I und deren Kombinationen mit einem oder mehreren der genannten herbiziden Wirkstoffe können auf verschiedene Art formuliert werden, je nachdem welche biologischen und/oder chemischphysikalischen Parameter vorgegeben sind. Als Formulierungsmöglichkeiten kommen beispielsweise in Frage: Spritzpulver (WP), emulgierbare Konzentrate (EC), wasserlösliche Pulver (SP), wasserlösliche Konzentrate (SL), konzentrierte Emulsionen (EW) wie Öl-in-Wasser und Wasser-in-Öl-Emulsionen, versprühbare Lösungen oder Emulsionen, Kapselsuspensionen (CS), Dispersionen auf Öl- oder Wasserbasis (SC), Suspoemulsionen, Suspensionskonzentrate, Stäubemittel (DP), ölmischbare Lösungen (OL), Beizmittel, Granulate (GR) in Form von Mikro-, Sprüh-, Aufzugs- und Adsorptionsgranulaten, Granulate für die Boden- bzw. Streuapplikation, wasserlösliche Granulate (SG), wasserdispergierbare Granulate (WG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie" Band 7, C. Hauser Verlag München, 4. Aufl. 1986; Wade van Valkenburg, "Pesticide Formulations", Marcel Dekker N.Y., 1973; K. Martins, "Spray Drying Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J.; H.v.Olphen "Intruduction to Clay Colloid Chemistry", 2nd Ed., J. Wiley & Sons, N.Y., Marsden "Solvents Guide", 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. INc., N.Y. 1964; Schönfeldt, "Grenzflächenaktive Äthylenoxidaddukte", Wiss. Verlagsgesell., Stuttgart 1976; Winnacker-Küchler "Chemische Technolgie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer oder nichtionischer Art (Netzmittel, Dispergiermittel), z.B. polyoxethylierte Alkylphenole, polyoxethylierte Fettalkohole und Fettamine, Fettalkoholpolyglykolethersulfate, Alkansulfonate oder Alkylarylsulfonate und Dispergiermittel, z.B. ligninsulfonsaures Natrium, 2,2,-dinaphthylmethan-6,6'-disulfonsaures Natrium, dibutylnaphthalinsulfonsaures Natrium oder auch oleylmethyltaurinsaures Natrium enthalten.

Emulgierbare Konzentrate werden durch Auflösen des Wirkstoffes in einem organischen Lösungsmittel. z.B. Butanol, Cyclohexanon, Dimethylformamid, Xylol oder auch höhersiedenden Aromaten oder Kohlenwasserstoffen unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte (z.B. Blockpolymere), Alkylpolyether, Sorbitanfettsäureester, Polyoxyethylensorbitanfettsäureester oder Polyoxethylensorbitester.

Stäubemittel erhält man durch Vermahlen des Wirkstoffes mit fein verteilten festen Stoffen, z.B. Talkum, natürlichen Tonen, wie Kaolin, Bentonit und Pyrophillit, oder Diatomeenerde.

Granulate können entweder durch Verdüsen des Wirkstoffes auf adsorptionsfähiges, granuliertes Inertmaterial hergestellt werden oder durch Aufbringen von Wirkstoffkonzentraten mittels Klibemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden. Wasserdispergierbare Granulate werden nach üblichen Verfahren wie Sprühtrocknung, Wirbelbett-Granulierung, Tellergranulierung, mit Hochgeschwindigkeitsmischern und Extrusion in der Regel ohne festes Inertmaterial hergestellt.

Die agrochemischen Zubereitungen enthalten in der Regel 0,1 bis 99 Gew.-%, insbesondere 0,1 bis 95 Gew.-%, Wirkstoffe der Formel I (Antidot) oder des Antidot/Herbizid-Wirkstoffgemischs und 1 bis 99,9 Gew.-%, insbesondere 5 bis 99,8 Gew.-%, eines festen oder flüssigen Zusatzstoffes und 0 bis 25 Gew.-%, insbesondere 0,1 bis 25 Gew.-%. eines Tensides.

In Spritzpulvern beträgt die Wirkstoffkonzentration z.B. etwa 10 bis 90 Gew.%, der Rest zu 100 Gew.-% besteht aus üblichen Formulierungsbestandteilen. Bei emulgierbaren Konzentraten beträgt die Wirkstoffkonzentration etwa 1 bis 80 Gew.-% Wirkstoffe. Staubförmige Formulierungen enthalten etwa 1 bis 20 Gew.-% an Wirkstoffen, versprühbare Lösungen etwa 0,2 bis 20 Gew.-% Wirkstoffe. Bei Granulaten wie wasserdispergierbaren Granulaten hängt der Wirkstoffgehalt zum Teil davon ab, ob die wirksame Verbindung flüssig oder fest vorliegt. In der Regel liegt der Gehalt bei den in Wasser dispergierbaren Granulaten zwischen 10 und 90 Gew.-%.
Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Formulierungen gegebenenfalls in üblicher Weise verdünnt, z.B. bei Spritzpulvern, emulgierbaren Konzentraten, Dispersionen und wasserdispergierbaren Granulaten mittels Wasser. Staubförmige Zubereitungen, Granulate sowie versprühbare Lösungen werden vor der Anwendung üblicherweise nicht mehr mit weiteren inerten Stoffen verdünnt. Mit den äußeren Bedingungen wie Temperatur, Feuchtigkeit, der Art des verwendeten Herbizids u.a. variiert die erforderliche Aufwandmenge der "Antidots".

Folgende Beispiele dienen zur Erläuterung der Erfindung:

### A. Formulierungsbeispiele

a) Ein Stäubmittel wird erhalten, indem man 10 Gew.-Teile einer Verbindung der Formel I oder eines Wirkstoffgemischs aus einem Herbizid und eine Verbindung der Formel I und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel I oder eines Wirkstoffgemischs aus einem Herbizid und einem Safener der Formel I, 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel I oder eines Wirkstoffgemischs aus einem Herbizid und einem Safener der Formel I, 6 Gew.-Teilen Alkylphenolpolyglykolether (^{R}Triton X 207), 3 Gew.-Teilen Isotridecanolpolyglykolether (8 EO) und 71 Gew.- Teilen paraffinischem Mineralöl (Siedebereich z.B. ca. 255 bis über 277°C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel I oder eines Wirkstoffgemischs aus einem Herbizid und einem Safener der Formel I, 75 Gew.Teilen Cyclohexanon als Lösemittel und 10 Gew.-Teilen oxethyliertes Nonylphenol als Emulgator.
e) Ein in Wasser dispergierbares Granulat wird erhalten, indem man
   - 75 Gew.-Teile: einer Verbindung der Formel I oder eines Wirkstoffgemischs aus einem Herbizid und einem Safener der Formel I,
   - 10 Gew.-Teile: ligninsulfonsaures Calcium,
   - 5 Gew.-Teile: Natriumlaurylsulfat,
   - 3 Gew.-Teile: Polyvinylalkohol und
   - 7 Gew.-Teile: Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   - 25 Gew.-Teile: einer Verbindung der Formel I oder eines Wirkstoffgemischs aus einem Herbizid und einem Safener der Formel I,
   - 5 Gew.-Teile: 2,2'-dinaphthylmethan-6,6'-disulfonsaures Natrium,
   - 2 Gew.-Teile: oleoylmethyltaurinsaures Natrium,
   - 1 Gew.-Teile: Polyvinylalkohol,
   - 17 Gew.-Teile: Calciumcarbonat und
   - 50 Gew.-Teile: Wasser
   auf einer Kolloidmühle homogensiert und vorzerkleinert, anschließend auf einer Perlmühle mahlt und die so erhaltene Suspension in einem Sprühturm mittels einer Einstoffdüse zerstäubt und trocknet.

### B. Chemische Beispiele

### 5-t-Butyl-2-isoxazolin-3-carbonsäureethylester (Beispiel 3; Tabelle I)

50,49 g 3,3-Dimethylbuten-(1) und 9,09 g 2-Chlor-2-hydroximinoessigsäureethylester werden in 500 ml Ether vorgelegt und 6,0 g Triethylamin in 100 ml Ether langsam zugetropft. Anschließend wird Wasser zugegeben und mehrmals mit Ether extrahiert. Die Etherphase wird mit MgSO₄ getrocknet, am Rotationsverdampfer eingeengt und über eine Kieselgelsäule (Lösungsmittel = n Heptan:Essigester = 9:1) getrennt.

Ausbeute: 9,0 g der gewünschten Reinsubstanz
Überschüssiges 3,3-Dimethylbuten-(1) wird zurückgewonnen.

### 3-t-Butyl-2-isoxazolin-5-carbonsäureethylester (Beispiel 3; Tabelle II)

13,6 g Trimethylacetohydroximoylchlorid und 20 g Acrylsäureethylester werden in 200 ml Ether gelöst. Unter Eiskühlung tropft man langsam 10,1 g Triethylamin zu. Mit Wasser wird ausgeschüttelt, die Etherphase mit MgSO₄ getrocknet. Nach Abdestillieren des Lösungsmittels wird destilliert. So werden bei 80-83°C und 0,2 mbar 17,9 g Produkt erhalten.

In analoger Weise werden die Verbindungen der Tabellen I und II hergestellt.

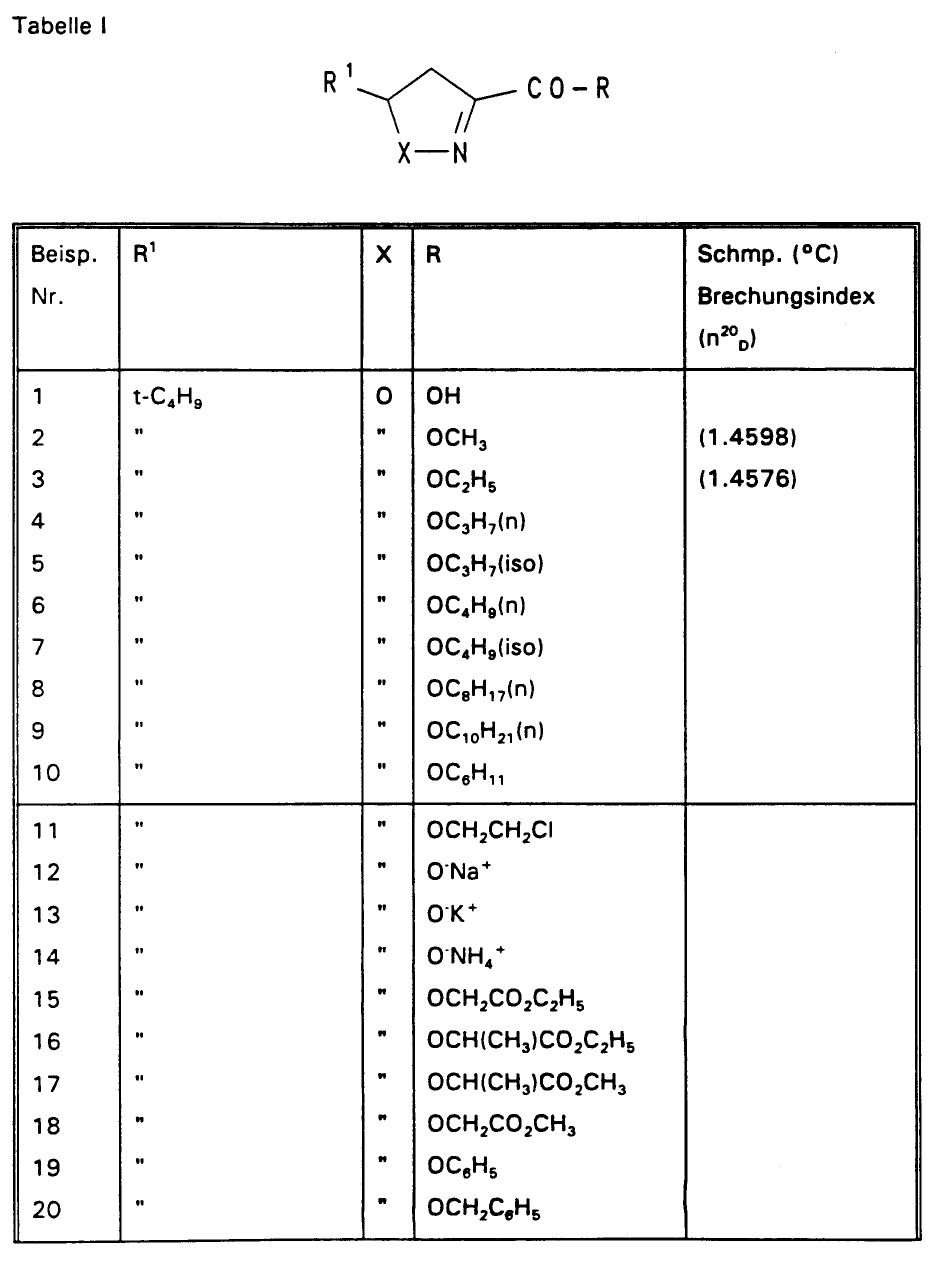

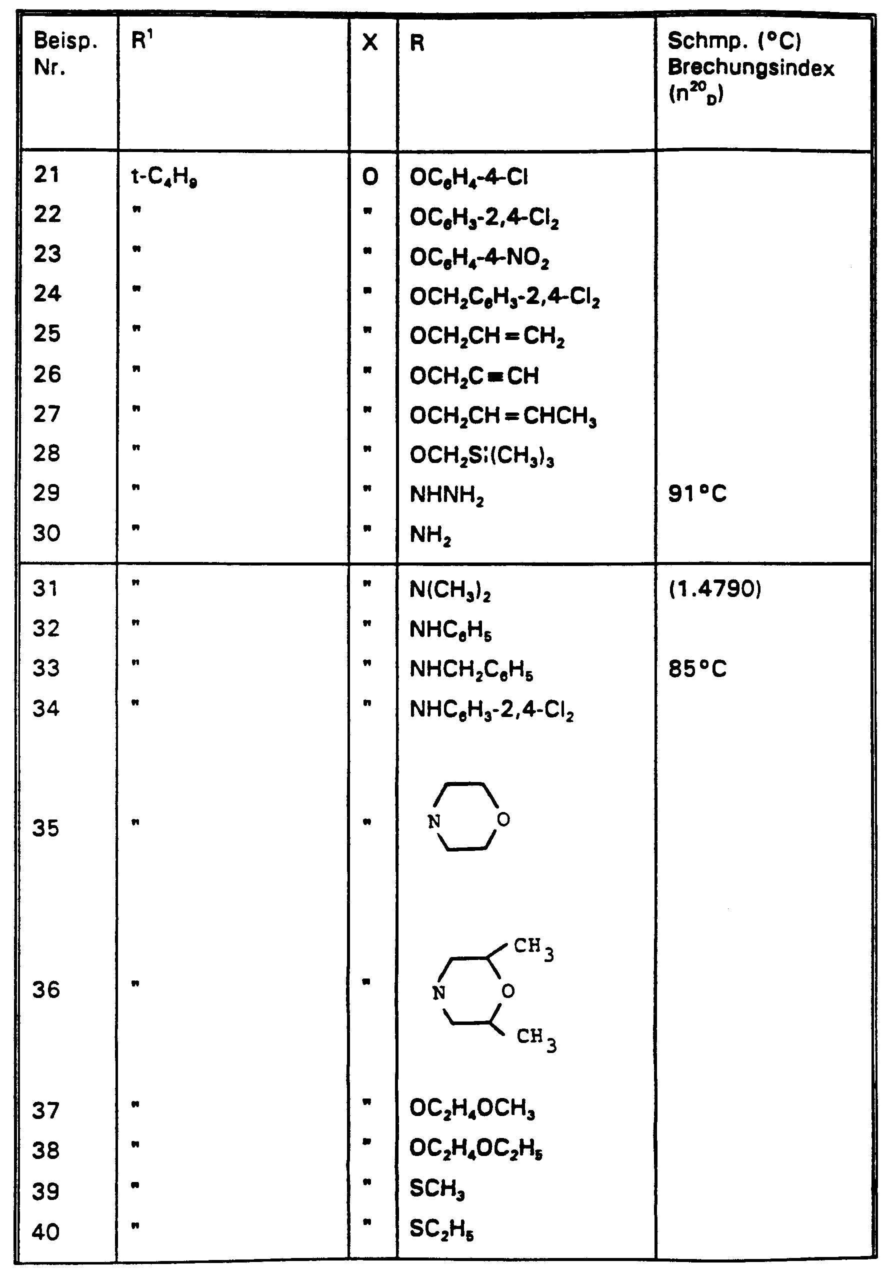

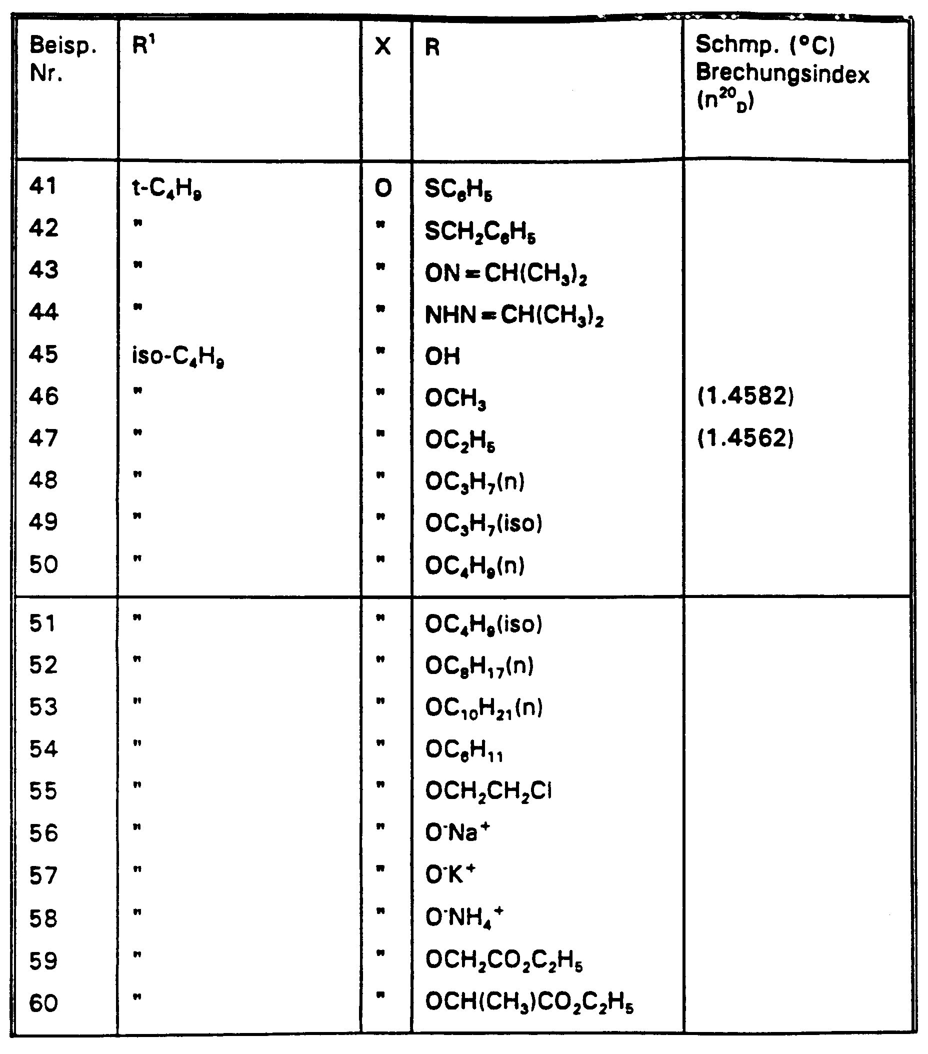

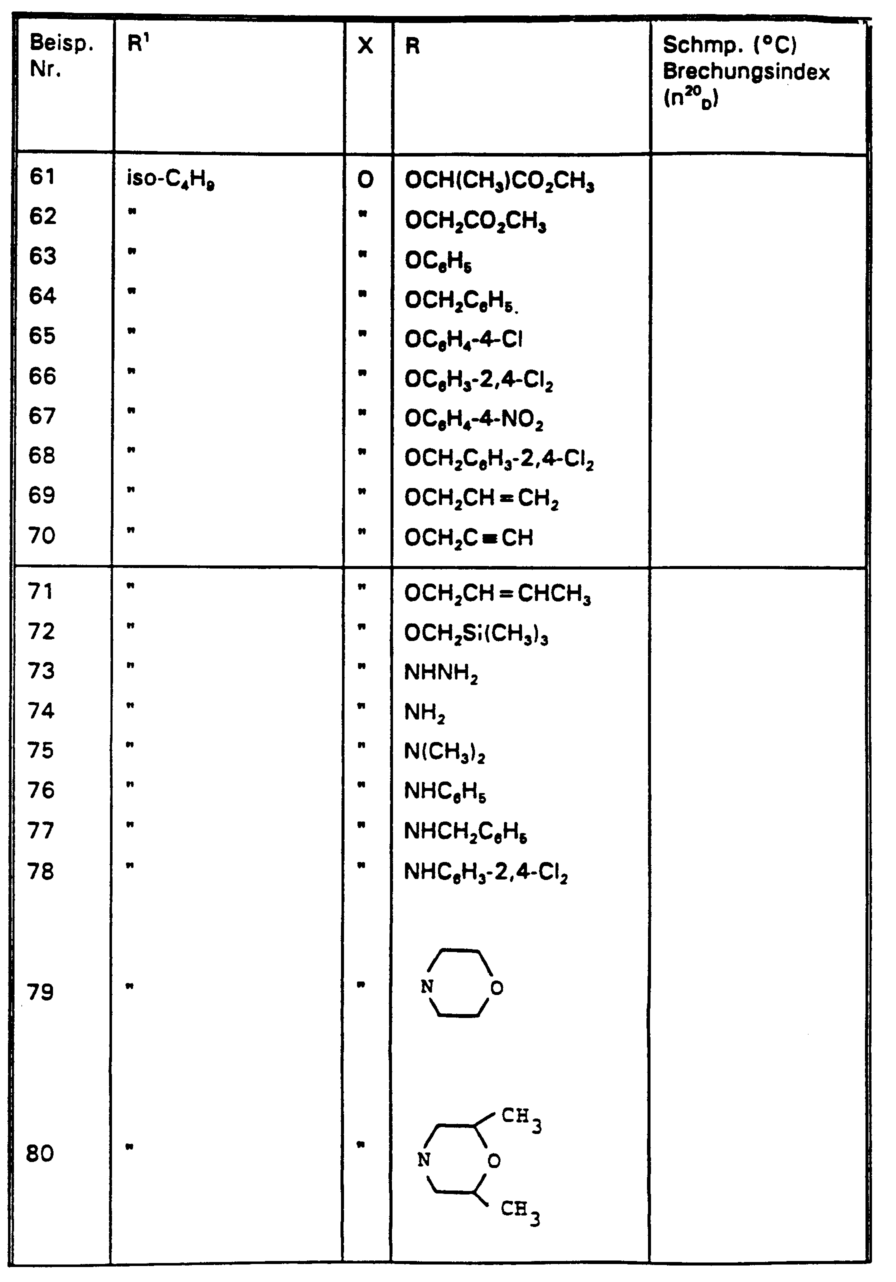

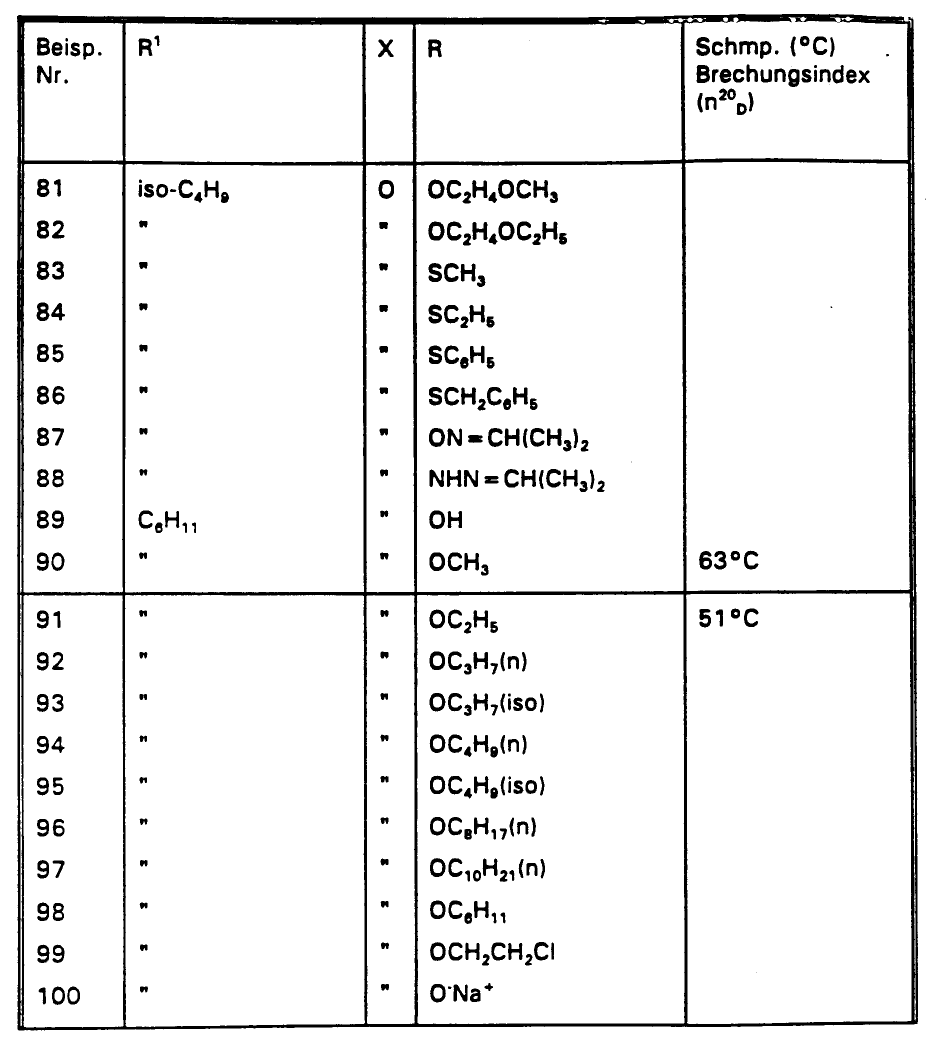

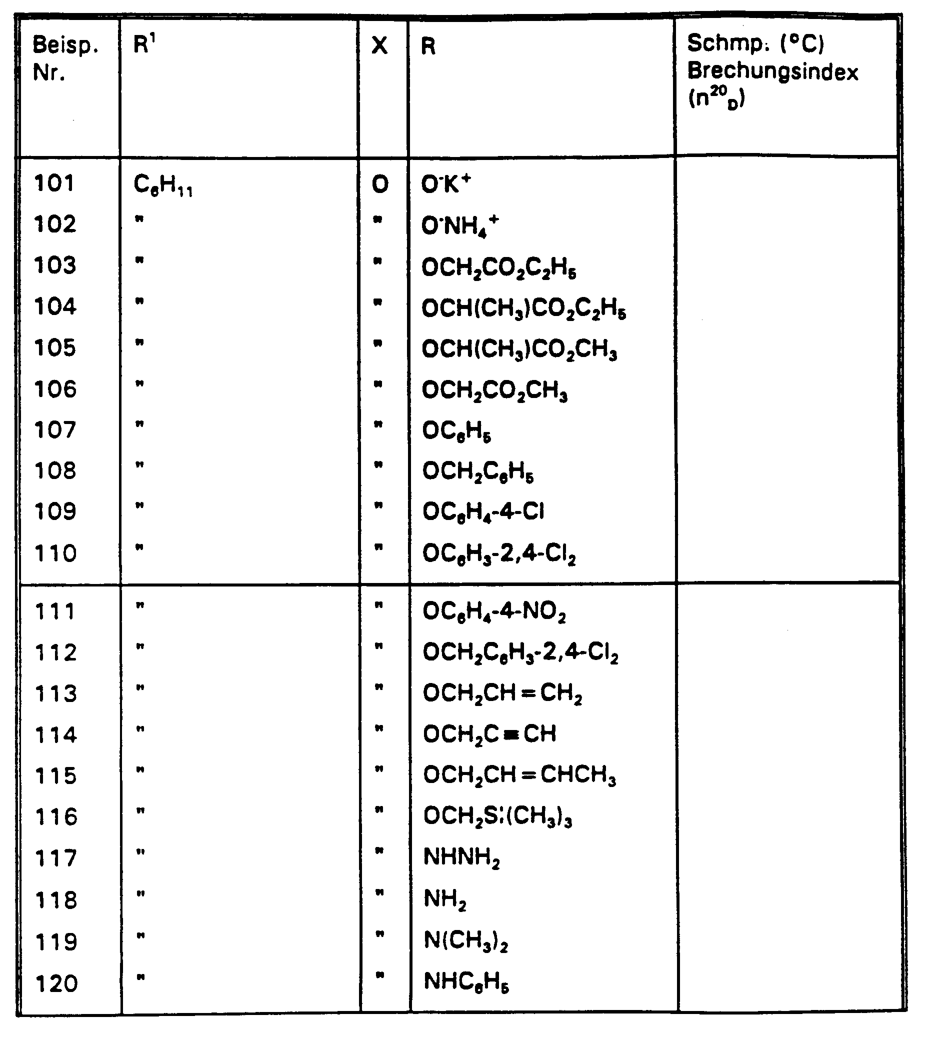

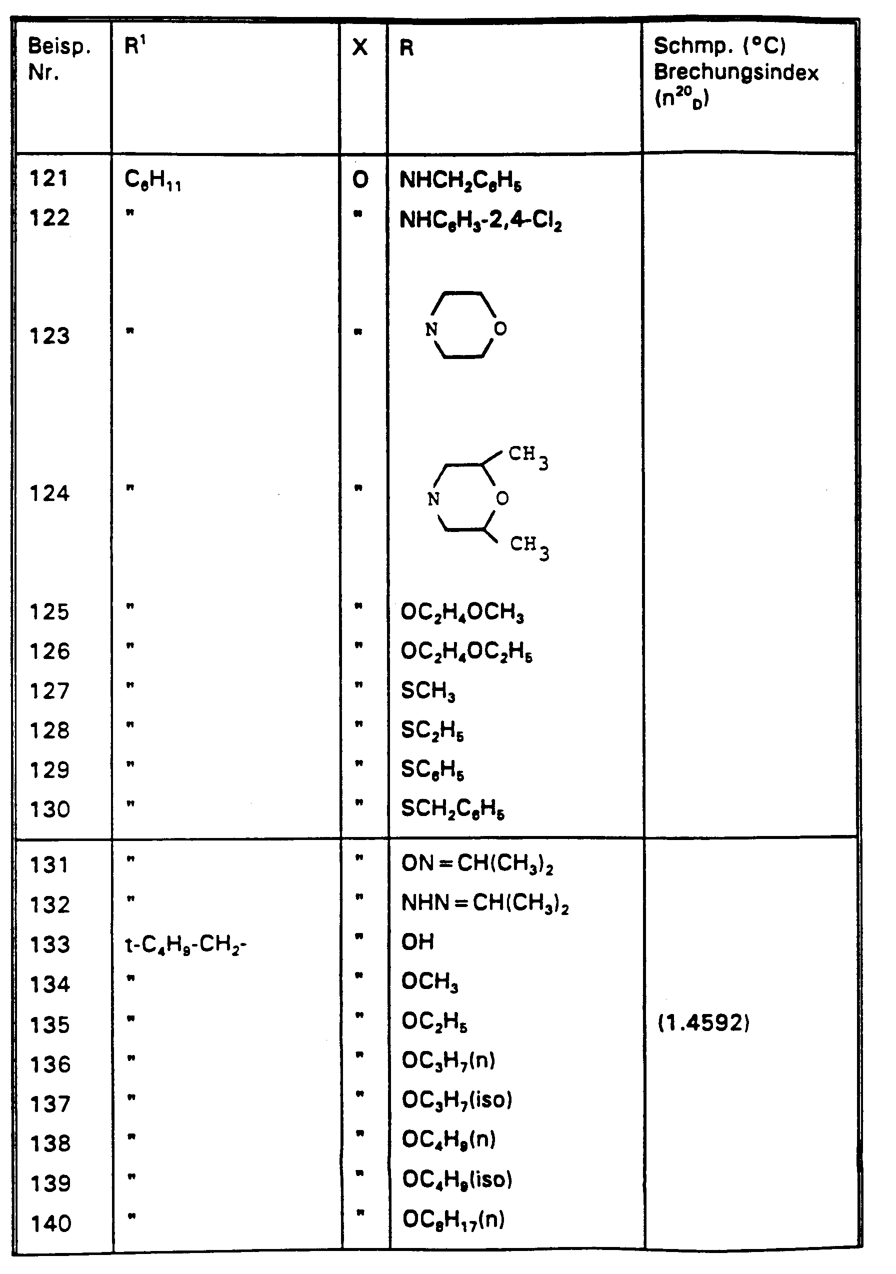

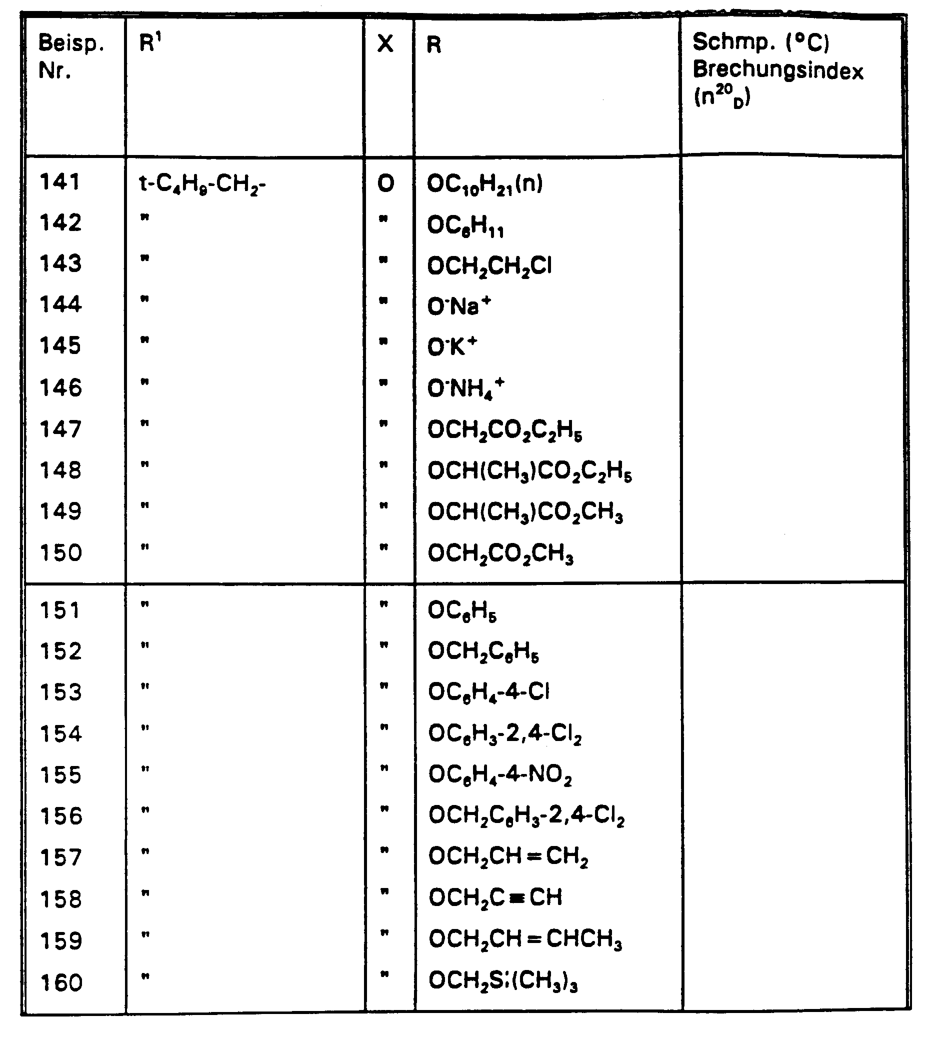

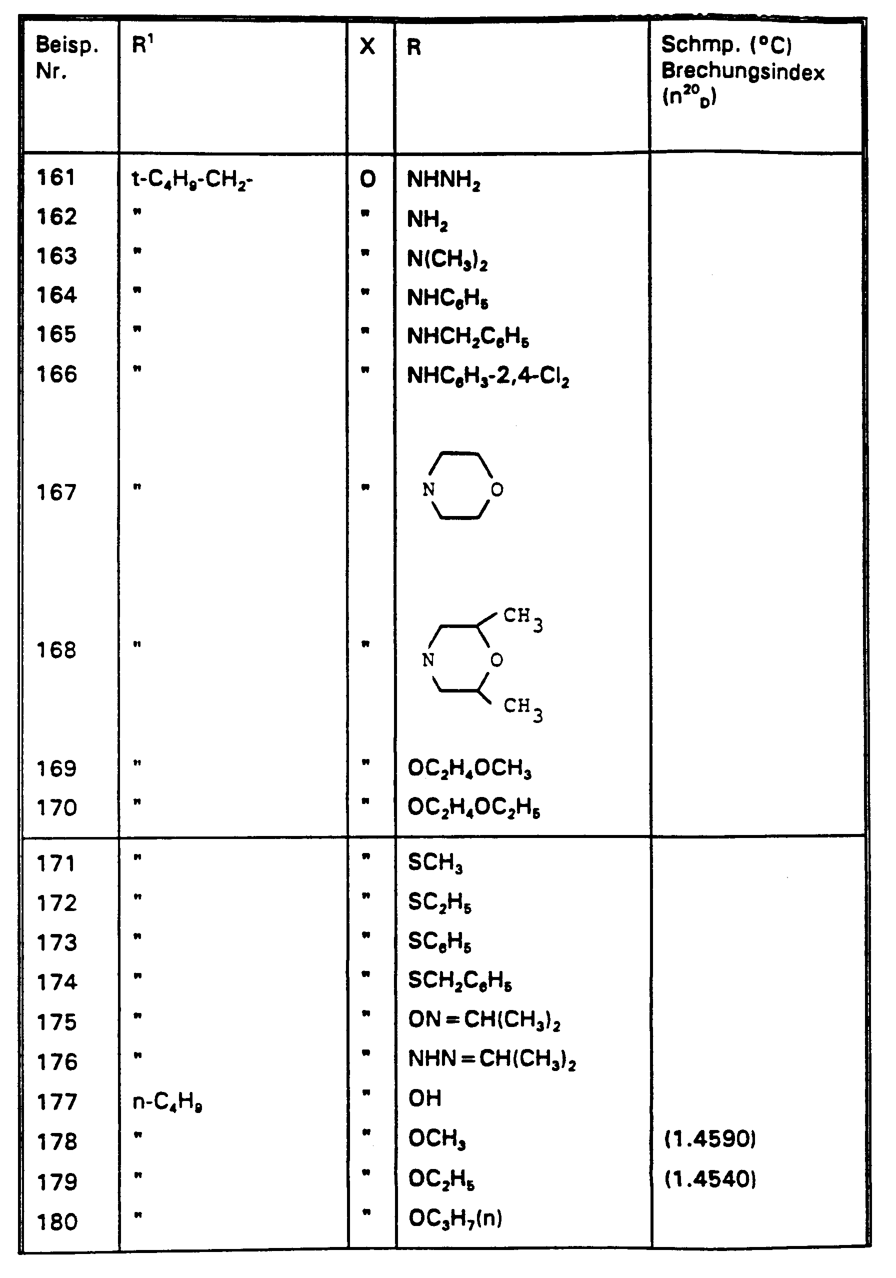

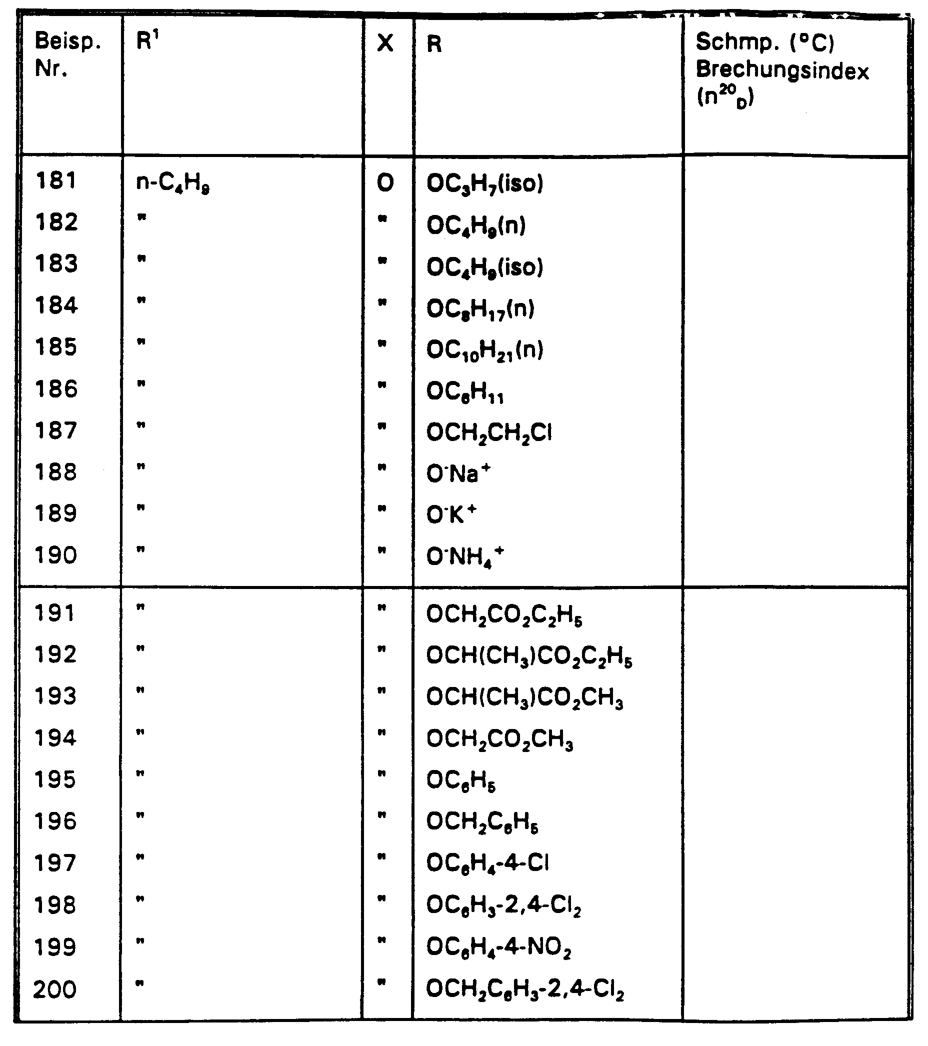

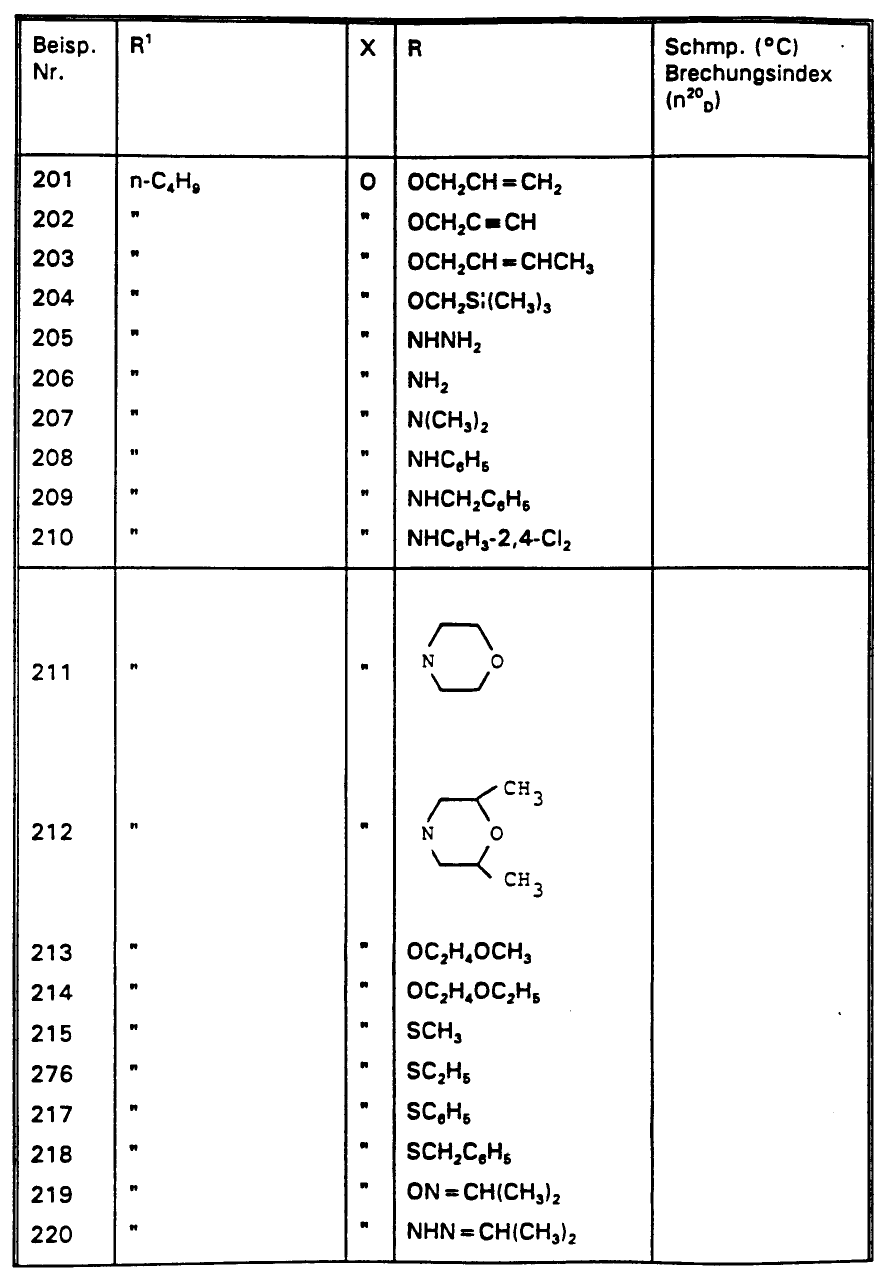

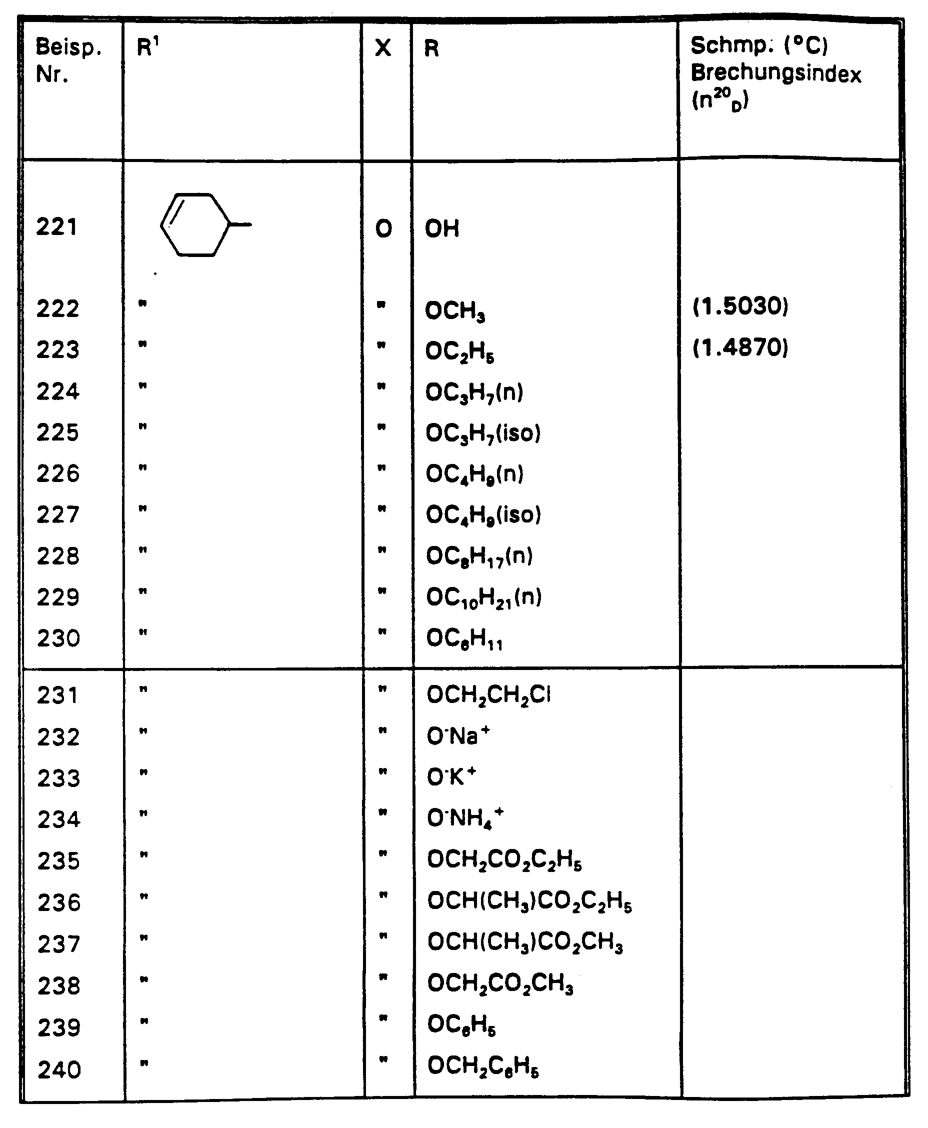

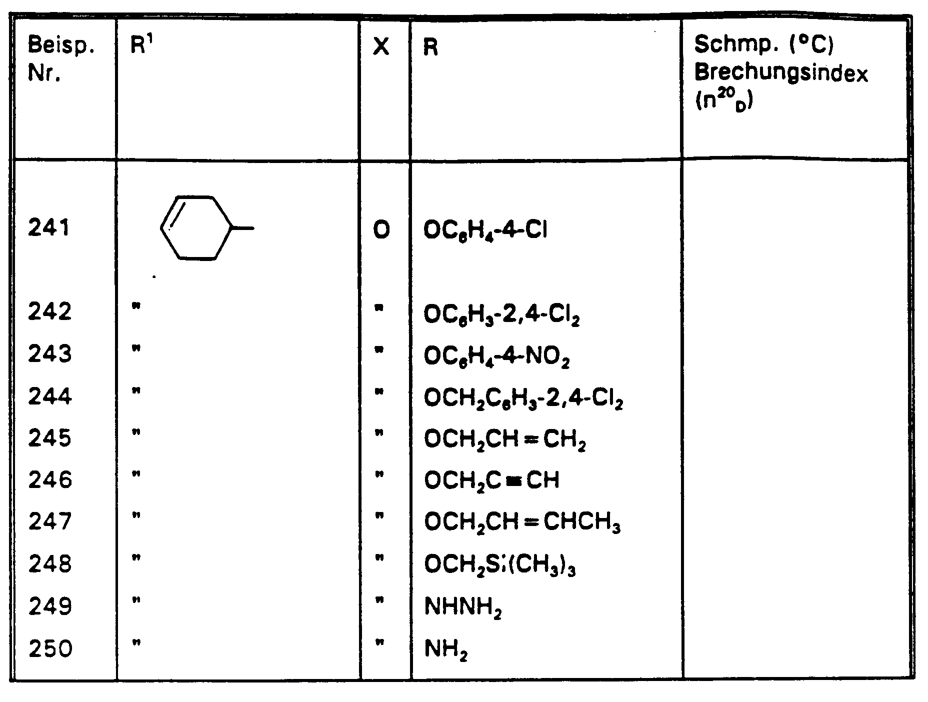

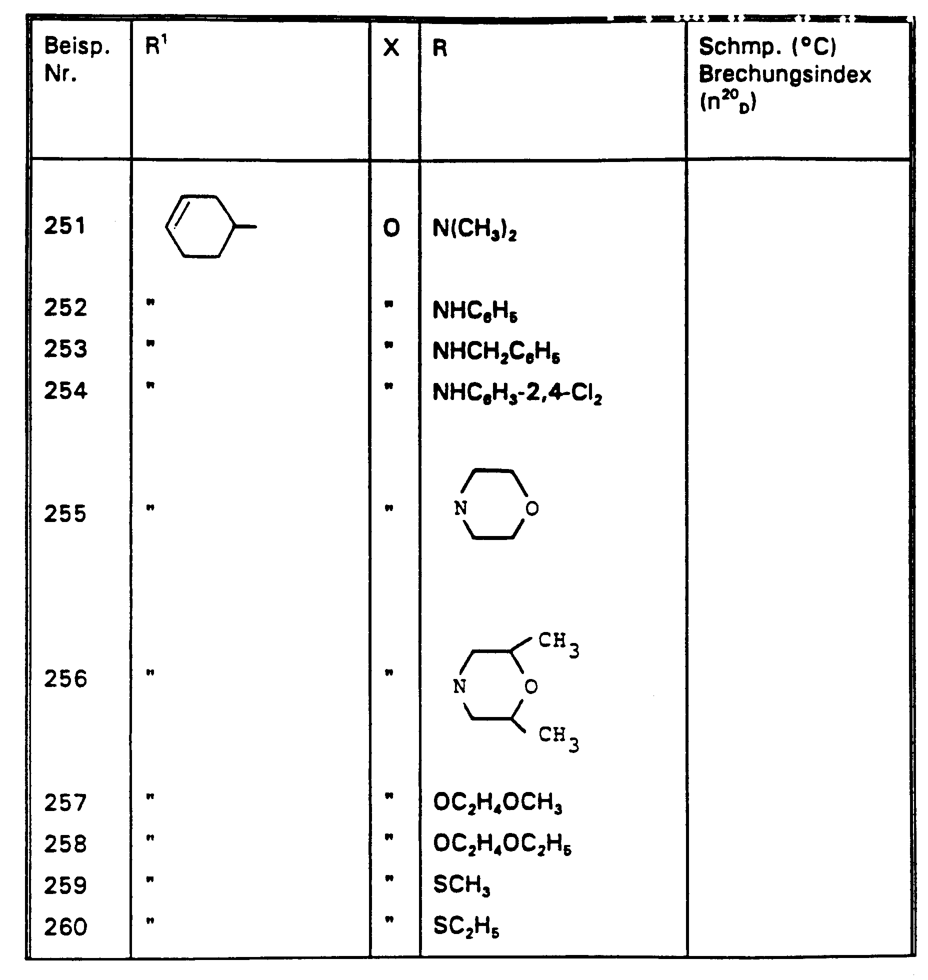

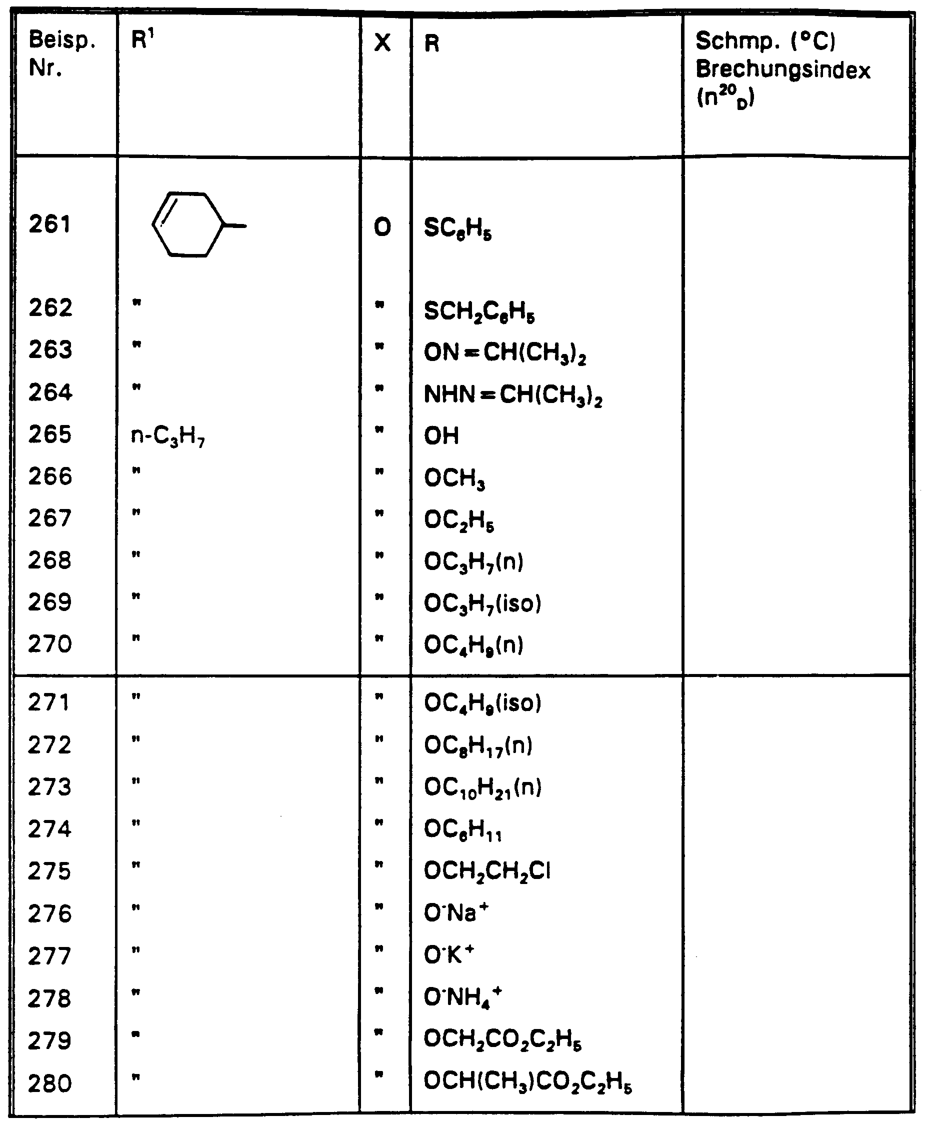

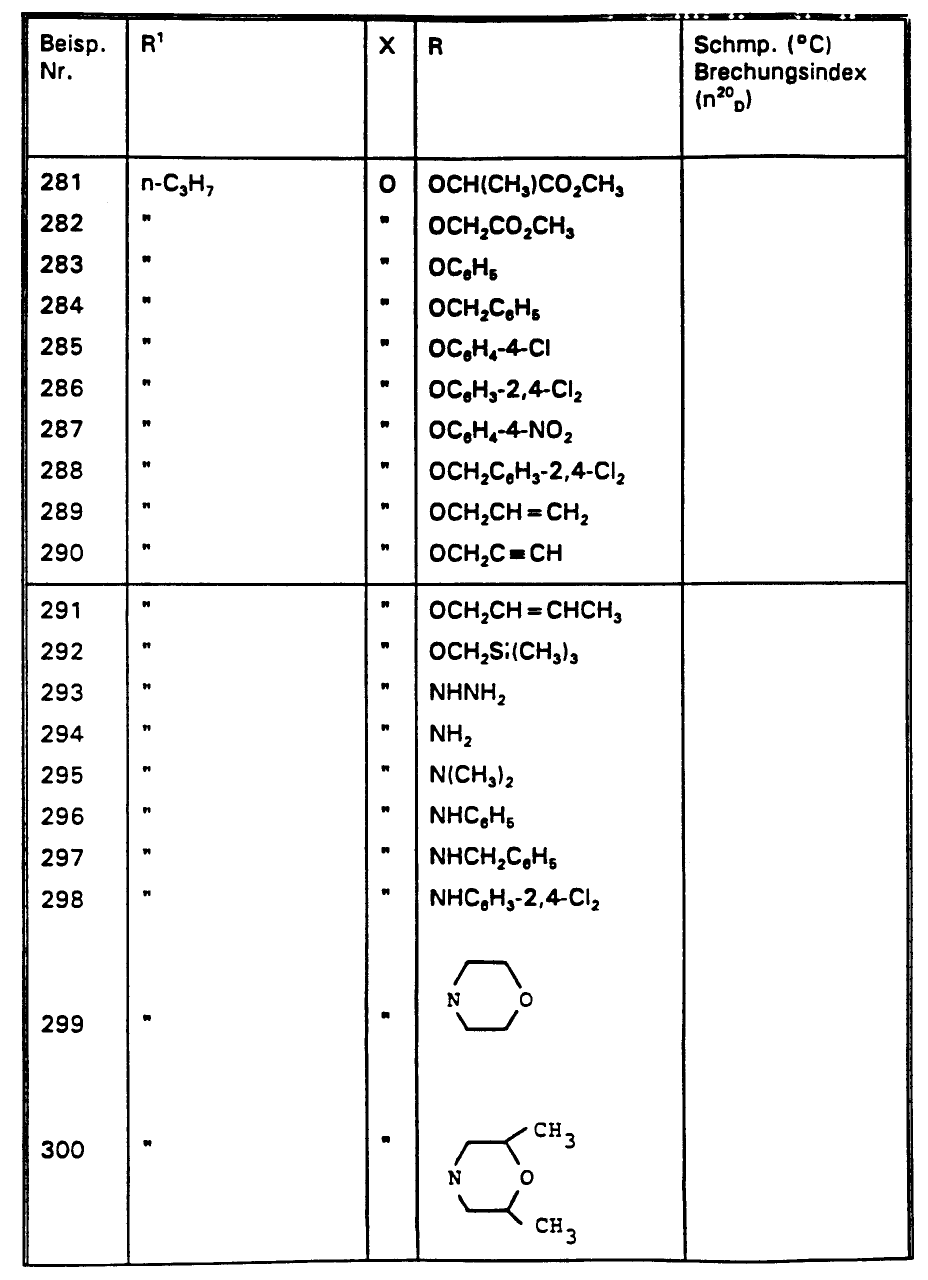

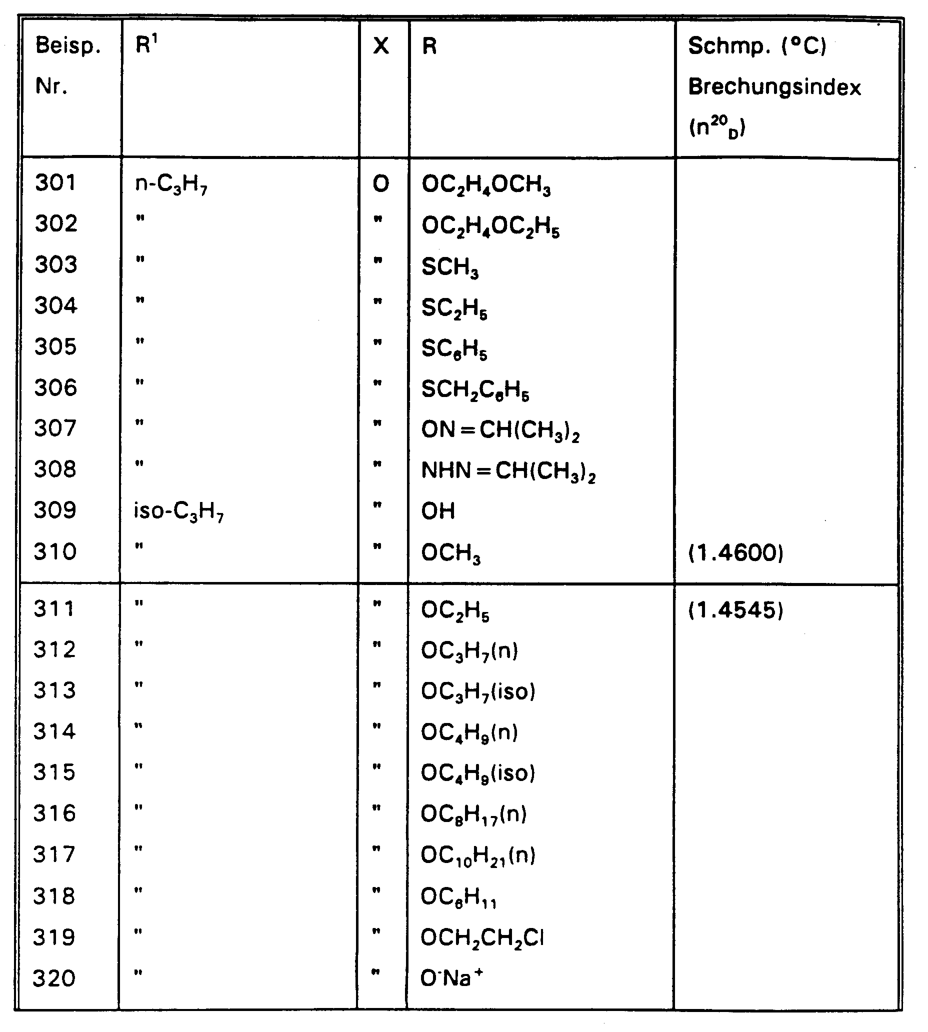

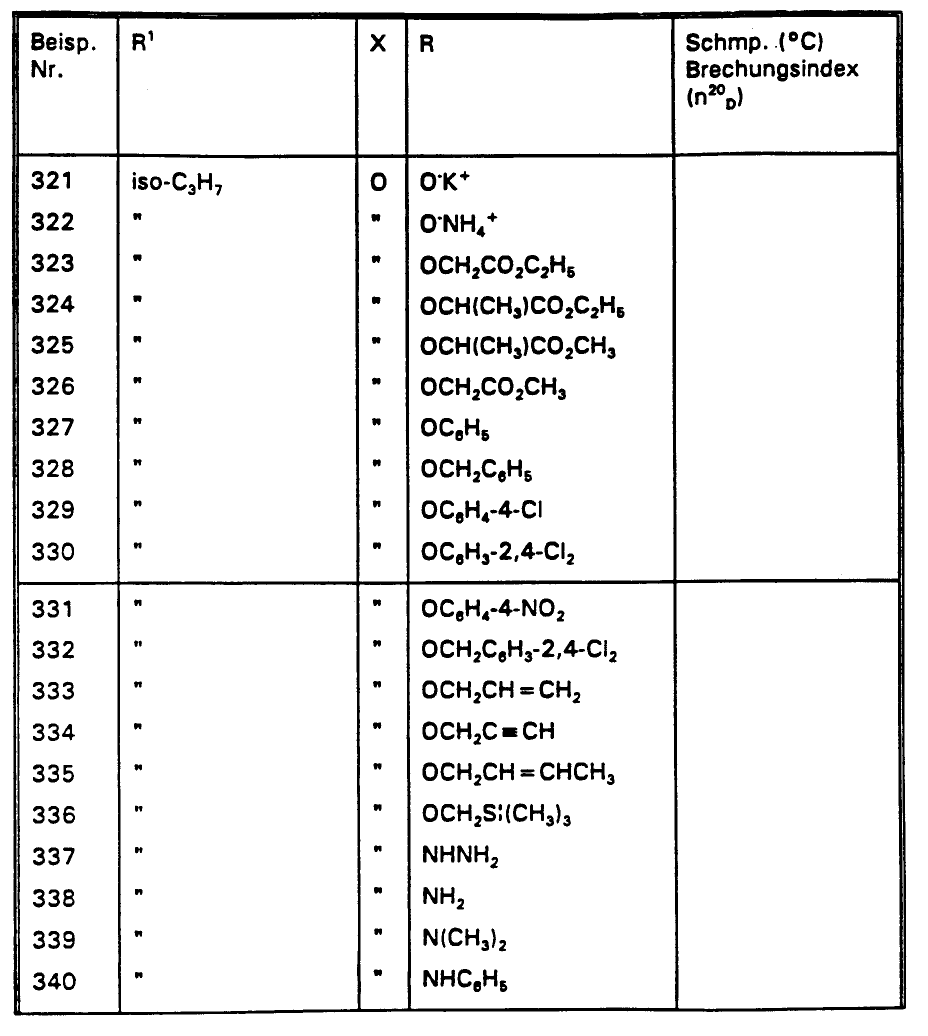

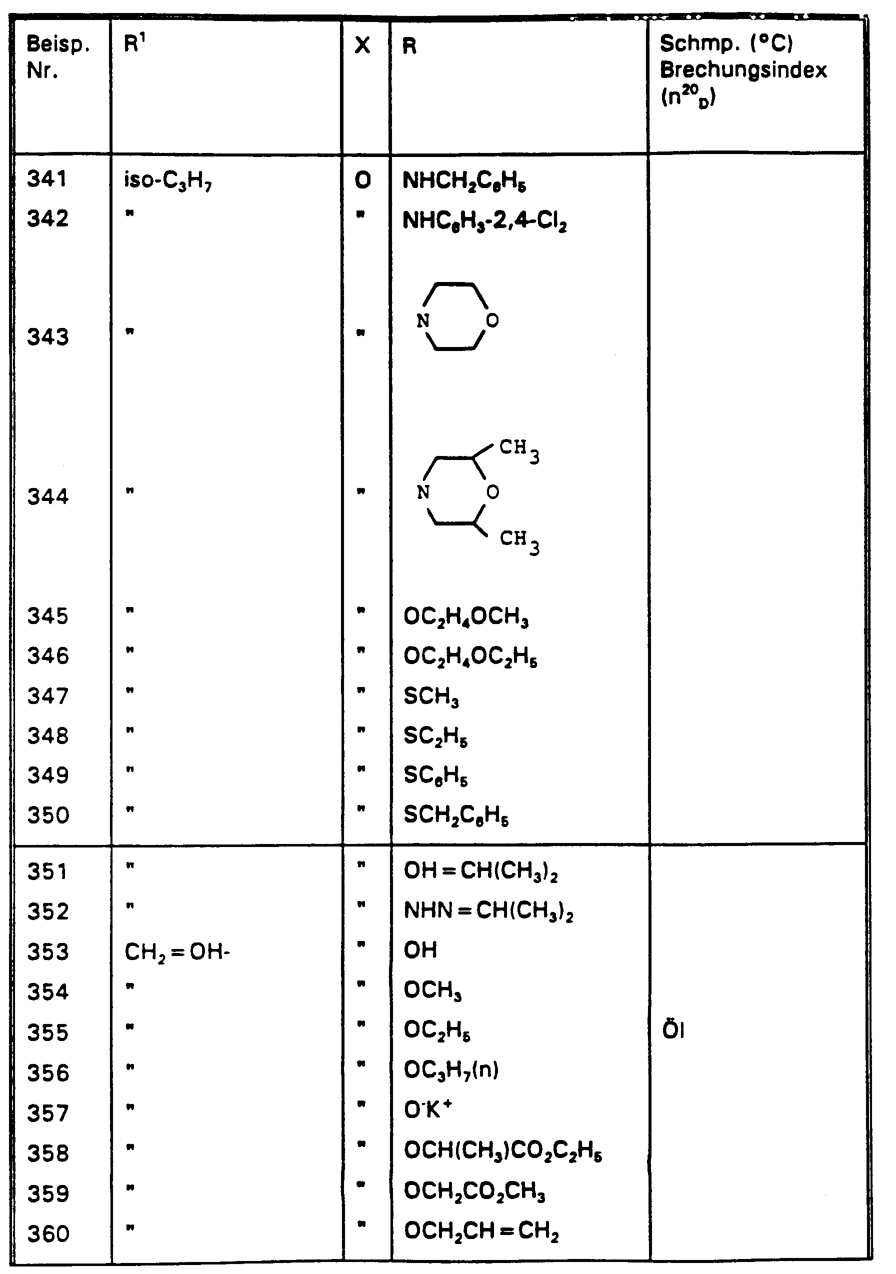

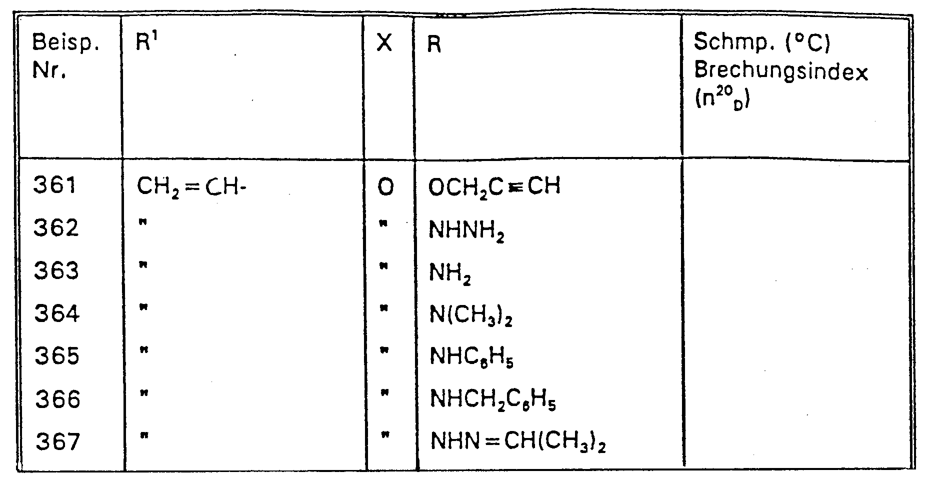

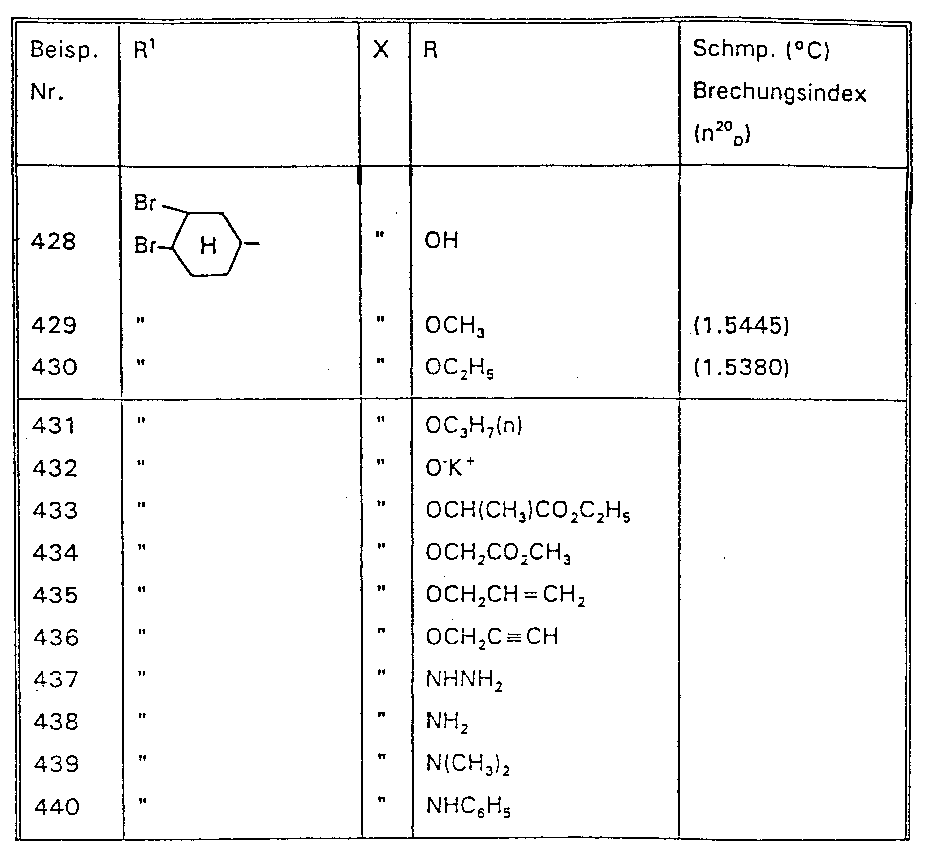

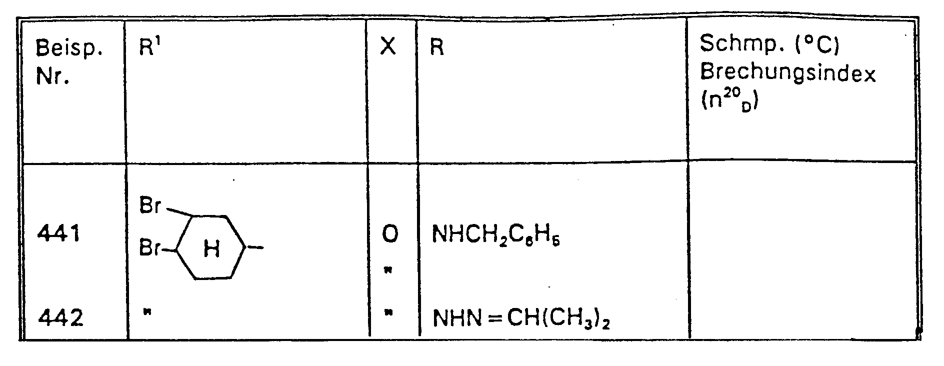

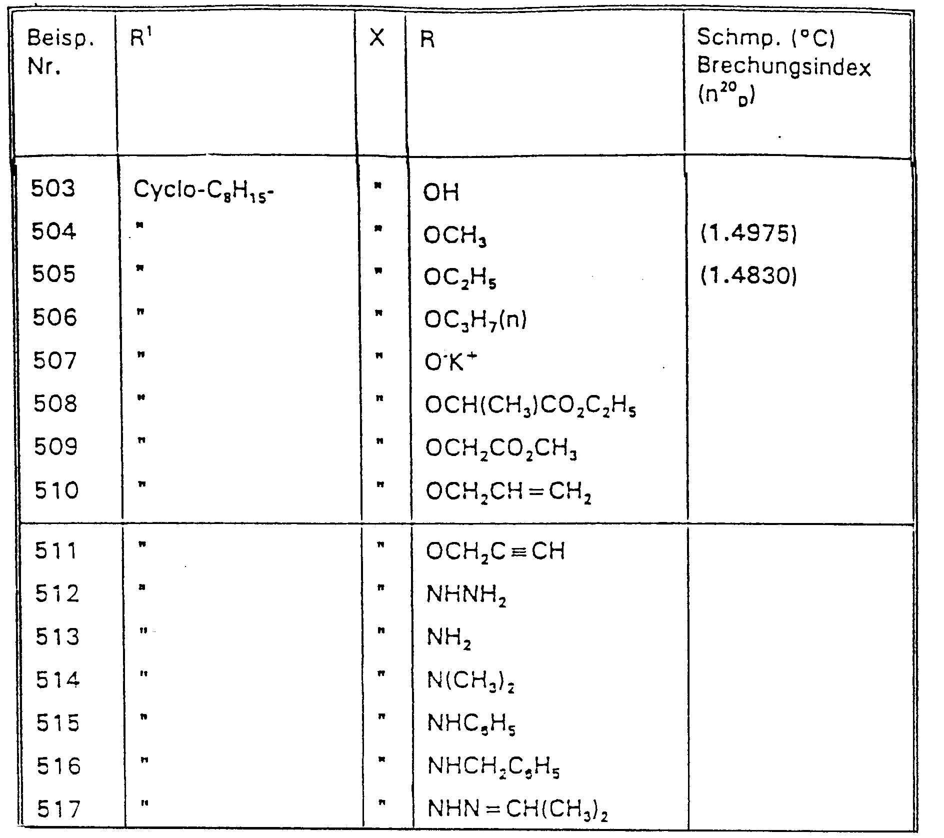

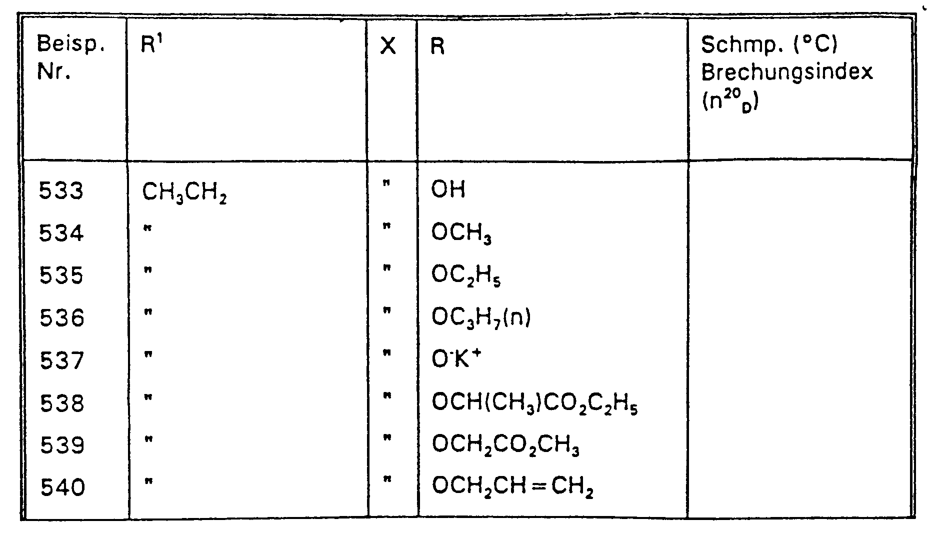

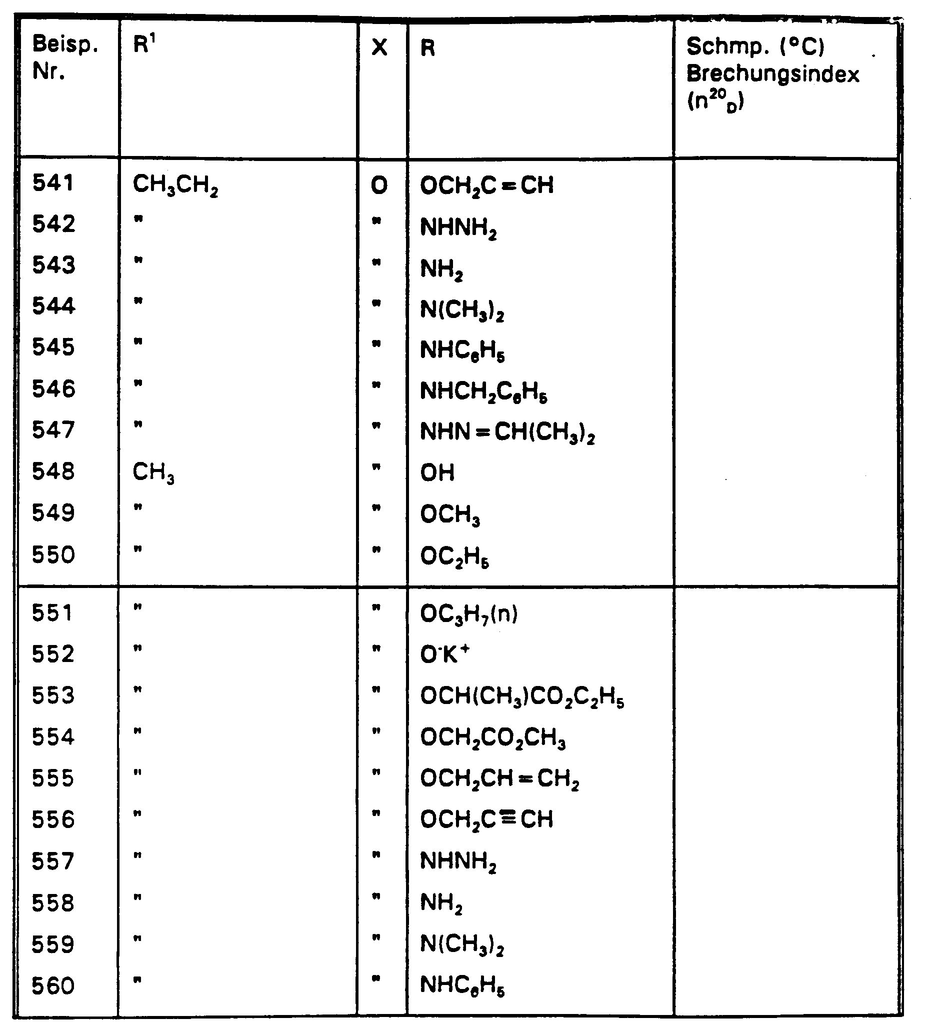

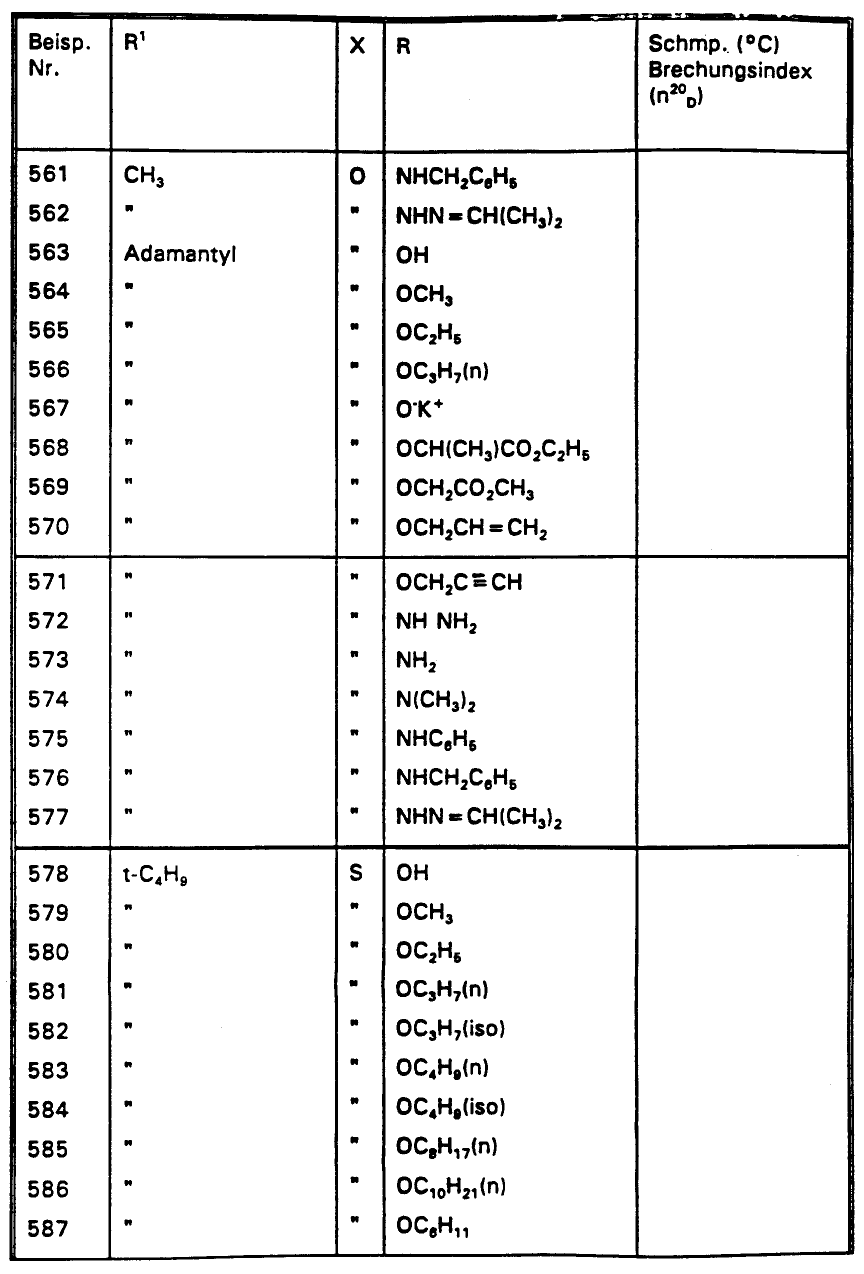

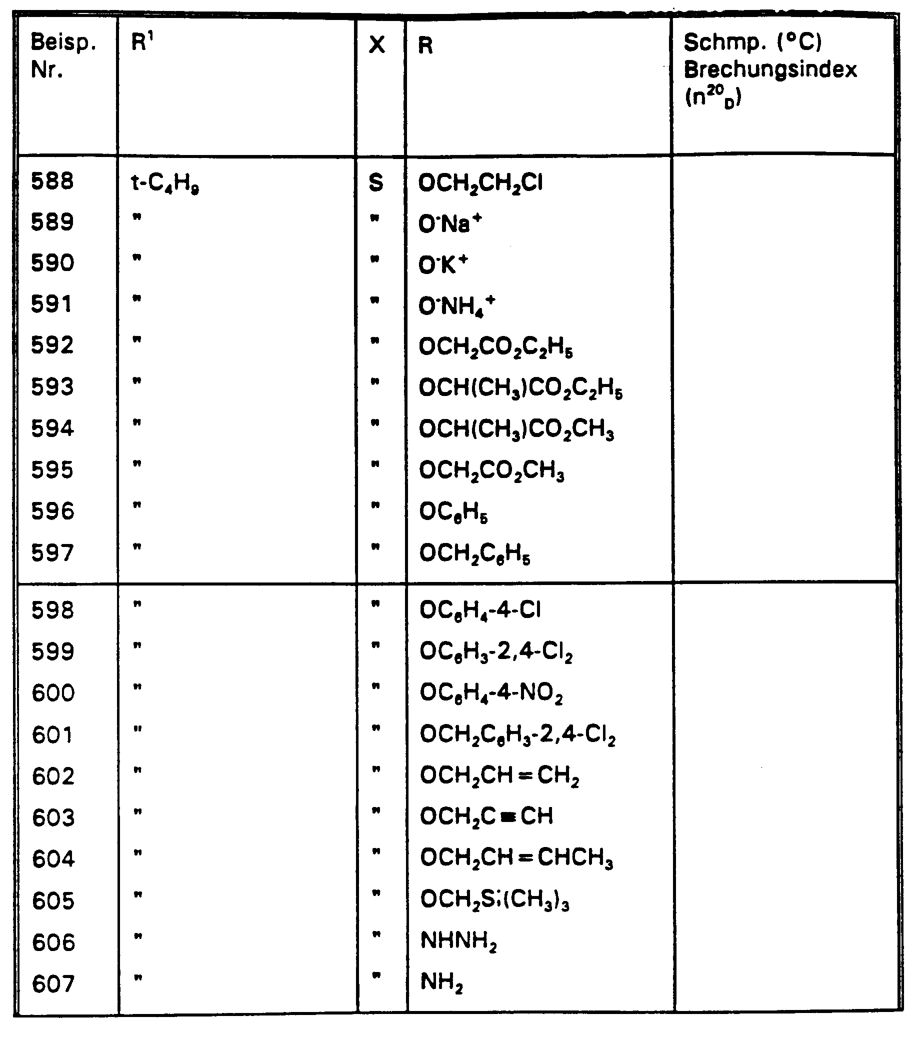

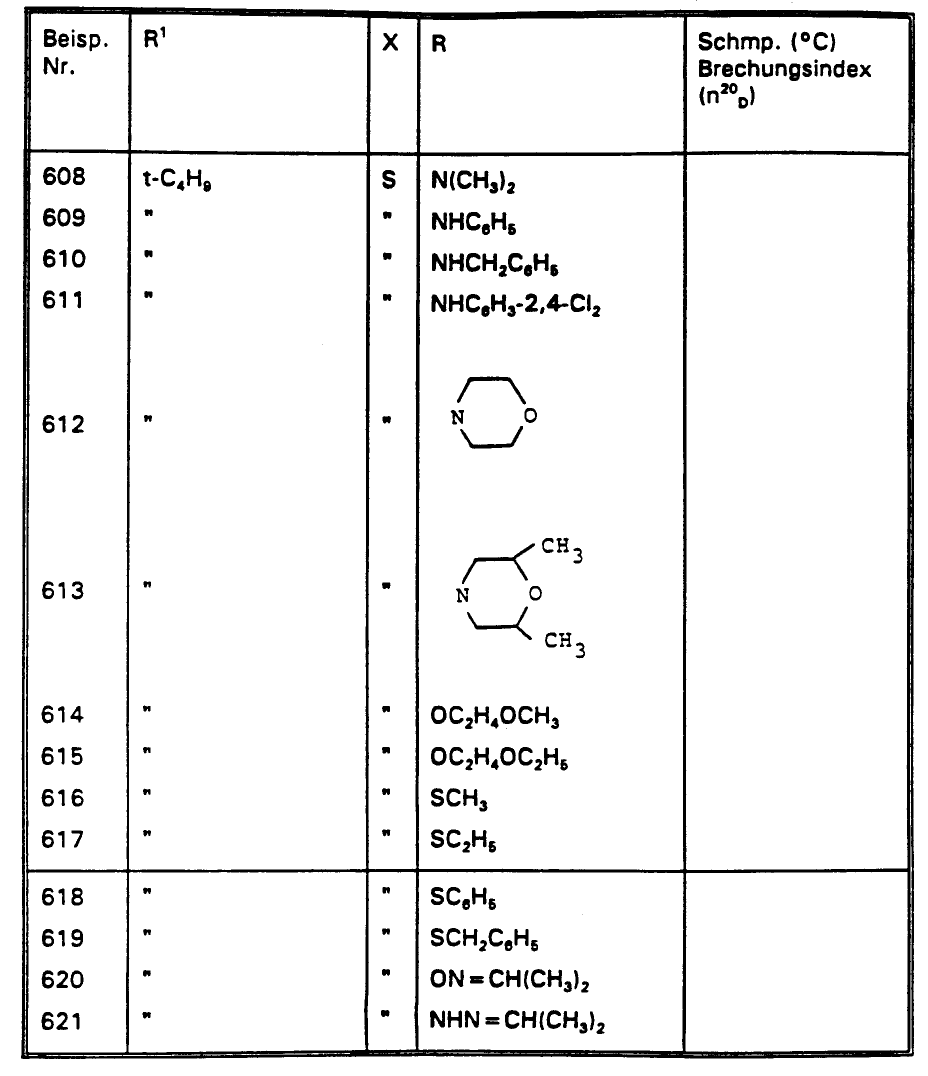

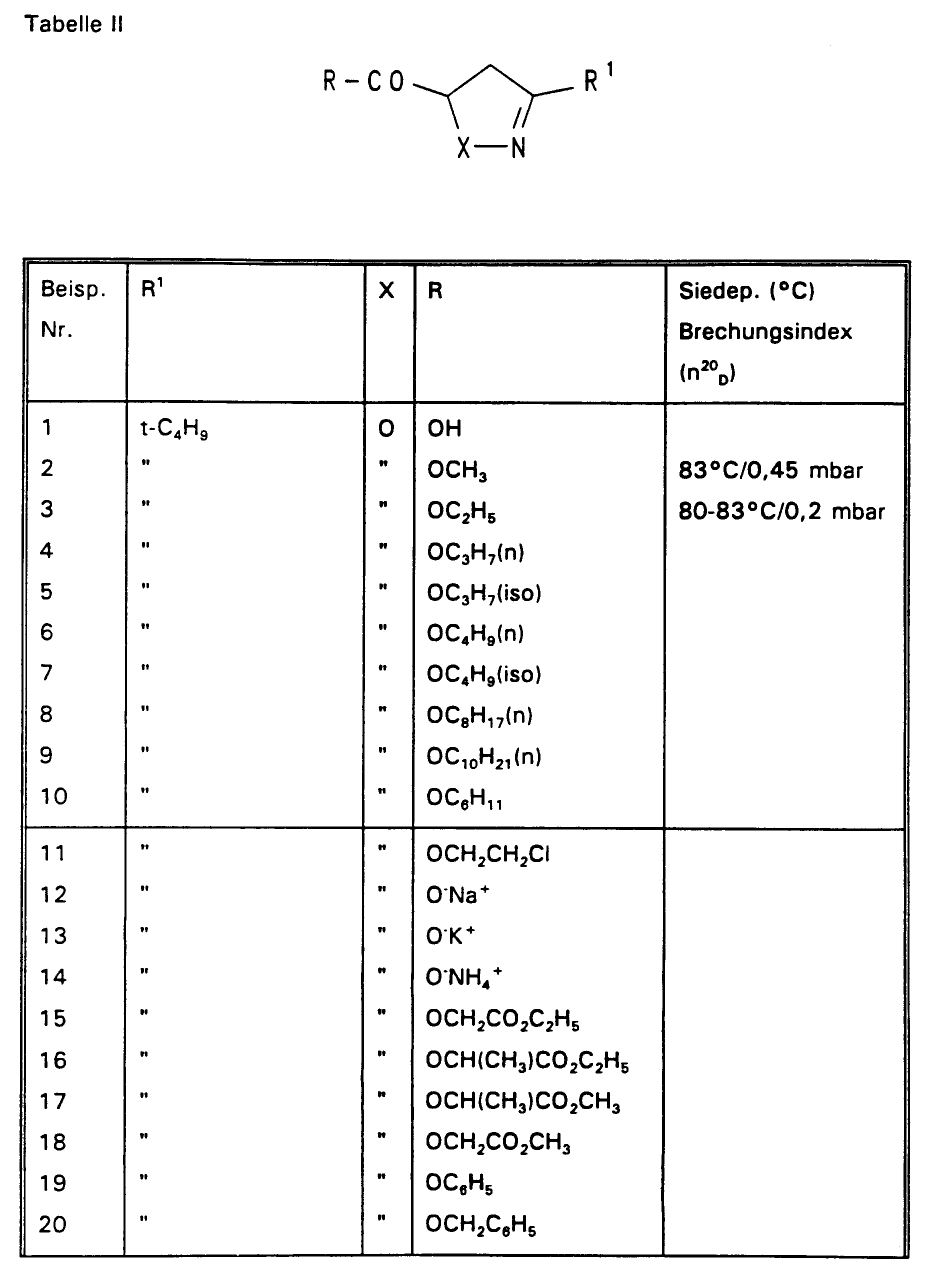

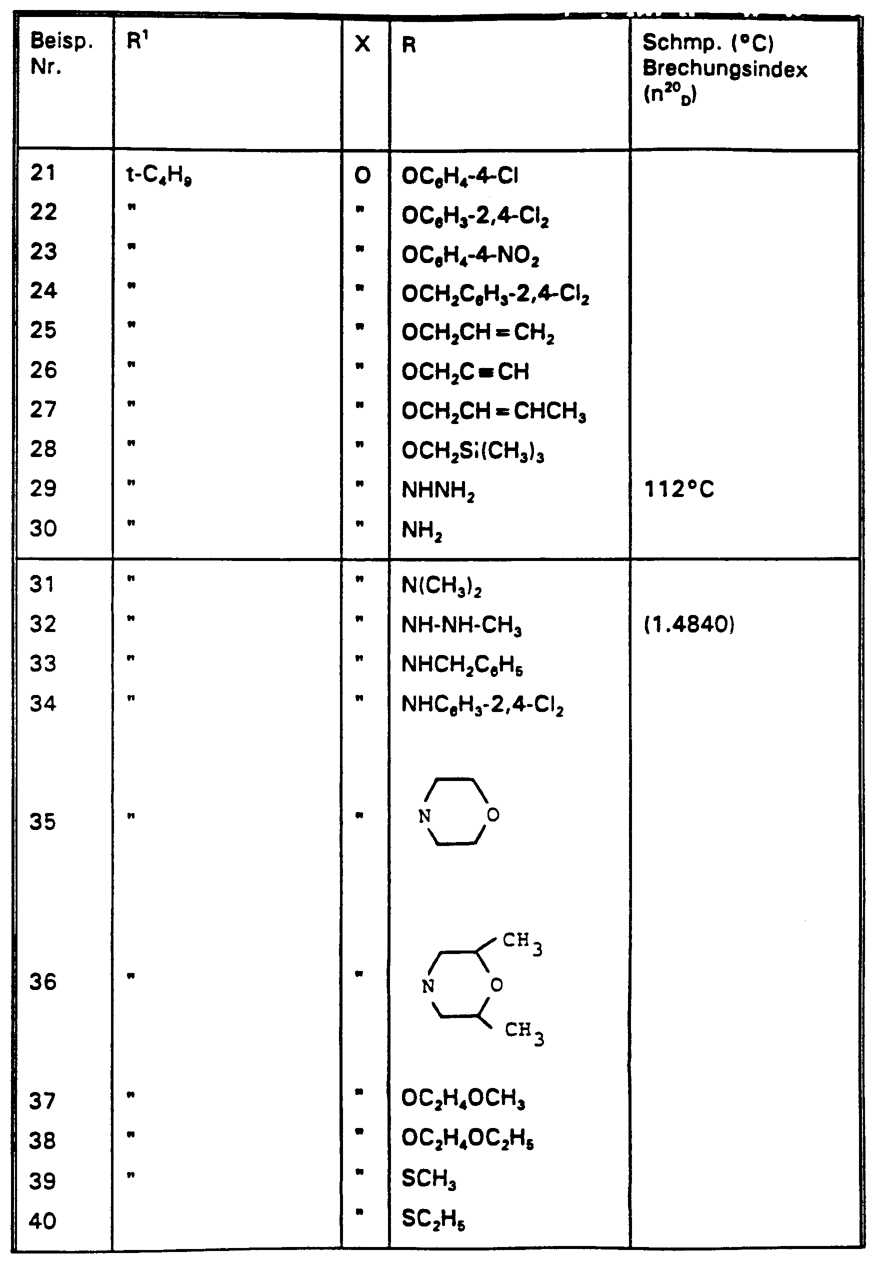

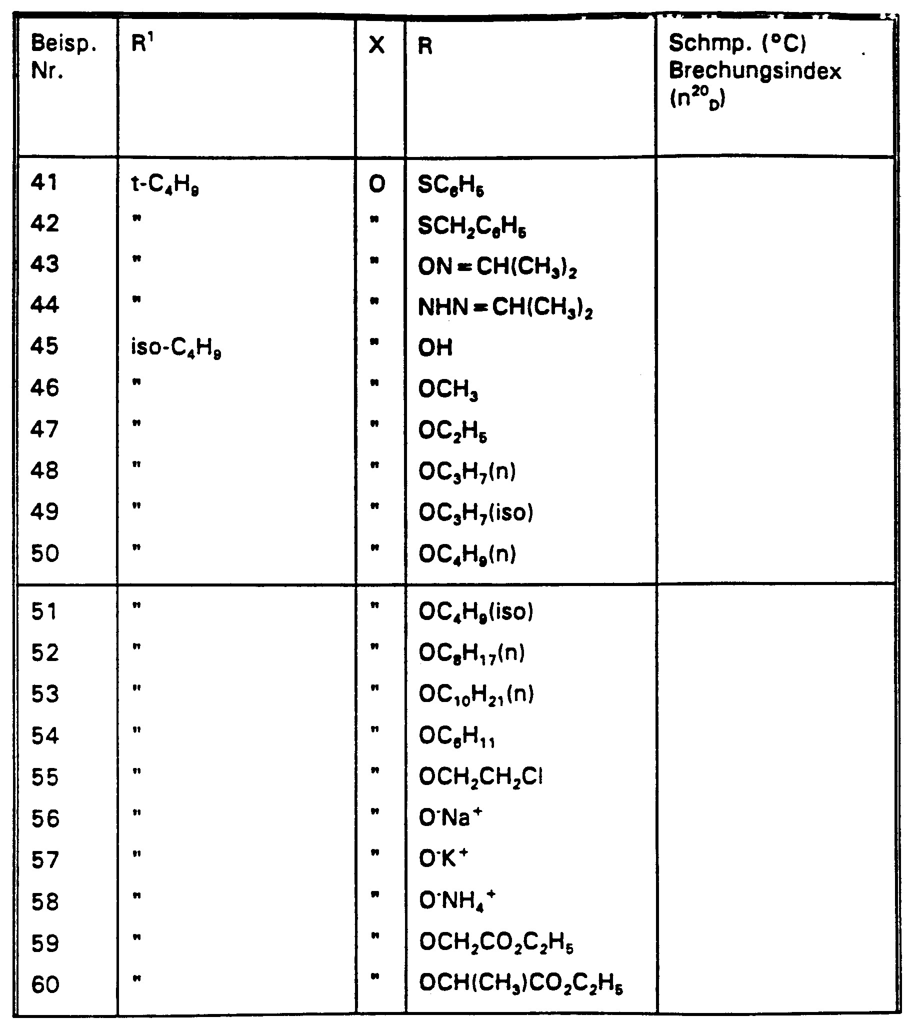

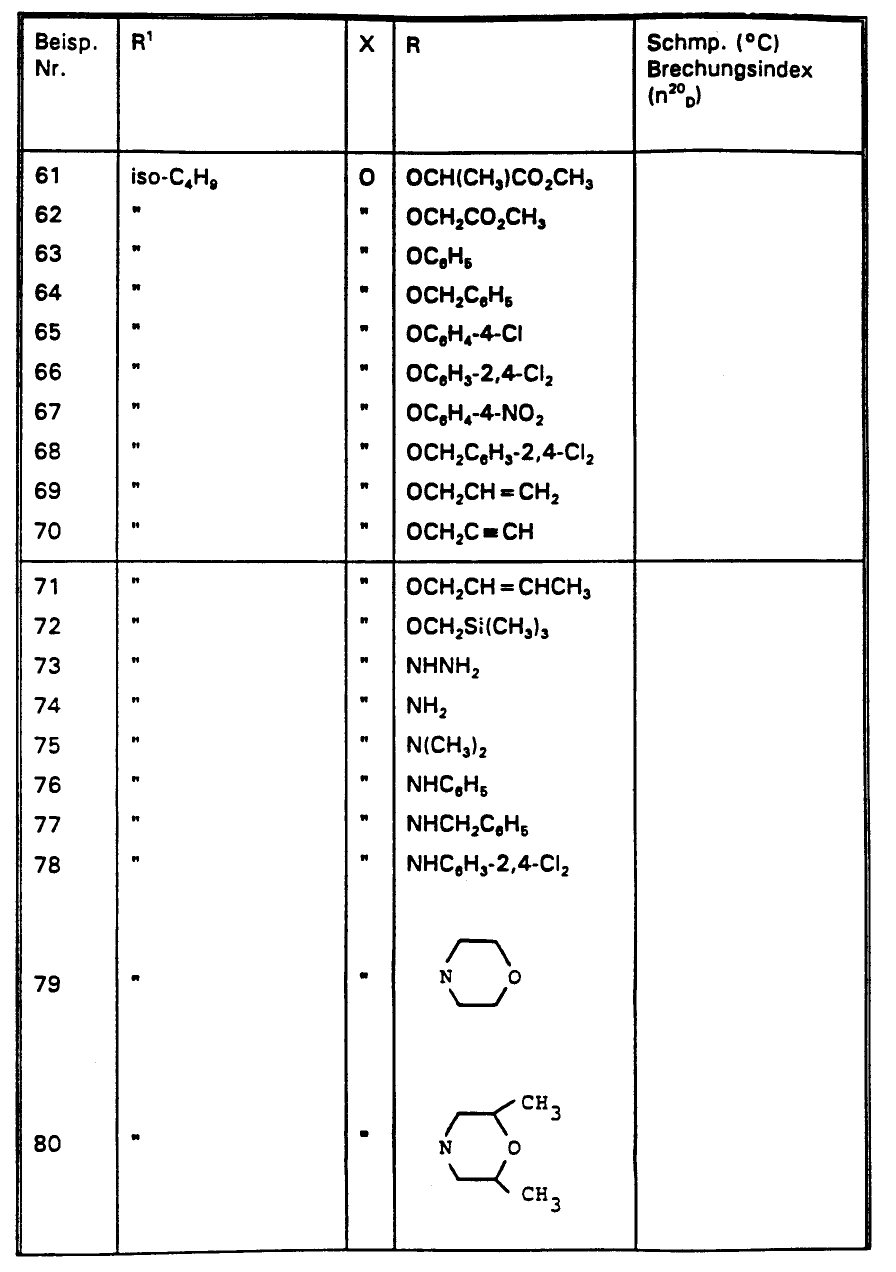

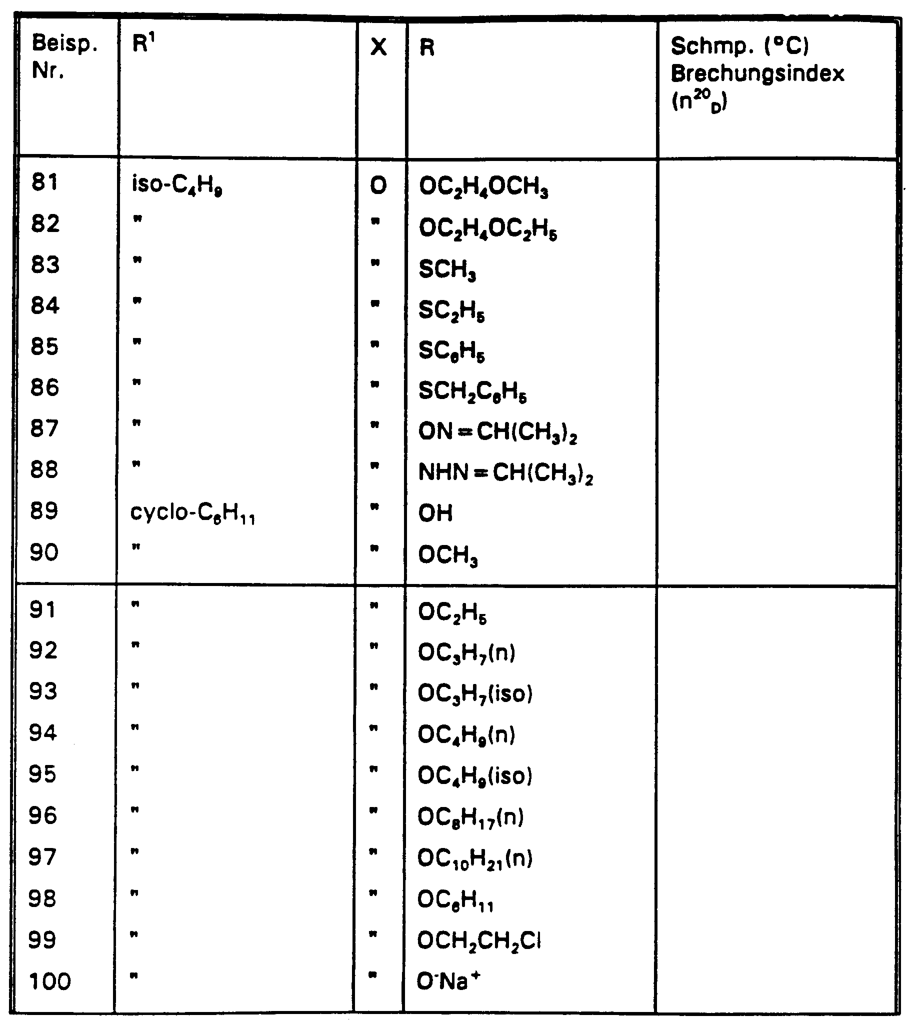

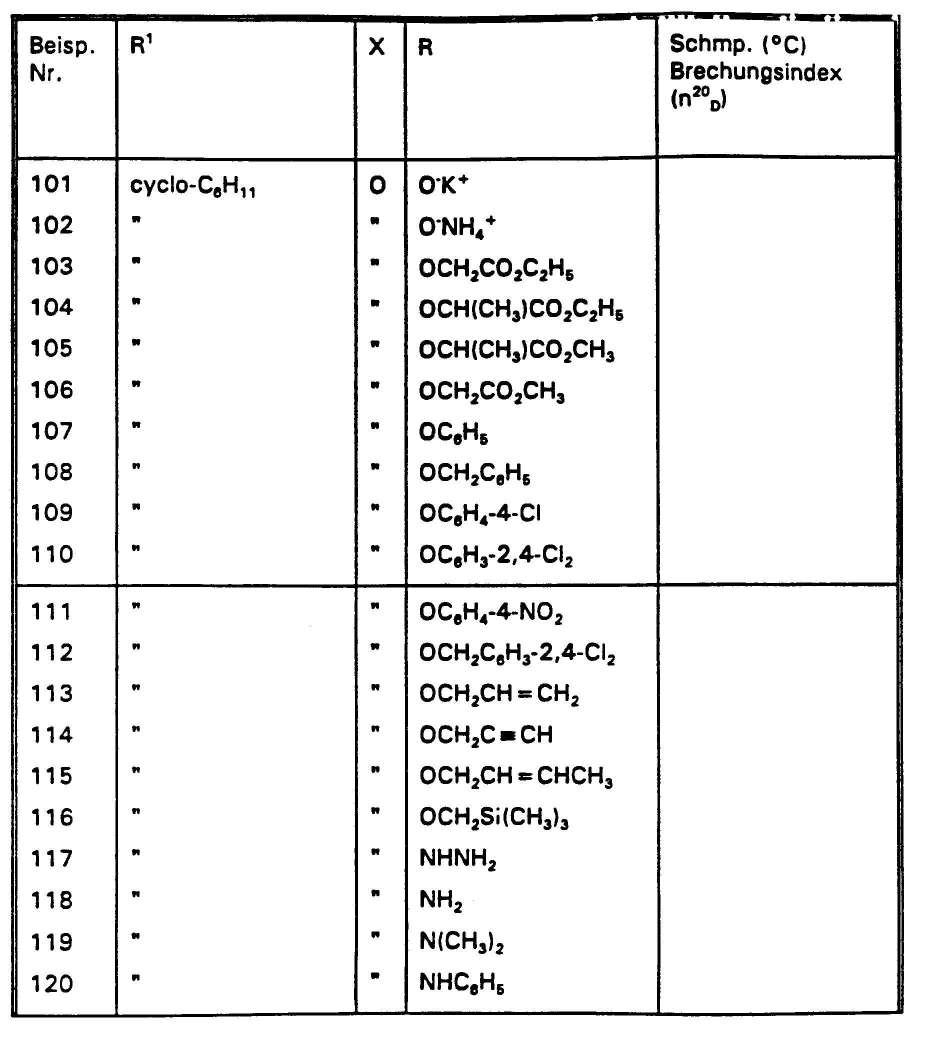

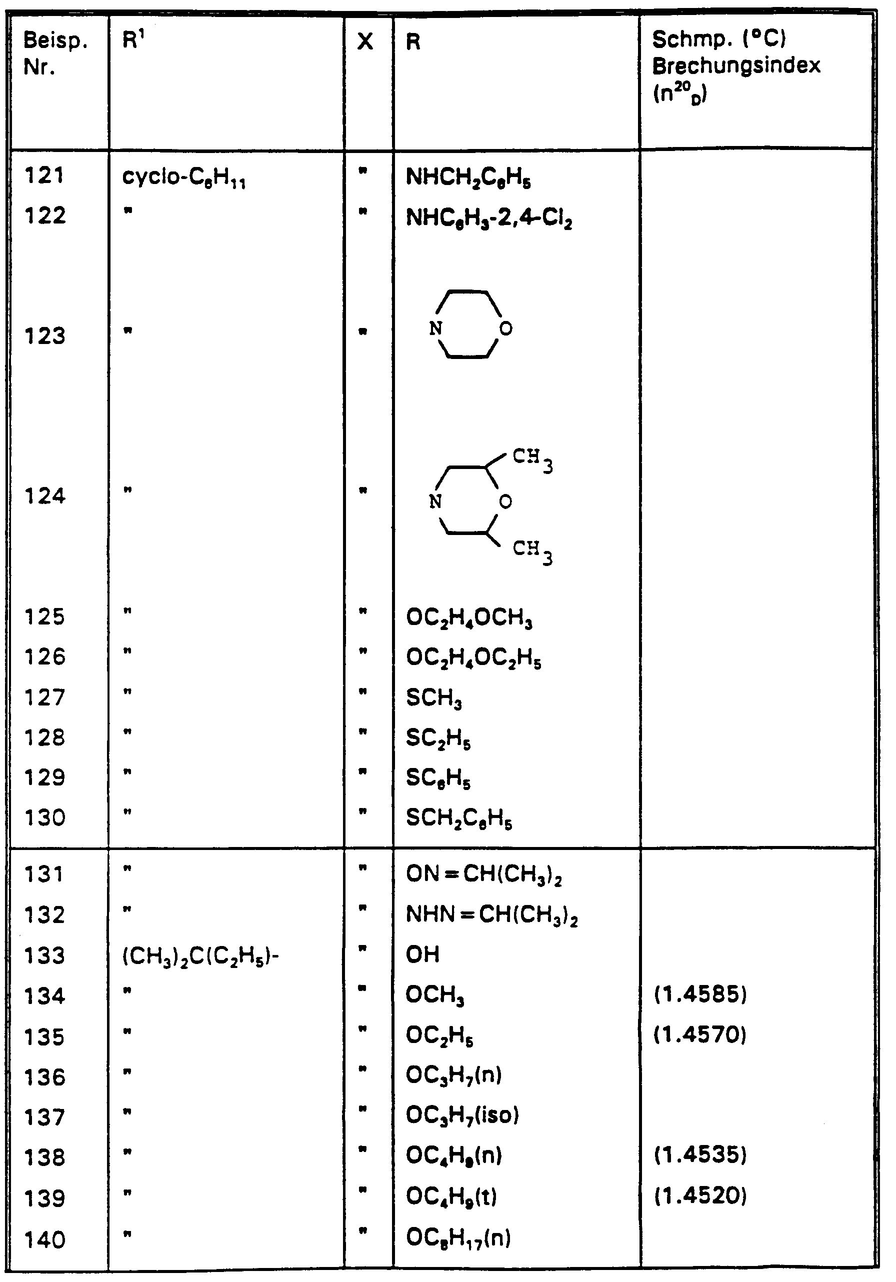

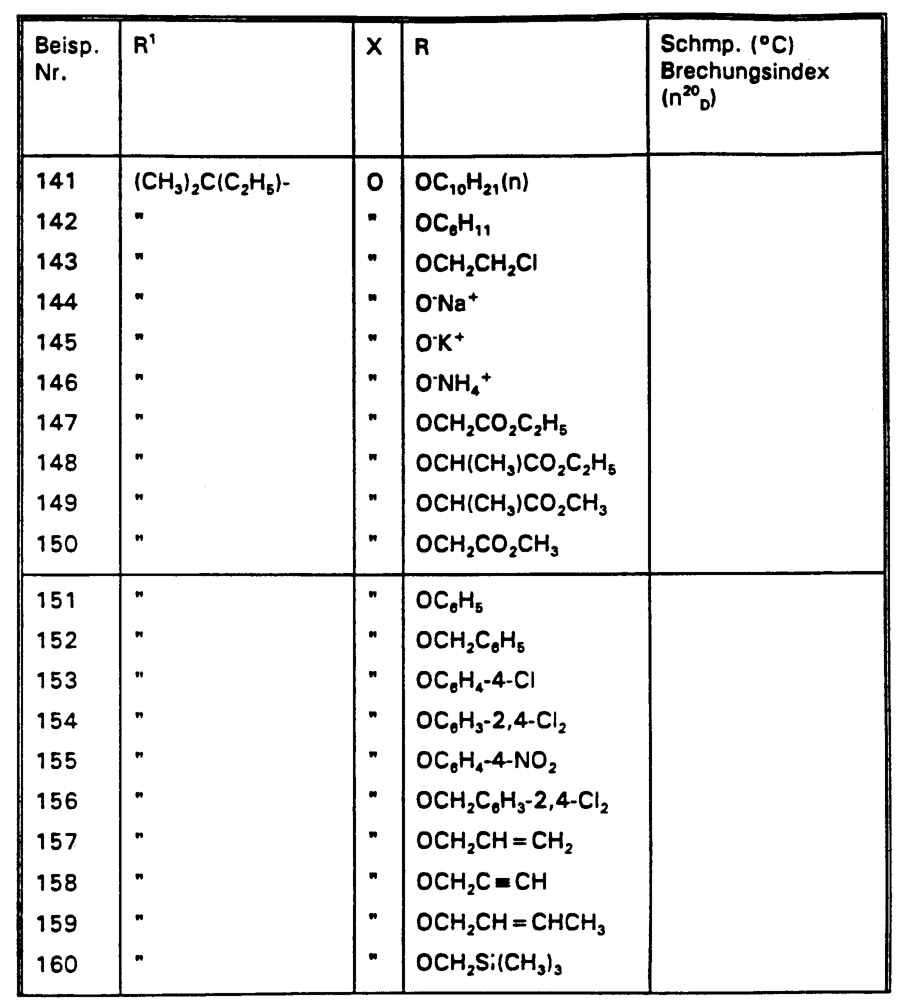

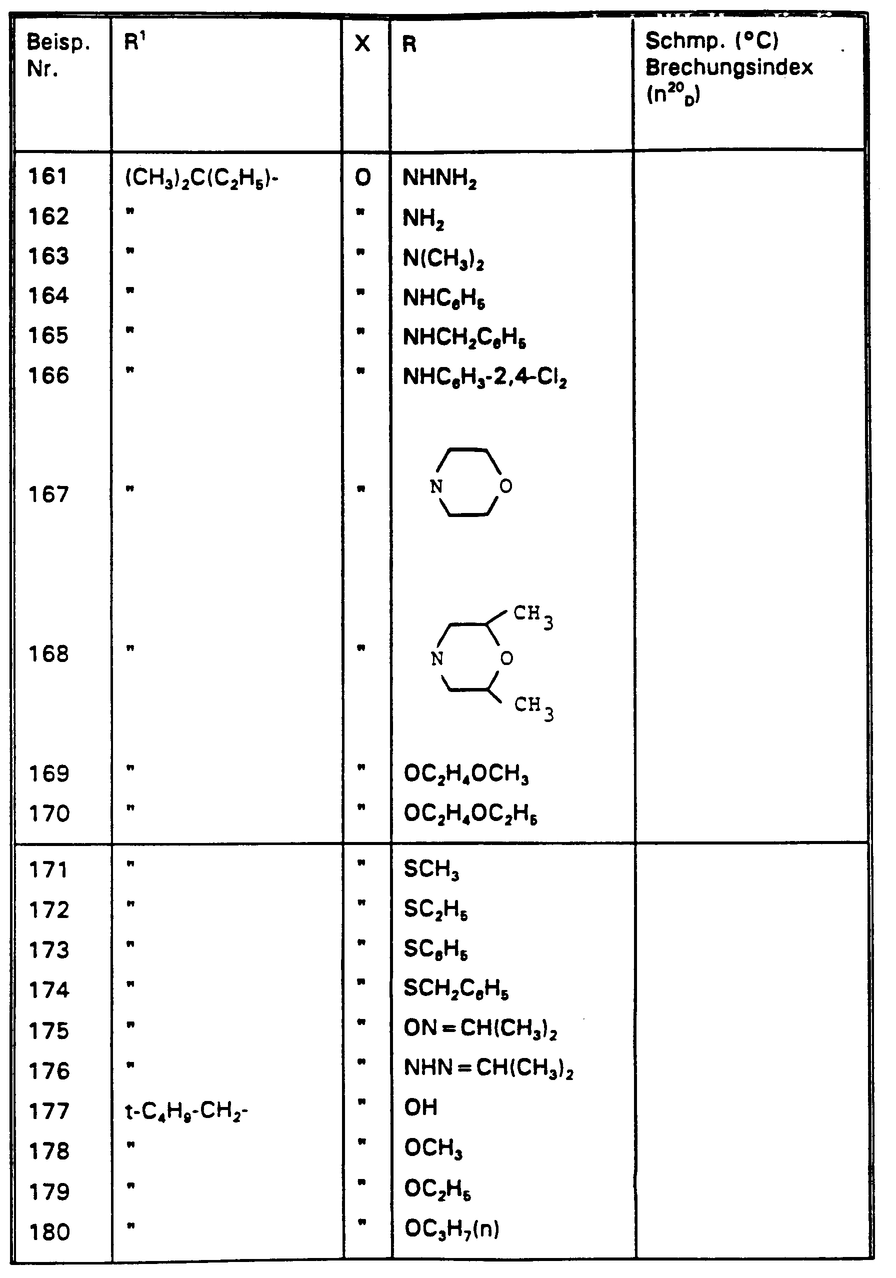

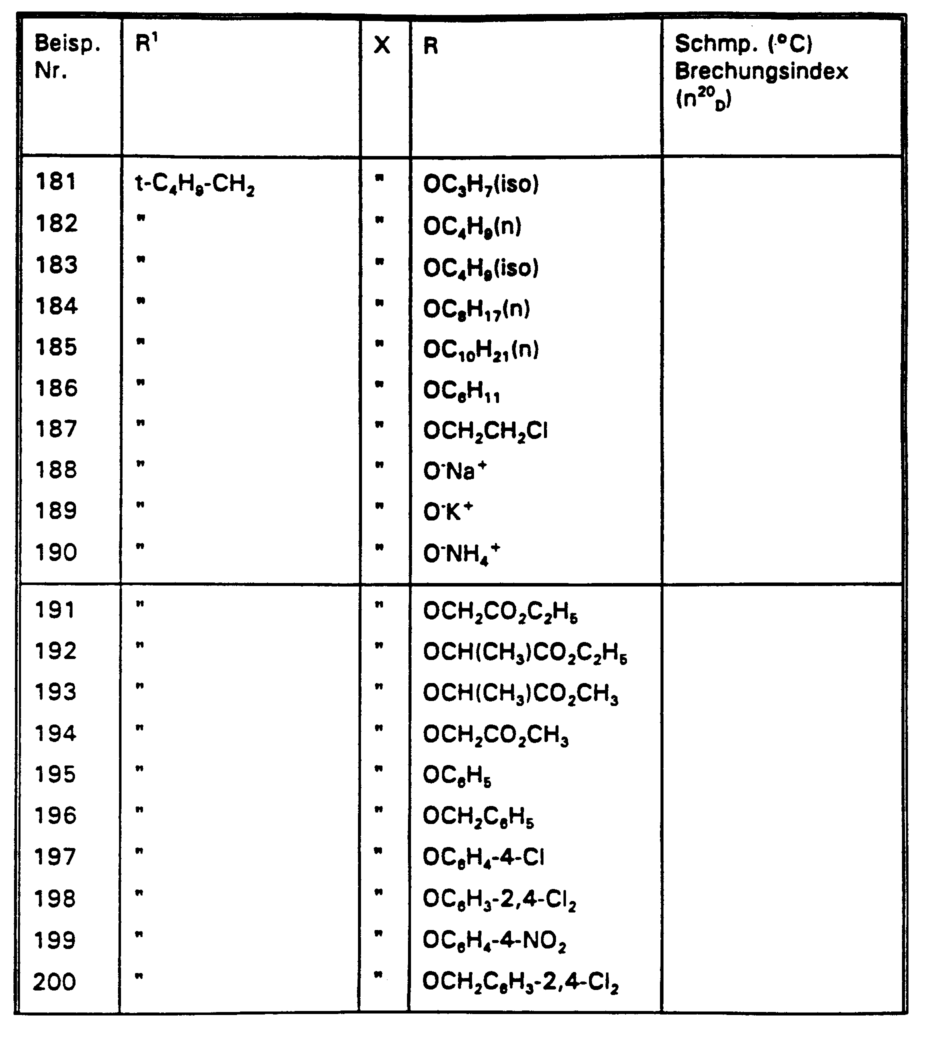

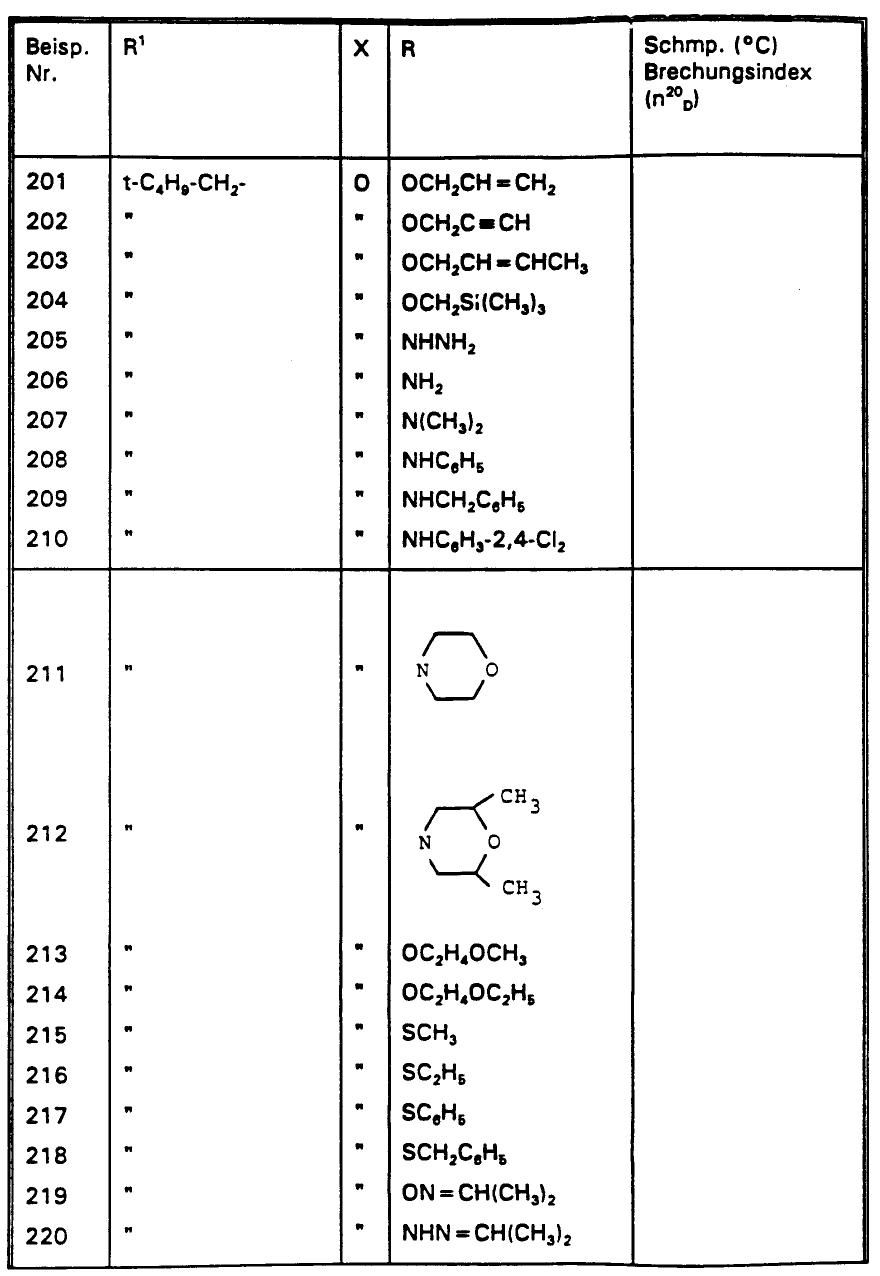

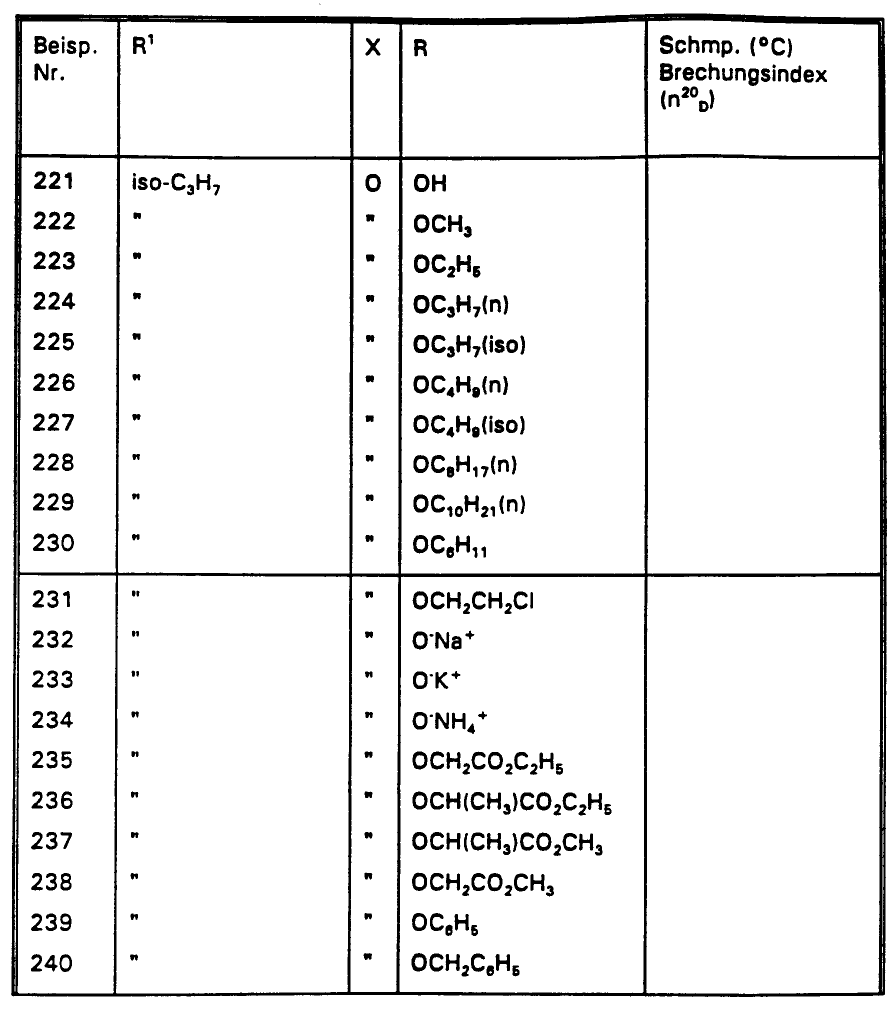

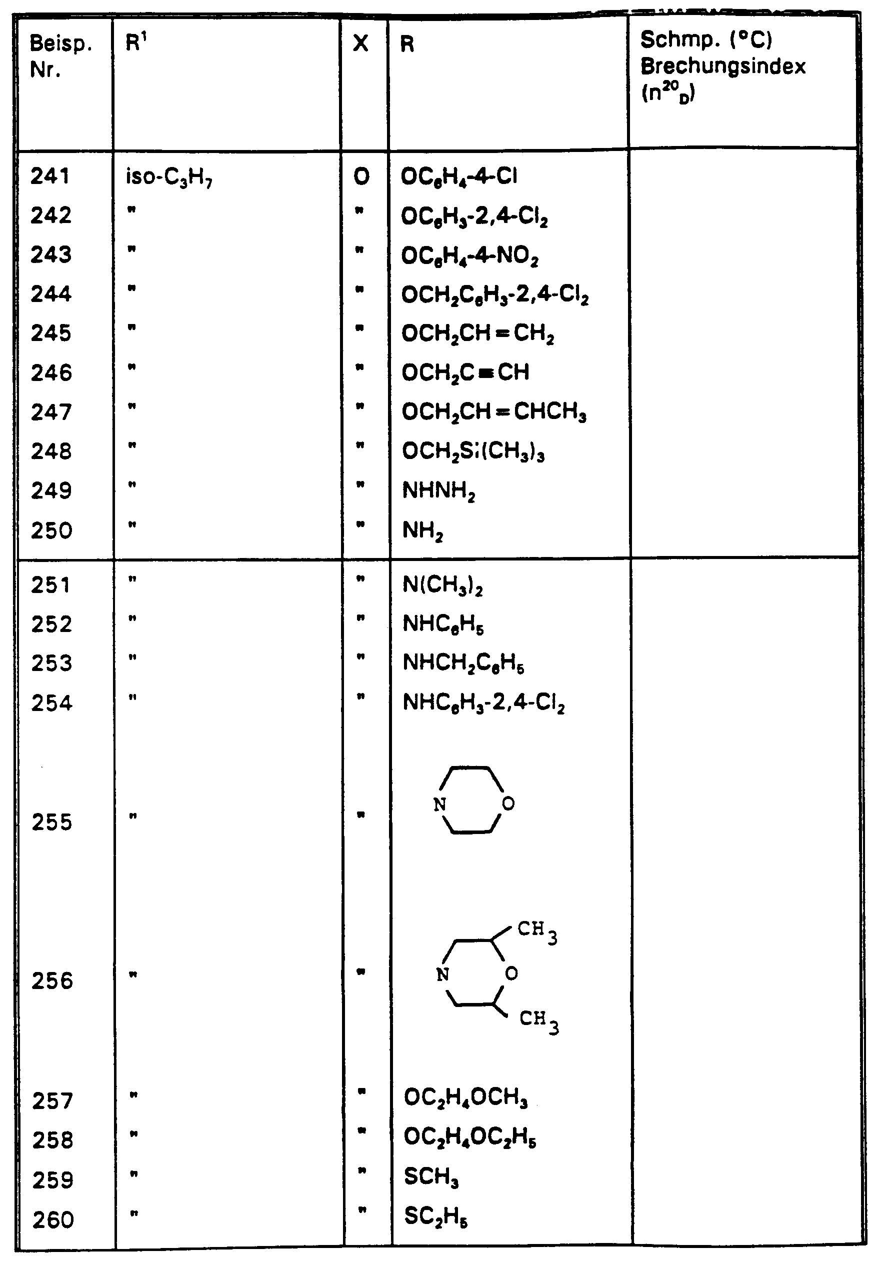

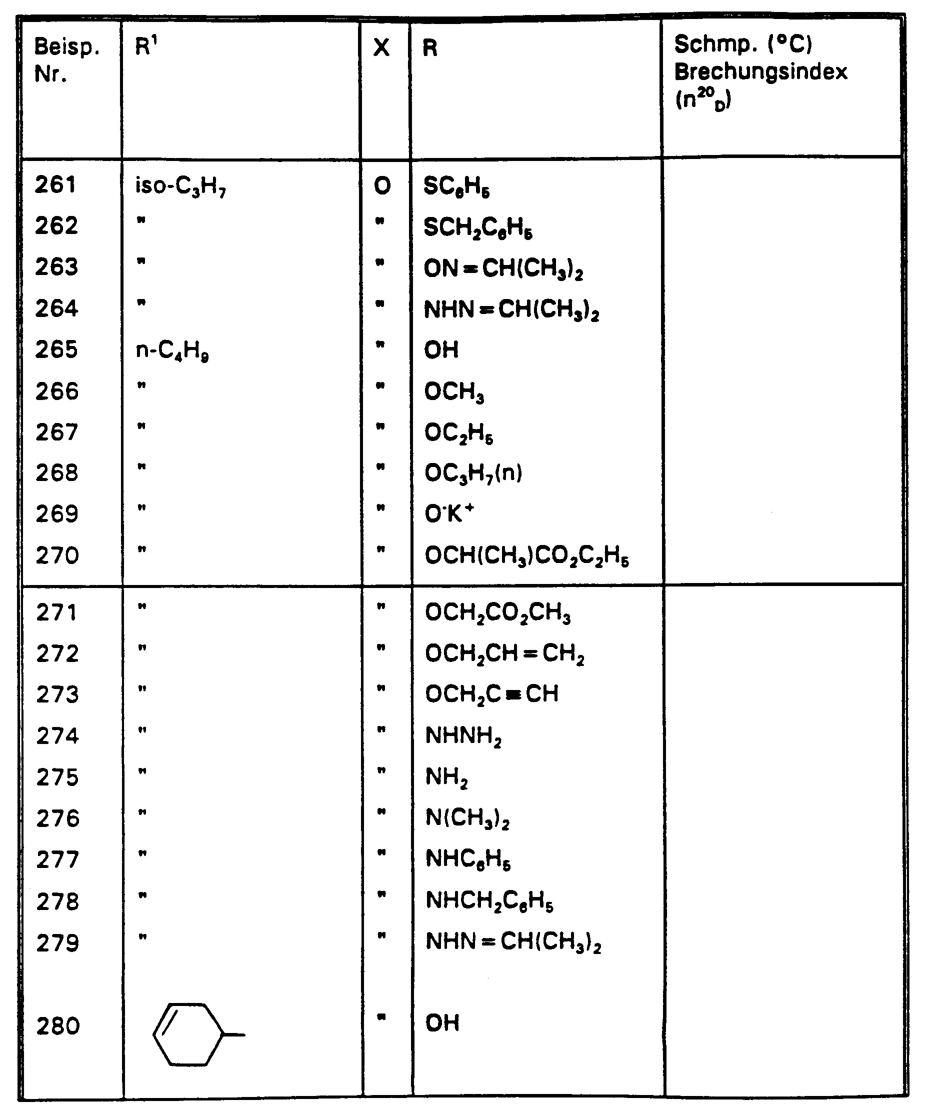

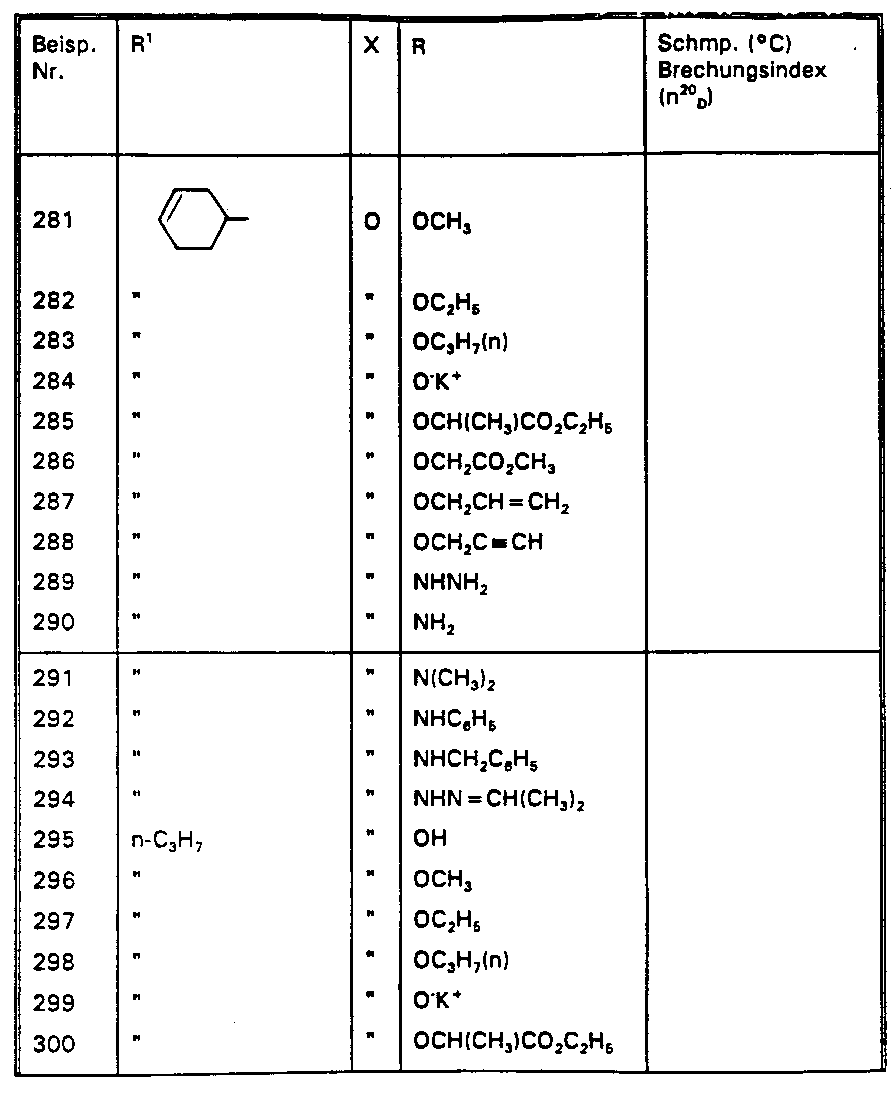

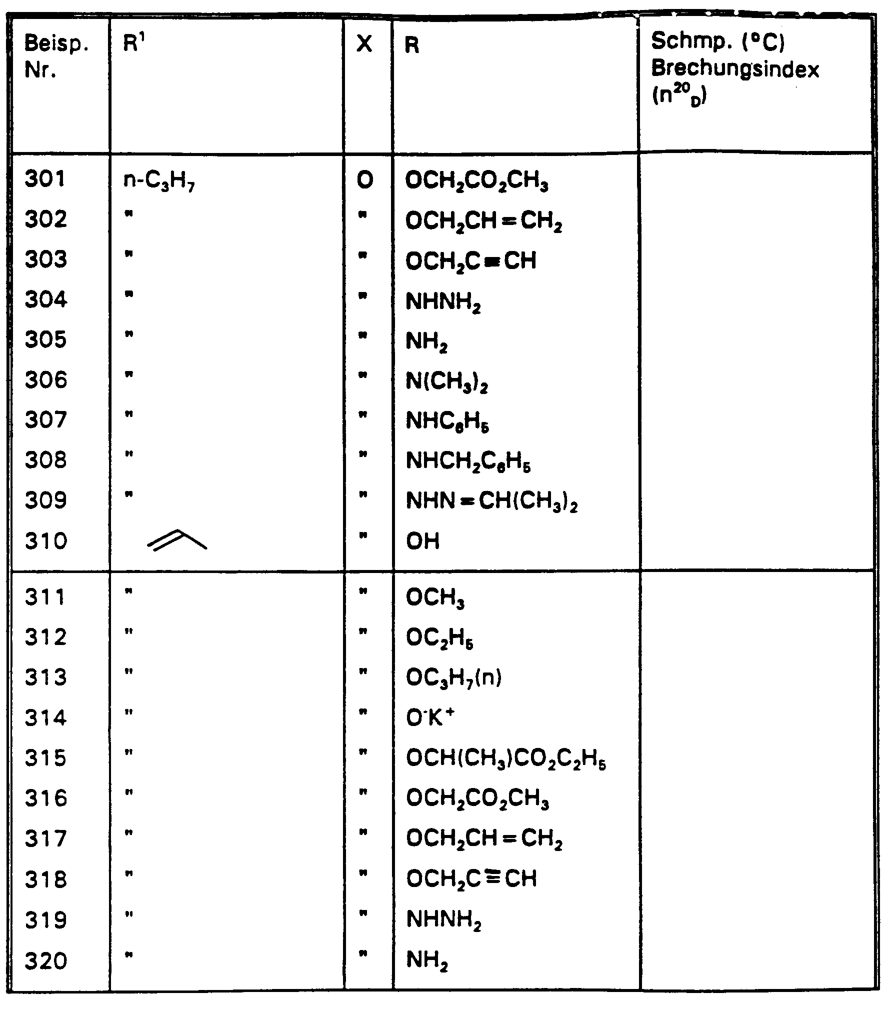

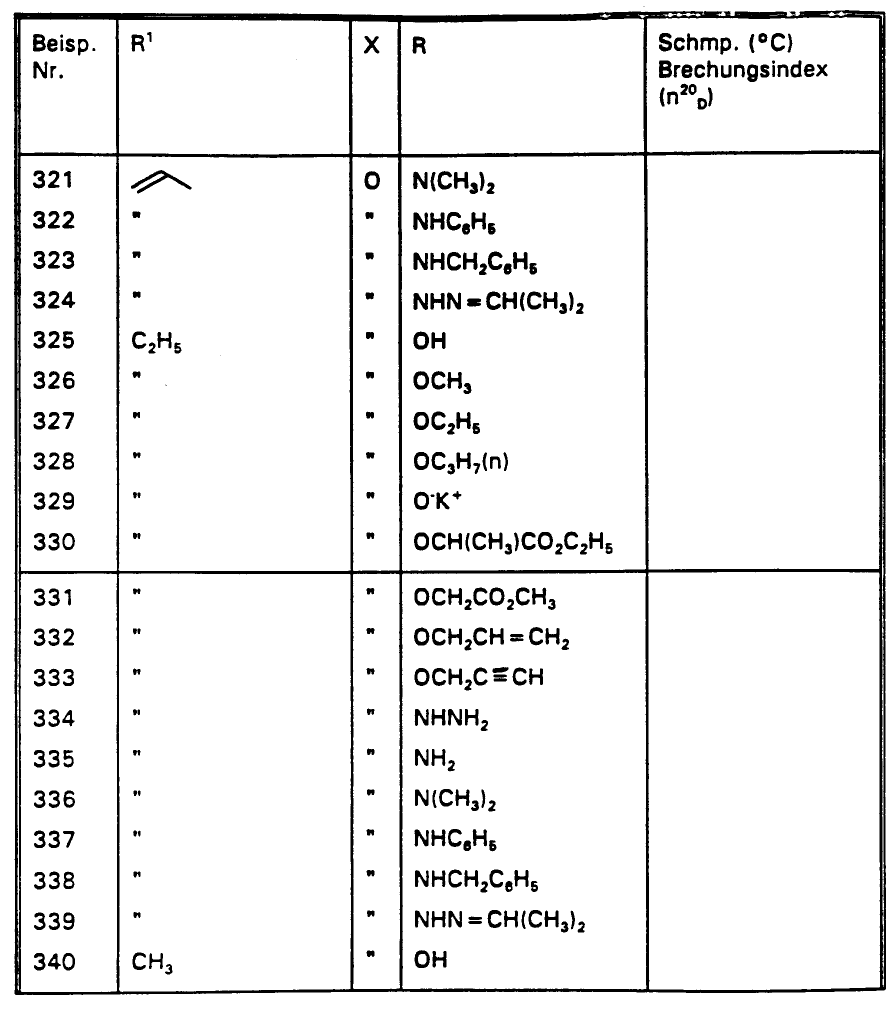

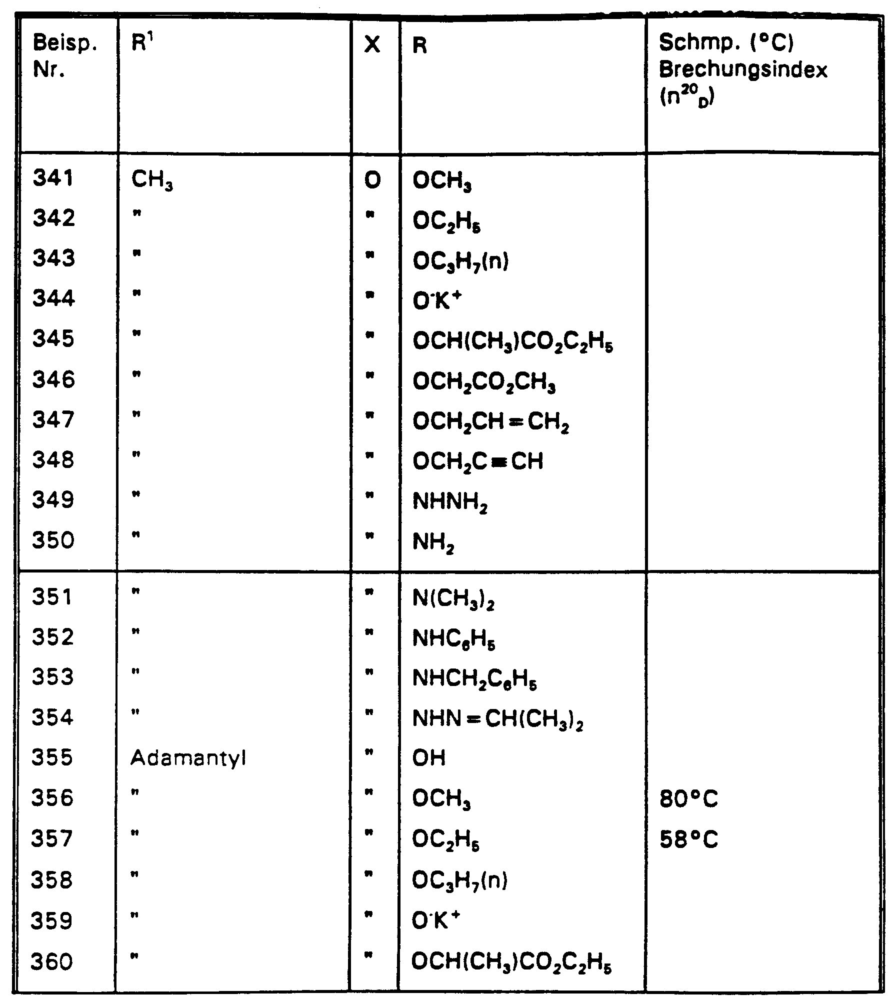

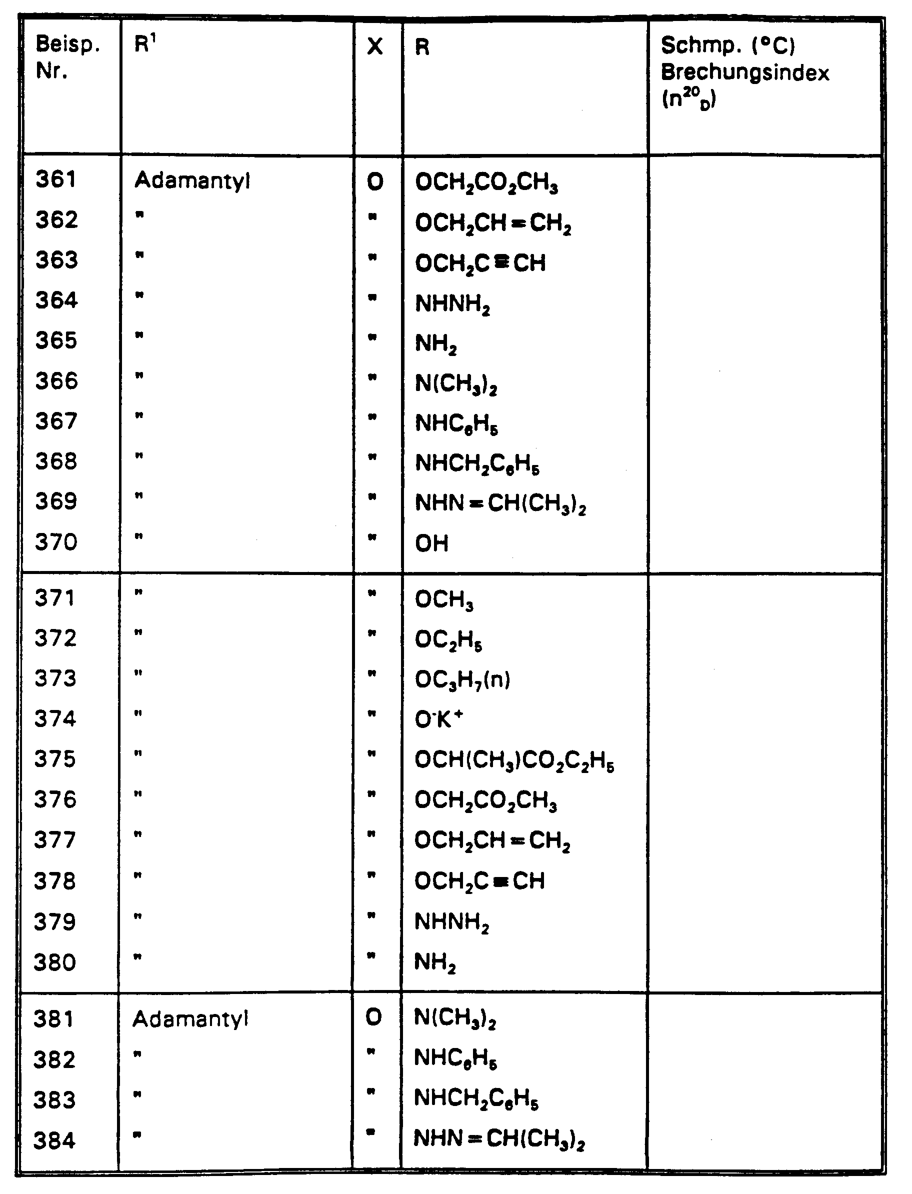

### Biochemischer Teil

Biochemische Identifizierung von Verbindungen mit Safenereigenschaften in Weizen und Gerste.

Aus der Literatur ist bekannt, daß Safener durch Beschleunigung des Abbaus des Herbizids in der Kulturpflanze wirken (Plant. Physical. (1971) 47, 10-14/Pesticide Biochemistry and Physiology 37, 211-218 (1990)).

Im wesentlichen werden Herbizide entweder durch Hydroxylierung oder durch Konjugation mit Glutathion entgiftet. Weitere Umsetzungen sind möglich, spielen jedoch für den entscheidenden, entgiftenden Schritt eine nachrangige oder untergeordnete Rolle (W.J. Owen: Metabolism of herbicides - detoxification as a basis of selectivity, Cambridge University Press (1989)).

Es gelang in Weizen- und Gerstekeimlingen das erste Abbauprodukt des Herbizids Fenoxaprop-ethyl, dünnschichtchromatographisch nachzuweisen. Es handelt sich dabei um das Benzoxazolon-Konjugat mit Glutathion, welches entsteht, wenn eine Glutathion-S-Transferase das Herbizid unter Spaltung der Etherbrücke mit Glutathion konjugiert (siehe Abbildung 1). Als Standard wurde radioaktives Benzoxazolon-Konjugat mit auf die Dünnschichtplatte aufgetragen. Das Abbauprodukt in Weizen und Gerste kochromatographiert mit diesem Standard. Der RF-Wert beträgt 0,64 in Butanol:Eisessig:Wasser = 12:3:5 auf Kieselgelplatten.

Gegenstand der vorliegenden Erfindung ist auch ein Verfahren zur Selektion von Verbindungen mit Safenerpotential.

Im folgenden ist dieses Verfahren am Beispiel des Herbizids Fenoxaprop-ethyl beschrieben. Das Verfahren ist zur Selektion von Safenern für Herbizide geeignet, die durch Konjugation mit Glutathion entgiftet werden. Das Verfahren ist aber auch zur Selektion von Safenern für Herbizide geeignet, die auf anderem Wege abgebaut werden.

### Methode:

### 1. Saatgutbeizung

Je 5 g Getreidesaatgut (Weizen: Schirokko, Gerste: Igri-Wintergerste, Mais: Mutin KWS, Hafer: Flämingsnova, Reis: Bahia Spanien) sowie einige Ungräser (Flughafer, Ackerfuchsschwanz) wurden in jeweils 1 ml Methylenchlorid geschwenkt, in dem 8 mg eines Safeners gelöst war. Nachdem alle Körner benetzt waren, wurde das Lösungsmittel 3 min unter Vakuum abgedampft.

### 2. Keimung

Die derart vorbehandelten Körner wurden in geschlossenen Plastik-Containern auf feuchtem Filterpapier ausgelegt. Die Keimung erfolgte in einem Phytoschrank bei konstant 25°C und einer Licht-Dunkelphase von 16 h zu 8 h.

### 3. Herstellung der Rohextrakte

Jeweils 500 mg Keimlingsmaterial wurden in flüssigem Stickstoff eingefroren und in Gegenwart von 1 ml Extraktionspuffer gemörsert. Der Extraktionspuffer enthielt 0,2 M Tris/HCl, pH 7,8 1 mM EDTA und 7,5 % (w/v] Polyvinylpolypyrolidon (PVPP). Der Extrakt wurde durch 10minütige Zentrifugation bei 10000 rpm und 4°C vom Zelldebris befreit, der resultierende Überstand für die nachfolgenden Messungen benutzt.

### 4. Optische Bestimmung der Glutathion-S-Transferase (GST) Aktivität

Um eine etwaige Erhöhung der GST-Aktivität in den Extrakten nach Behandlung mit einem potentiellen Safenermolekül zu bestimmen, wurde das universelle Substrat 1-Chlor-2,4-Dinitrobenzol (CDNB) als Substrat eingesetzt. Während das Substrat CDNB ein Absorptionsmaximum bei 280 nm aufweist, kann man die Entstehung des Glutathion-Konjugates S-(2,4-Dinitrophenyl)Glutathion bei 340 nm verfolgen.

Der Reaktionsansatz enthielt tvpischerweise 0,1 M Kalium-P-Puffer, pH 6,5, 5 mM Glutathion (red), 1 mM CDNB und 0,1 ml Keimlingsextrakt in einem Endvolumen von 1 ml. Die Entstehung des Konjugates wurde über 10 min bei 340 nm verfolgt. Als Maß für die GST-Aktivität wurden die jeweiligen Delta OD 340 nm/min miteinander verglichen.

### 5. Semiquantitative Bestimmung des Herbizidabbauproduktes

Konjugate des schwer wasserlöslichen Herbizids Fenoxaprop-ethyl mit Glutathion sollten wesentlich hydrophiler sein als das Ausgangsprodukt. Daher wurde das ¹⁴C markierte Herbizid in Gegenwart von Glutathion in den Keimlingsextrakten inkubiert und in einem Zweiphasensystem analysiert.

Der Test enthielt typischerweise 1 mM Fenoxaprop-ethyl (nicht radioaktiv), 1 µCi ¹⁴C-Fenoxaprop-ethyl, 10 mM Glutathion (red), 0,1 M Kalium-P-Puffer, pH 6,5 und 5 µl Extrakt. Das Endvolumen betrug 50 µl und die Inkubation wurde für eine Stunde bei 37°C durchgeführt. Die Reaktion wurde durch Zugabe von 450 µl Wasser gestoppt und die Wasserphase je zweimal mit 500 µl Chloroform und einmal mit 500 µl Ether extrahiert. Die Wasserphase wurde entnommen und die die entstandenen, hydrophilen Konjugate durch Flüssigkeitsszintillationszählung bestimmt.

### 6. Identifizierung und Quantifizierung des Abbauprodukts

Zur Identifizierung des Abbauprodukts des Herbizids in Getreide wurden Aliquots der Inkubationen auf Dünnschichtplatten aufgetrennt und durch Autoradiographie visualisiert.

Der Inkubationsansatz enthielt folgende Komponenten:
0,1 M Tris/HCl, pH 8,5, 1 µCi ¹⁴C-Fenoxaprop-ethyl, 10 mM Glutathion (red) und 10 µl Keimlingsextrakt. Das Endvolumen wurde mit Wasser auf 50 µl eingestellt. Die Inkubation erfolgte für 2 h bei 37°C. Gleiche Volumina verschiedener Extrakte wurden auf Dünnschichtplatten (Cellulose-F oder Kieselgel) aufgetragen und in n-Butanol:Eisessig:Wasser = 12:3:5 entwickelt. Als Marker für die Position des Abbauproduktes wurde reines S-(6-Chlorbenzoxazol-2-yl)Glutathion (Phenyl-U-¹⁴C) aufgetragen. Es handelt sich dabei um das Benzoxazolon-Konjugat des Herbizids, welches aus theoretischen Überlegungen als erstes Abbauprodukt erwartet wurde. Nach Beendigung der Chromatographie wurde die Platte getrocknet und einer Autoradiographie unterworfen.

Als Ergebnis zeigten sich nur zwei radioaktive markierte Banden, von denen eine mit dem unbehandelten radioaktiv markierten und zur Kontrolle aufgetragen Fenoxaprop-ethyl kochromatographiert. Dabei handelt es sich mit großer Wahrscheinlichkeit um den Anteil des Herbizids, der in unserem System nicht in Lösung gegangen ist und somit nicht für die enzymatische Reaktion im wäßrigen Extrakt zur Verfügung stand. Die zweite Bande, mit einem RF-WErt von 0,64, kochromatographierte mit dem Benzoxazolonkonjugat des Herbizids. Weitere Banden wurden nicht beobachtet.

Die zweidimensionale Auftrennung solcher Inkubationen kann in verschiedenen Laufmittelgemischen durchgeführt werden, z.B.: eine Auftrennung mit n-Butanol:Eisessig:Wasser = 12:3:5 in der 1. Dimension und n-Heptan:Eisessigsäureethylester = 1:1 in der 2. Dimension bestätigte den Befund, daß als einziges Abbauprodukt das Glutathion-Konjugat entsteht.

Zur direkten Quantifizierung des Abbauproduktes wurde die entsprechende Bande aus der Dünnschichtplatte ausgeschnitten und die Menge des Abbauproduktes durch Flüssigkeitsszintillationszählung ermittelt.

Im Vergleich der Mengen an Abbauprodukt von nicht behandelten Kontrollen und mit Safener behandeltem Saatgut manifestiert sich die Safenerwirkung durch einen erhöhten Abbau des Herbizids nach Safenerbehandlung. Die Beschleunigung des Abbaus durch bisher bekannte Safener betrug je nach Safenermolekül und Pflanzenart, einen Faktor 2 bis 6 im Vergleich zu den unbehandelten Kontrollen.

### Abbildung 1: Abbauschema des Herbizids Fenoxaprop-ethyl in Getreide

Als erstes nicht mehr herbizid wirksames Produkt entsteht ein Konjugat mit Glutathion, bei gleichzeitiger Spaltung des Moleküls. Die freie Säure wirkt noch herbizid (J. of Agricultural end Food Chemistry 1984, 32, 187/Dissertation: Heinz Sochar: 1986, Metabolismusuntersuchungen von Fenoxaprop-ethyl an empfindlichen und unempfindlichen Pflanzen unter besonderer Berücksichtigung polarer Metabolite).

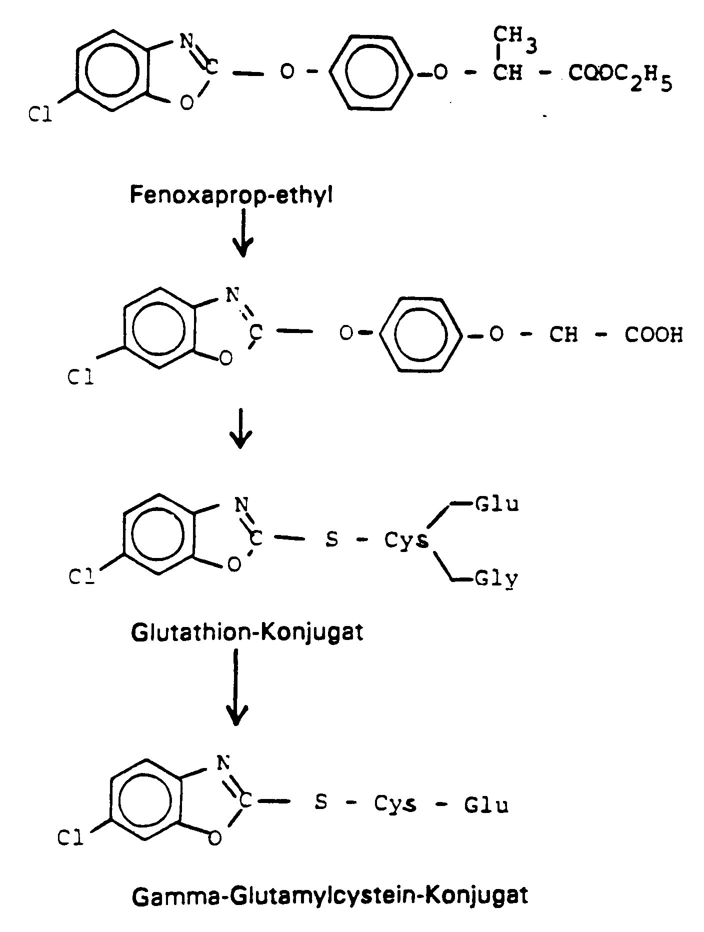

In der folgenden Tabelle III sind die Ergebnisse zusammengefaßt, die für Verbindungen der Formel (I) erhalten wurden. Als Vergleichsverbindung wurde der bekannte Safener 1-(2,4-Dichlorphenyl)-5-trichlormethyl-1,2,4-triazol-3-carbonsäureethylester (Beisp. Nr. 36 -(EP-A-0174562 (US-4,639,266)) untersucht (S). Als Herbizid wurde Fenoxaprop-ethyl eingesetzt.

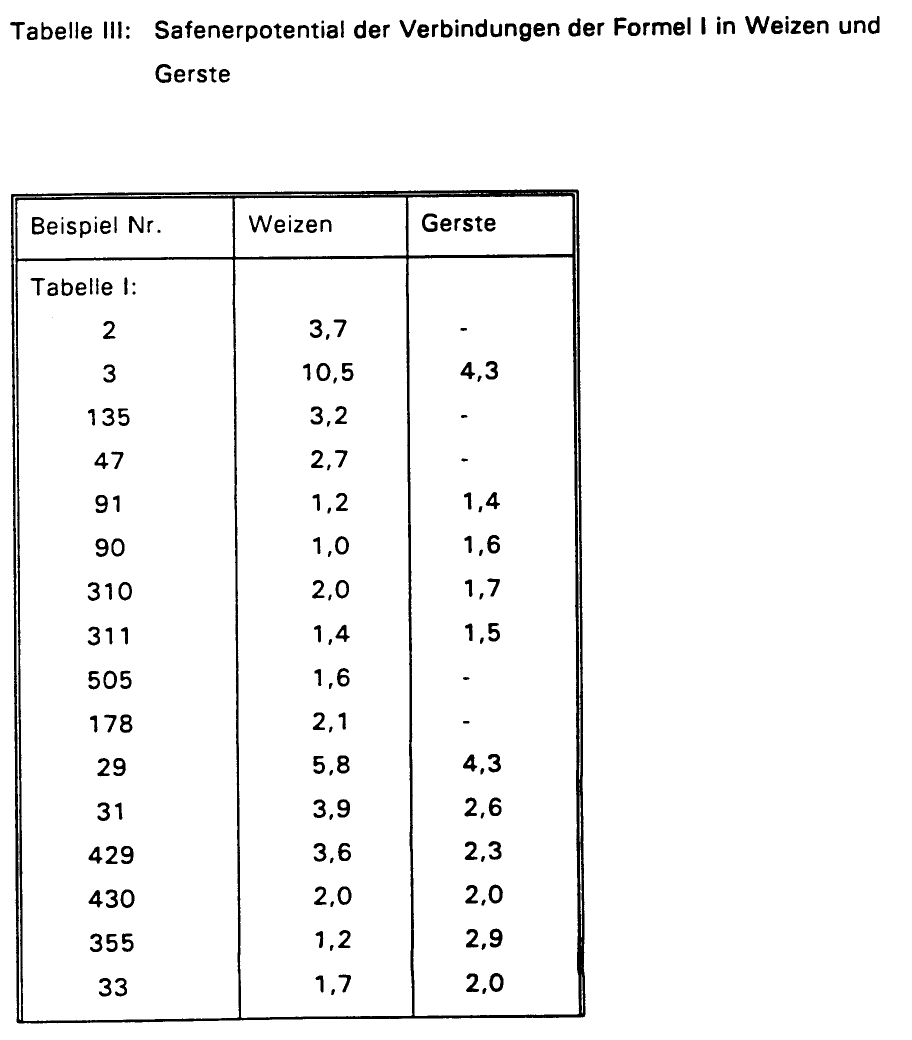

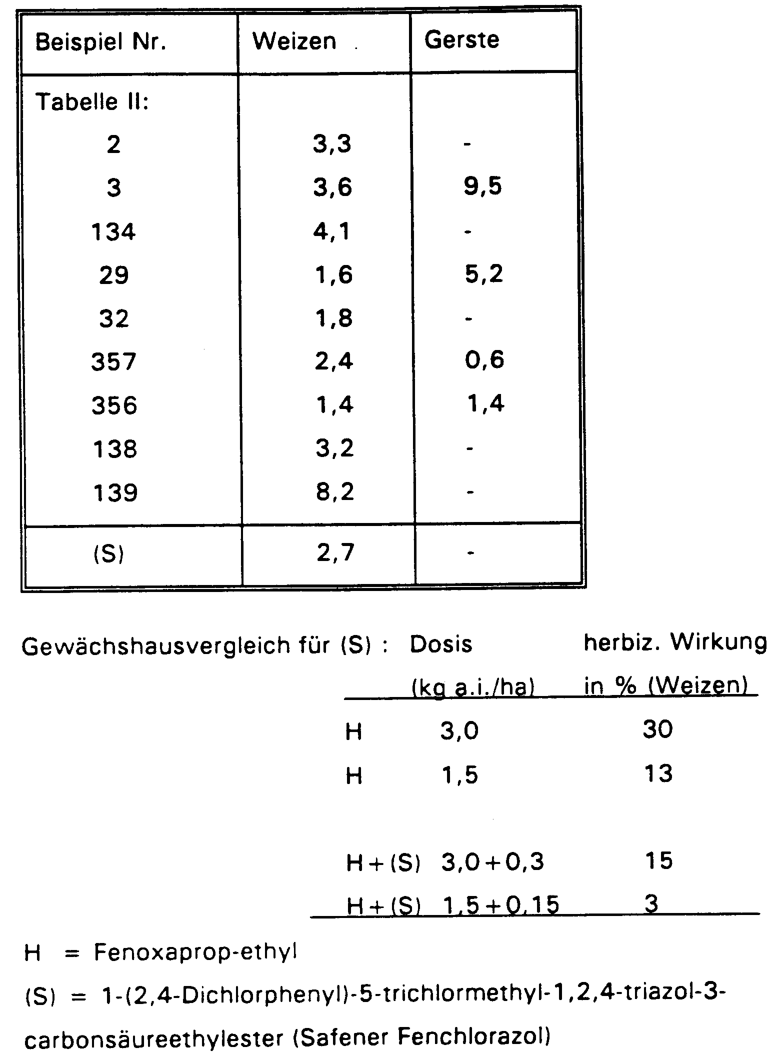

### Zur Erläuterung:

Die unter Weizen und Gerste angegebenen Werte geben den Faktor an, um den sich die Bildung des Abbauproduktes beschleunigt, wenn man potentielle Safenermoleküle im Vergleich zur nicht behandelten Kontrolle auf das jeweilige Saatgut einwirken läßt. Die Menge der Abbauprodukte wurde nach der quantitativen Methode bestimmt.

### Biologische Beispiele

### Beispiel 1

Weizen und Gerste wurden im Gewächshaus in Plastiktöpfen bis zum 3-4 Blattstadium herangezogen und dann nacheinander mit erfindungsgemäßen Verbindungen und Herbiziden im Nachlautverfahren behandelt. Die Herbizide und die Verbindungen der Formel (I) wurden dabei in Form wäßriger Suspensionen bzw. Emulsionen mit einer Wasseraufwandmenge von umgerechnet 600 bis 800 l/ha ausgebracht. 3-4 Wochen nach der Behandlung wurden die Pflanzen visuell auf jede Art von Schädigung durch die ausgebrachten Herbizide bonitiert, wobei insbesondere das Ausmaß der anhaltenden Wachstumshemmung berücksichtigt wurde. Die Bewertung erfolgte in Prozentwerten im Vergleich zu unbehandelten Kontrollen.

Die Ergebnisse aus Tabelle IV veranschaulichen, daß die erfindungsgemäßen Verbindungen starke Herbizidschäden an Kulturpflanzen effektiv reduzieren können.

Selbst bei starken Überdosierungen des Herbizids H werden bei den Kulturpflanzen auftretende schwere Schädigungen deutlich reduziert und geringere Schäden völlig aufgehoben. Mischungen aus Herbiziden und erfindungsgemäßen Verbindungen eignen sich deshalb in ausgezeichneter Weise zur selektiven Unkrautbekämpfung in Getreidekulturen.

**Tabelle IV**

| Safenerwirkung der erfindungsgemäßen Verbindungen im Gewächshaus | | | |
|---|---|---|---|
| Bsp. Nr. | kg a.i./ha | TRAE | HOVU |
| H | 2,0 | 80 | - |
| | 0,2 | - | 85 |
| H+3. Tab. I | 2,0 + 1,25 | 20 | - |
| H+3. Tab. I | 0,2 + 1,25 | - | 30 |
| H+3. Tab. II | 2,0 + 1,25 | 25 | - |
| H+3. Tab. II | 0,2 + 1,25 | - | 35 |
| Abkürzungen: TRAE = Triticum aestivum HOVU = Hordeum vulgare a.i. = Aktivsubstanz H = 2-(4-(6-Chlorbenzoxazol-2-yloxy)phenoxy-propionsäureethylester (Fenoxaprop-ethyl) - = nicht getestet 3. Tab.I = Verbindung Nr.3 aus Tabelle I 3. Tab.II = Verbindung Nr. 3 aus Tabelle II | | | |

## Patentansprüche (Patentansprüche für folgende(n) Vertragsstaat(en): DE, FR, GB, IT)

1. Kulturpflanzenschützendes Mittel, welches mindestens eine Verbindung der Formel (I) oder deren Salz, 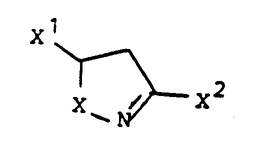 in welcher entweder
X¹ = R¹ und X² = CO-R bedeutet oder
X¹ = CO-R und X² = R¹ bedeutet,
worin
X ein Sauerstoff- oder Schwefelatom, insbesondere ein Sauerstoffatom bedeutet, und
R
a) Hydroxy oder Mercapto,
b) Alkoxy, Alkenyloxy, Alkinyloxy, Alkylthio, Alkenylthio, Alkinylthio, Cycloalkyloxy oder Cycloalkylthio bedeutet, das jeweils unsubstituiert oder ein- oder mehrfach durch Reste aus der Gruppe enthaltend Phenyl, Alkoxy, Alkenyloxy, Alkinyloxy, Benzyloxy, Phenyloxy, Cycloalkyloxy, Alkylthio, Mono- und Dialkylamino, Cyano, Halogen und NO₂ substituiert ist,
c) Benzyloxy, Phenyloxy, Benzylthio oder Phenylthio bedeutet, das jeweils unsubstituiert oder ein- oder mehrfach durch Reste aus der Gruppe enthaltend Alkyl, Alkenyl, Alkinyl, Halogen, Cyano, Nitro, Alkoxy, Alkenyloxy, Alkinyloxy, Alkylthio, Mono- und Dialkylamino, Phenyloxy und Benzyloxy substituiert ist,
d) Trialkylsilylalkoxy, Aryldialkylsilyloxy, Aralkyldialkylsilyloxy, Diarylalkylsilyloxy oder Diaralkylalkylsilyloxy bedeutet,
e) einen Rest der Formel NR'R' bedeutet, worin die Reste R' für gleiche oder verschiedene Reste aus der Gruppe Alkyl, Alkenyl, Alkinyl und Cycloalkyl stehen,
f) Pyridino, Morpholino, Alkylmorpholino, Dialkylmorpholino, Hydrazino, Alkylhydrazino oder Dialkylhydrazino bedeutet,
g) einen Rest der Formel 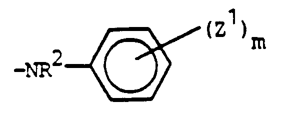 bedeutet, worin R² Wasserstoff, Alkyl, Alkenyl oder Alkinyl und die Reste Z¹ unabhängig voneinander Halogen, Nitro, Alkyl, Alkenyl, Alkoxy oder Phenoxy und m eine ganze Zahl von 0 bis 5 sind,
h) einen Rest der Formel 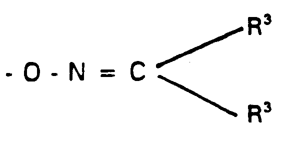 bedeutet, worin die Reste R³ jeweils unabhängig voneinander Alkyl oder, gemeinsam mit dem sie verknüpfenden C-Atom, Cycloalkyl bedeuten.
i) einen Rest der Formel
- O - CR⁴R⁴ - CO - R⁵
bedeutet, worin die R⁴ unabhängig voneinander Wasserstoff, Alkyl, Alkenyl, Alkinyl, Aryl, Benzyl, Alkoxy, Alkenyloxy, Alkinyloxy oder Phenoxy bedeuten und R⁵ Wasserstoff, Alkyl, Alkenyl, Alkinyl, Aryl oder Benzyl bedeutet,
k) einen Rest der Formel 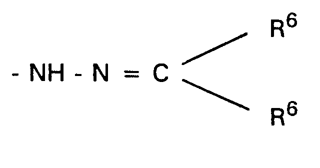 bedeutet, worin die R⁶ unabhängig voneinander Alkyl, Wasserstoff oder Aryl oder, gemeinsam mit dem sie verknüpfenden C-Atom, Cycloalkyl bedeuten, oder
l) einen Rest der Formel
- O - CR⁷R⁷ - CO - R⁸
bedeutet, worin die Reste R⁷ unabhängig voneinander Wasserstoff, Alkyl, Alkenyl, Alkinyl, Aryl, Benzyl, Alkoxy, Alkenyloxy, Alkinyloxy oder Phenoxy bedeuten und R⁸ die vorstehend unter a) bis k) für R angegebene Bedeutung hat, und
R¹ = Wasserstoff, Alkyl, Alkenyl, Alkinyl, Cycloalkyl, Cycloalkenyl, wobei die 4 letztgenannten Gruppen unsubstituiert oder ein- oder mehrfach durch Reste aus der Gruppe enthaltend Phenyl, Hydroxy, Alkoxy, Alkenyloxy, Alkinyloxy, Benzyloxy, Phenyloxy, Cycloalkyloxy, Alkylthio, Mono- und Dialkylamino, Cyano, Halogen und Nitro substitutiert sind, bedeutet,
und übliche Formulierungshilfsmittel enthält.

2. Mittel gemäß Anspruch 1, in welchem in Formel (I)
R
a) Hydroxy oder Mercapto bedeutet,
b) (C₁-C₁₀)-Alkoxy, (C₂-C₄)-Alkenyloxy, (C₂-C₄)-Alkinyloxy, (C₁-C₄)-Alkylthio, (C₂-C₄)-Alkenylthio, (C₂-C₄)-Alkinylthio, (C₃-C₈)-Cycloalkoxy oder (C₃-C₈)-Cycloalkylthio bedeutet, das jeweils unsubstituiert oder ein- oder mehrfach durch Reste aus der Gruppe Phenyl, (C₁-C₄)-Alkoxy, (C₂-C₄)-Alkenyloxy, (C₂-C₄)-Alkinyloxy, Benzyloxy, Phenyloxy, (C₃-C₈)-Cycloalkyloxy, (C₁-C₄)-Alkylthio, Mono- und Di-(C₁-C₄)-Alkylamino, Cyano, Halogen und NO₂ substituiert ist,
c) Phenyloxy, Phenylthio, Benzyloxy oder Benzylthio bedeutet, das jeweils unsubstituiert oder durch 1 bis 3 Reste aus der Gruppe (C₁-C₄)-Alkyl, (C₂-C₄)-Alkenyl, (C₂-C₄)-Alkinyl, Halogen, Cyano, NO₂, (C₁-C₄)-Alkoxy, (C₂-C₄)-Alkenyloxy, (C₂-C₄)-Alkinyloxy, (C₁-C₄)-Alkylthio, Mono- und Di-(C₁-C₄)-Alkylamino, Phenyloxy und Benzyloxy substituiert ist,
d) einen Rest der Formel -NR'R' bedeutet, worin R' (C₁-C₄)-Alkyl bedeutet,
e) Pyridino, Morpholino, Dimethylmorpholino oder Hydrazino bedeutet,
f) einen Rest der Formel 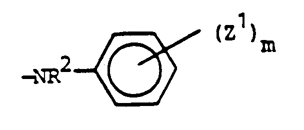 bedeutet, worin R², H oder (C₁-C₄)-Alkyl, die Reste Z¹ unabhängig voneinander Halogen, Nitro, (C₁-C₄)-Alkyl, (C₁-C₄)-Alkoxy oder Phenoxy und m 0 bis 3 bedeuten,
g) einen Rest der Formel -O-N=CR³R³ bedeutet, worin die R³ (C₁-C₄)-Alkyl, insbesondere Methyl, oder gemeinsam mit dem sie verknüpfenden C-Atom Cyclohexyl oder Cyclopentyl bedeuten,
h) einen Rest der Formel -O-CR⁴R⁴-CO-R⁵ bedeutet, worin die R⁴ unabhängig voneinander für H, (C₁-C₄)-Alkyl, (C₂-C₄)-Alkenyl, (C₂-C₄)-Alkinyl, Phenyl, Benzyl oder (C₁-C₄)-Alkoxy stehen und R⁵ für (C₁-C₄)-Alkyl, (C₂-C₄)-Alkenyl, (C₂-C₄)-Alkinyl, Phenyl oder Benzyl steht,
i) einen Rest der Formel -NH-N = CR⁶R⁶ bedeutet, worin die Reste R⁶ unabhängig voneinander H, (C₁-C₄)-Alkyl oder Phenyl oder gemeinsam mit dem sie verknüpfenden C-Atom Cyclohexyl oder Cyclopentyl bedeuten, oder
k) einen Rest der Formel -O-CR⁷R⁷-CO-R⁸ bedeutet, worin die Reste R⁷ unabhängig voneinander für H, (C₁-C₄)-Alkyl, (C₂-C₄)-Alkenyl, (C₂-C₄)-Alkinyl, Phenyl, Benzyl oder (C₁-C₄)-Alkoxy stehen und R⁸ die vorstehend unter a) bis i) für R genannten Bedeutung hat, und
R¹ (C₁-C₁₈)-Alkyl, (C₁-C₁₈)-Alkenyl, (C₁-C₁₈)-Alkinyl, Cycloalkyl oder Cycloalkenyl bedeutet, wobei die 4 letztgenannten Gruppen unsubstituiert oder ein- oder mehrfach durch Reste aus der Gruppe enthaltend Phenyl, (C₁-C₄)-Alkoxy, (C₁-C₄)-Alkenyloxy, (C₁-C₄)-Alkyl, (C₁-C₄)-Alkinyloxy, Benzyloxy, Phenyloxy, Cycloalkyloxy, Alkylthio, Mono- und Dialkylamino, Cyano, Halogen und NO₂ substituiert sind.

3. Mittel gemäß Anspruch 1 oder 2, in welchem in Formel I
R (C₁-C₄)-Alkoxy, Hydrazino, Dimethylamino oder Benzylamino und
R¹ (C₃-C₆)-Alkyl, Cyclohexyl, Halogenocyclohexyl, Cyclooctyl, Ethenyl, Methylhydrazino oder Adamantyl bedeutet.

4. Mittel gemäß einem der Ansprüche 1 bis 3, in welchem in Formel (I) X für ein Sauerstoffatom steht.

5. Mittel gemäß einem der Ansprüche 1 bis 4, dadurch gekennzeichnet, daß es zusätzlich mindestens einen herbiziden Wirkstoff enthält.

6. Mittel gemäß Anspruch 5, dadurch gekennzeichnet, daß die herbiziden Wirkstoffe aus der Klasse Carbamate, Thiocarbamate, Halogenacetanilide, substituierten Phenoxy-, Naphthoxy- und Phenoxy-phenoxycarbonsäurederivate sowie Heteroaryloxy-phenoxyalkancarbonsäurederivate, Cyclohexandionabkömmlinge, Imidazolinone und Sulfonylharnstoffe sind.

7. Verbindungen der Formel (I), wie sie in einem der Ansprüche 1 bis 4 definiert sind, sowie deren Salze, ausgenommen Verbindungen der Formel (I), worin
a) X Sauerstoff, X¹ = R¹, X² = COR und
- R = OCH₃ und R¹ = n-C₆H₁₃, n-C₈H₁₇ oder n-C₁₂H₂₅ oder
- R = OC₂H₅ und
R¹ = H, CH₃, n-C₄H₉, n-C₆H₁₃, CH=CH₂, CH₂CH=CH₂, C(CH₃)=CH₂ oder C≡CH oder
- R = OH und R¹ = H oder CH₃ oder
- R = Phenylamino oder p-Chlor-phenylamino und R¹ = n-C₁₂H₂₅
bedeuten oder worin
b) X Sauerstoff, X¹ = COR, X² = R¹ und
- R = OCH₃ und R¹ = H, CH₃, C₂H₅ oder t-C₄H₉ oder
- R = OC₂H₅ und R¹ = (C₁-C₇)Alkyl
bedeuten.

8. Verfahren zur Herstellung einer Verbindung gemäß Anspruch 7, dadurch gekennzeichnet, daß man a) im Falle X¹ = R¹ und X² = COR eine Verbindung der Formel (II)
H₂C = CH - R¹ (II)
mit einer Verbindung der Formel (III) 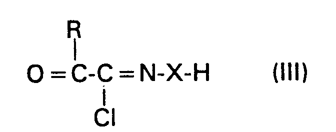 umsetzt, wobei in den Formeln (II) und (III) X, R und R¹ die bei Formel (I) definierte Bedeutung haben, und daß man b) im Falle X¹ = COR und X² = R¹ eine Verbindung der Formel (IV) 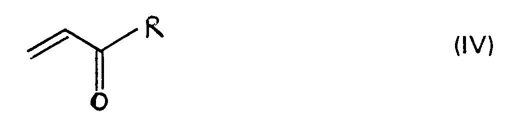 mit einer Verbindung der Formel (V) 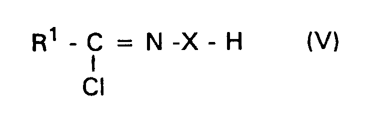 umsetzt, wobei in den Formeln (IV) und (V) X, R und R¹ die bei Formel (I) definierte Bedeutung haben, und die nach a) oder b) erhaltene Verbindung gegebenenfalls in ihr Salz überführt.

9. Verfahren zur Bekämpfung von unerwünschten Pflanzen in Nutzpflanzenkulturen, dadurch gekennzeichnet, daß man mindestens einen herbiziden Wirkstoff in Kombination mit mindestens einer gemäß einem der Ansprüche 1 bis 4 definierten Verbindung der Formel I auf Pflanzen, Pflanzenteile, Pflanzensamen oder die Anbaufläche appliziert.

10. Verwendung von einer gemäß einem der Ansprüche 1 bis 4, definierten Verbindung der Formel (I) zum Schutz von Kulturpflanzen vor phytotoxischen Nebenwirkungen von Herbiziden.

11. Verfahren zur Herstellung eines Mittels gemäß einem der Ansprüche 1 bis 6, dadurch gekennzeichnet, daß man mindestens eine Verbindung der Formel (I), gegebenenfalls zusammen mit mindestens einem herbiziden Wirkstoff, und Formulierungshilfsmittel analog üblichen Methoden in eine für den Pflanzenschutz geeignete Anwendungsform bringt.

## Patentansprüche (Patentansprüche für folgende(n) Vertragsstaat(en): ES)

1. Verwendung einer Verbindung der Formel (I) oder deren Salz, 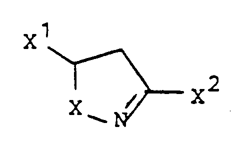 in welcher entweder
X¹ = R¹ und X² = CO-R bedeutet oder
X¹ = CO-R und X² = R¹ bedeutet,
worin
X ein Sauerstoff- oder Schwefelatom, insbesondere ein Sauerstoffatom bedeutet, und
R
a) Hydroxy oder Mercapto,
b) Alkoxy, Alkenyloxy, Alkinyloxy, Alkylthio, Alkenylthio, Alkinylthio Cycloalkyloxy oder Cycloalkylthio bedeutet, das jeweils unsubstituiert oder ein- oder mehrfach durch Reste aus der Gruppe enthaltend Phenyl, Alkoxy, Alkenyloxy, Alkinyloxy, Benzyloxy, Phenyloxy, Cycloalkyloxy, Alkylthio, Mono- und Dialkylamino, Cyano, Halogen und NO₂ substituiert ist,
c) Benzyloxy, Phenyloxy, Benzylthio oder Phenylthio, das jeweils unsubstituiert oder ein- oder mehrfach durch Reste aus der Gruppe enthaltend Alkyl, Alkenyl, Alkinyl, Halogen, Cyano, Nitro, Alkoxy, Alkenyloxy, Alkinyloxy, Alkylthio, Mono- und Dialkylamino, Phenyloxy und Benzyloxy substituiert ist,
d) Trialkylsilylalkoxy, Aryldialkylsilyloxy, Aralkyldialkylsilyloxy, Diarylalkylsilyloxy oder Diaralkylalkylsilyloxy bedeutet,
e) einen Rest der Formel NR'R' bedeutet, worin die Reste R' für gleiche oder verschiedene Reste aus der Gruppe Alkyl, Alkenyl, Alkinyl und Cycloalkyl stehen,
f) Pyridino, Morpholino, Alkylmorpholino, Dialkylmorpholino, Hydrazino, Alkylhydrazino oder Dialkylhydrazino bedeutet,
g) einen Rest der Formel 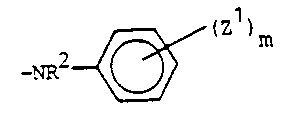 bedeutet, worin R² Wasserstoff, Alkyl, Alkenyl oder Alkinyl und die Reste Z¹ unabhängig voneinander Halogen, Nitro, Alkyl, Alkenyl, Alkoxy oder Phenoxy und m eine ganze Zahl von 0 bis 5 sind,
h) einen Rest der Formel 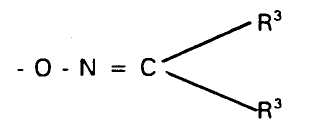 bedeutet, worin die Reste R³ jeweils unabhängig voneinander Alkyl oder, gemeinsam mit dem sie verknüpfenden C-Atom, Cycloalkyl bedeuten.
i) einen Rest der Formel
- O - CR⁴R⁴ - CO - R⁵
bedeutet, worin die R⁴ unabhängig voneinander Wasserstoff, Alkyl, Alkenyl, Alkinyl, Aryl, Benzyl, Alkoxy, Alkenyloxy, Alkinyloxy oder Phenoxy bedeuten und R⁵ Wasserstoff, Alkyl, Alkenyl, Alkinyl, Aryl oder Benzyl bedeutet,
k) einen Rest der Formel 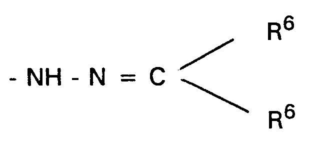 worin die R⁶ unabhängig voneinander Alkyl, Wasserstoff oder Aryl oder, gemeinsam mit dem sie verknüpfenden C-Atom, Cycloalkyl bedeuten, oder
l) einen Rest der Formel
- O - CR⁷R⁷ - CO - R⁸
bedeutet, worin die Reste R⁷ unabhängig voneinander Wasserstoff, Alkyl, Alkenyl, Alkinyl, Aryl, Benzyl, Alkoxy, Alkenyloxy, Alkinyloxy oder Phenoxy bedeuten und R⁸ die vorstehend unter a) bis k) für R angegebene Bedeutung hat, und
R¹ = Wasserstoff, Alkyl, Alkenyl, Alkinyl, Cycloalkyl, Cycloalkenyl, wobei die 4 letztgenannten Gruppen unsubstituiert oder ein- oder mehrfach durch Reste aus der Gruppe enthaltend Phenyl, Hydroxy, Alkoxy, Alkenyloxy, Alkinyloxy, Benzyloxy, Phenyloxy, Cycloalkyloxy, Alkylthio, Mono und Dialkylamino, Cyano, Halogen und Nitro substitutiert sind, zum Schutz von Kulturpflanzen vor phytotoxischen Nebenwirkungen von Herbiziden.

2. Verwendung gemäß Anspruch 1, in welchem in Formel (I)
R
a) Hydroxy oder Mercapto bedeutet,
b) (C₁-C₁₀)-Alkoxy, (C₂-C₄)-Alkenyloxy, (C₂-C₄)-Alkinyloxy, (C₁-C₄)-Alkylthio, (C₂-C₄)-Alkenylthio, (C₂-C₄)-Alkinylthio, (C₃-C₈)-Cycloalkoxy oder (C₃-C₈)-Cycloalkylthio bedeutet, das unsubstituiert oder ein- oder mehrfach durch Reste aus der Gruppe Phenyl, (C₁-C₄)-Alkoxy, (C₂-C₄)-Alkenyloxy, (C₂-C₄)-Alkinyloxy, Benzyloxy, Phenyloxy, (C₃-C₈)-Cycloalkyloxy, (C₁-C₄)-Alkylthio, Mono- und Di-(C₁-C₄)-Alkylamino, Cyano, Halogen und NO₂ substituiert ist,
c) Phenyloxy, Phenylthio, Benzyloxy oder Benzylthio Bedeutet, das jeweils unsubstituiert oder durch 1 bis 3 Reste aus der Gruppe (C₁-C₄)-Alkyl, (C₂-C₄)-Alkenyl, (C₂-C₄)-Alkinyl, Halogen, Cyano, NO₂, (C₁-C₄)-Alkoxy, (C₂-C₄)-Alkenyloxy, (C₂-C₄)-Alkinyloxy, (C₁-C₄)-Alkylthio, Mono- und Di-(C₁-C₄)-Alkylamino, Phenyloxy und Benzyloxy substituiert ist,
d) einen Rest der Formel -NR'R' bedeutet, worin R' (C₁-C₄)-Alkyl bedeutet,
e) Pyridino, Morpholino, Dimethylmorpholino oder Hydrazino bedeutet,
f) einen Rest der Formel 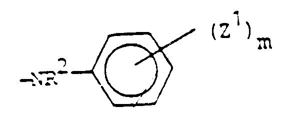 bedeutet, worin R², H oder (C₁-C₄)-Alkyl, die Reste Z¹ unabhängig voneinander Halogen, Nitro, (C₁-C₄)-Alkyl, (C₁-C₄)-Alkoxy oder Phenoxy und m 0 bis 3 bedeuten,
g) einen Rest der Formel -O-N = CR³R³ bedeutet, worin die R³ (C₁-C₄)-Alkyl, insbesondere Methyl, oder gemeinsam mit dem sie verknüpfenden C-Atom Cyclohexyl oder Cyclopentyl bedeuten,
h) einen Rest der Formel -O-CR⁴R⁴-CO-R⁵ bedeutet, worin die R⁴ unabhängig voneinander für H, (C₁-C₄)-Alkyl, (C₂-C₄)-Alkenyl, (C₂-C₄)-Alkinyl, Phenyl, Benzyl oder (C₁-C₄)-Alkoxy stehen und R⁵ für (C₁-C₄)-Alkyl, (C₂-C₄)-Alkenyl, (C₂-C₄)-Alkinyl, Phenyl oder Benzyl steht,
i) einen Rest der Formel -NH-N =CR⁶R⁶ bedeutet, worin die Reste R⁶ unabhängig voneinander H, (C₁-C₄)-Alkyl oder Phenyl oder gemeinsam mit dem sie verknüpfenden C-Atom Cyclohexyl oder Cyclopentyl bedeuten, oder
k) einen Rest der Formel -O-CR⁷R⁷-CO-R⁸ bedeutet, worin die Reste R⁷ unabhängig voneinander für H, (C₁-C₄)-Alkyl, (C₂-C₄)-Alkenyl, (C₂-C₄)-Alkinyl, Phenyl, Benzyl oder (C₁-C₄)-Alkoxy stehen und R⁸ die vorstehend unter a) bis i) für R genannten Bedeutung hat, und
R¹ (C₁-C₁₈)-Alkyl, (C₁-C₁₈)-Alkenyl, (C₁-C₁₈)-Alkinyl, Cycloalkyl oder Cycloalkenyl bedeutet, wobei die 4 letztgenannten Gruppen unsubstituiert oder ein- oder mehrfach durch Reste aus der Gruppe enthaltend Phenyl, (C₁-C₄)-Alkoxy, (C₁-C₄)-Alkenyloxy, (C₁-C₄)-Alkyl, (C₁-C₄)-Alkinyloxy, Benzyloxy, Phenyloxy, Cycloalkyloxy, Alkylthio, Mono- und Dialkylamino, Cyano, Halogen und NO₂ substituiert sind.

3. Verwendung gemäß Anspruch 1 oder 2, in welchem in Formel I
R (C₁-C₄)-Alkoxy, Hydrazino, Dimethylamino oder Benzylamino und
R¹ (C₃-C₆)-Alkyl, Cyclohexyl, Halogenocyclohexyl, Cyclooctyl, Ethenyl, Methylhydrazino oder Adamantyl bedeutet.

4. Verwendung gemäß einem der Ansprüche 1 bis 3, in welchem in Formel (I) X für ein Sauerstoffatom steht.

5. Verwendung gemäß einem der Ansprüche 1 bis 4, dadurch gekennzeichnet, die Verbindung der Formel (I) in einer geeigneten Anwendungsform zusammen mit einem Herbizid und üblichen Formulierungshilfsmitteln eingesetzt wird.

6. Verwendung gemäß Anspruch 5, dadurch gekennzeichnet, daß die herbiziden Wirkstoffe aus der Klasse Carbamate, Thiocarbamate, Halogenacetanilide, substituierten Phenoxy-, Naphthoxy- und Phenoxyphenoxycarbonsäurederivate sowie Heteroaryloxyphenoxyalkancarbonsäure-derivate, Cyclohexandionabkömmlinge, Imidazolinone und Sulfonylharnstoffe sind.

7. Verfahren zur Herstellung von Verbindungen der Formel (I), wie sie in einem der Ansprüche 1 bis 4 definiert sind, sowie deren Salze, ausgenommen Verbindungen der Formel (I), worin
a) X Sauerstoff, X¹ = R¹, X² = COR und
- R = OCH₃ und R¹ = n-C₆H₁₃, n-C₈H₁₇ oder n-C₁₂H₂₅ oder
- R = OC₂H₅ und
R¹ = H, CH₃, n-C₄H₉, n-C₆H₁₃, CH = CH₂, CH₂CH = CH₂, C(CH₃) = CH₂ oder C≡CH oder
R = OH und R¹ = H oder CH₃ oder
- R = Phenylamino oder p-Chlor-phenylamino und R¹ = n-C₁₂H₂₅
bedeuten oder worin
b) X Sauerstoff, X¹ = COR, X² = R¹ und
- R = OCH₃ und R¹ = CH₃, C₂H₅ oder t-C₄H₉ oder
- R = OC₂H₅ und R¹ = (C₁-C₇)Alkyl
bedeuten,
dadurch gekennzeichnet, daß man a) im Falle X¹ = R¹ und X² = COR eine Verbindung der Formel (II)
H₂C = CH - R¹ (II)
mit einer Verbindung der Formel (III) 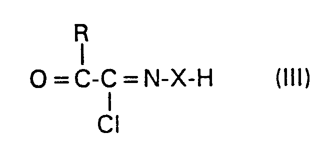 umsetzt, wobei in den Formeln (II) und (III) X, R und R¹ die bei Formel (I) definierte Bedeutung haben,
und daß man b) im Falle X¹ = COR und X² = R¹ eine Verbindung der Formel (IV) 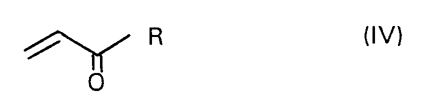 mit einer Verbindung der Formel (V) 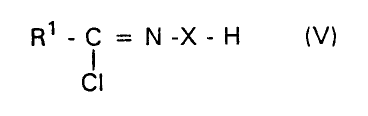 umsetzt, wobei in den Formeln (IV) und (V) X, R und R¹ die bei Formel (I) definierte Bedeutung haben,
und die nach a) oder b) erhaltene Verbindung gegebenenfalls in ihr Salz überführt.

8. Verfahren zur Bekämpfung von unerwünschten Pflanzen in Nutzpflanzenkulturen, dadurch gekennzeichnet, daß man mindestens einen herbiziden Wirkstoff in Kombination mit mindestens einer gemäß einem der Ansprüche 1 bis 4 definierten Verbindung der Formel I auf Pflanzen, Pflanzenteile, Pflanzensamen oder die Anbaufläche appliziert.

9. Verfahren zur Herstellung eines kulturpflanzenschützenden Mittels dadurch gekennzeichnet, daß man mindestens eine Verbindung der Formel (I), wie sie in einem der Ansprüche 1 bis 4 definiert ist, gegebenenfalls zusammen mit mindestens einem herbiziden Wirkstoff, und Formulierungshilfsmittel analog üblichen Methoden in eine für den Pflanzenschutz geeignete Anwendungsform bringt.

## Claims (Claims for the following Contracting State(s): DE, FR, GB, IT)

1. A crop-plant-protecting agent comprising at least one compound of the formula (I) or a salt thereof, 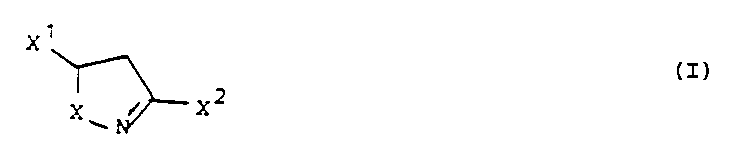 in which either
X¹ is R¹ and X² is CO-R or
X¹ is CO-R and X² is R¹,
where
X is an oxygen or sulfur atom, in particular an oxygen atom, and
R is
a) hydroxyl or mercapto,
b) alkoxy, alkenyloxy, alkynyloxy, alkylthio, alkenylthio, alkynylthio, cycloalkyloxy or cycloalkylthio, each of which is unsubstituted or monosubstituted or polysubstituted by radicals from the group comprising phenyl, alkoxy, alkenyloxy, alkynyloxy, benzyloxy, phenyloxy, cycloalkyloxy, alkylthio, mono- and dialkylamino, cyano, halogen and NO₂,
c) benzyloxy, phenyloxy, benzylthio or phenylthio, each of which is unsubstituted or monosubstituted or polysubstituted by radicals from the group comprising alkyl, alkenyl, alkynyl, halogen, cyano, nitro, alkoxy, alkenyloxy, alkynyloxy, alkylthio, mono- and dialkylamino, phenyloxy and benzyloxy,
d) trialkylsilylalkoxy, aryldialkylsilyloxy, aralkyldialkylsilyloxy, diarylalkylsilyloxy or diaralkylalkylsilyloxy,
e) a radical of the formula NR'R' where the radicals R' are identical or different radicals from the group comprising alkyl, alkenyl, alkynyl and cycloalkyl,
f) pyridino, morpholino, alkylmorpholino, dialkylmorpholino, hydrazino, alkylhydrazino or dialkylhydrazino,
g) a radical of the formula 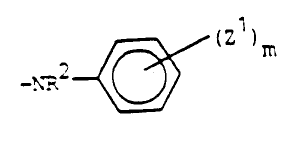 where R² is hydrogen, alkyl, alkenyl or alkynyl and the radicals Z¹ independently of one another are halogen, nitro, alkyl, alkenyl, alkoxy or phenoxy, and m is an integer from 0 to 5,
h) a radical of the formula 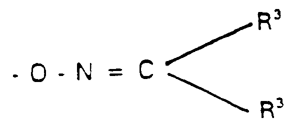 where the radicals R³ in each case independently of one another are alkyl or, together with the carbon atom linking them, are cycloalkyl,
i) a radical of the formula
-O-CR⁴R⁴-CO-R⁵
where the R⁴ radicals independently of one another are hydrogen, alkyl, alkenyl, alkynyl, aryl, benzyl, alkoxy, alkenyloxy, alkynyloxy or phenoxy and R⁵ is hydrogen, alkyl, alkenyl, alkynyl, aryl or benzyl,
k) a radical of the formula 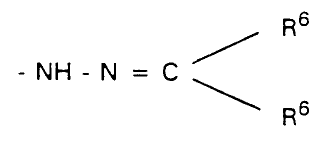 where the R⁶ radicals independently of one another are alkyl, hydrogen or aryl or, together with the carbon atom linking them, are cycloalkyl, or
l) a radical of the formula
-O-CR⁷R⁷-CO-R⁸
where the radicals R⁷ independently of one another are hydrogen, alkyl, alkenyl, alkynyl, aryl, benzyl, alkoxy, alkenyloxy, alkynyloxy or phenoxy and R⁸ has the meaning of R as mentioned above under a) to k), and
R¹ is hydrogen, alkyl, alkenyl, alkynyl, cycloalkyl or cycloalkenyl, where the last 4 groups mentioned are unsubstituted or mono- or polysubstituted by radicals from the group comprising phenyl, hydroxyl, alkoxy, alkenyloxy, alkynyloxy, benzyloxy, phenyloxy, cycloalkyloxy, alkylthio, mono- and dialkylamino, cyano, halogen and nitro,
and contains customary formulation auxiliaries.

2. An agent as claimed in claim 1, in which, in formula (I),
R is
a) hydroxyl or mercapto,
b) (C₁-C₁₀)alkoxy, (C₂-C₄)alkenyloxy, (C₂-C₄)-alkynyloxy, (C₁-C₄)alkylthio, (C₂-C₄)-alkenylthio, (C₂-C₄)alkynylthio, (C₃-C₈)-cycloalkoxy or (C₃-C₈)cycloalkylthio, each of which is unsubstituted or monosubstituted or polysubstituted by radicals from the group comprising phenyl, (C₁-C₄)-alkoxy, (C₂-C₄)alkenyloxy, (C₂-C₄)alkynyloxy, benzyloxy, phenyloxy, (C₃-C₈)cycloalkyloxy, (C₁-C₄)alkylthio, mono- and di(C₁-C₄)alkylamino, cyano, halogen and NO₂,
c) phenyloxy, phenylthio, benzyloxy or benzylthio, each of which is unsubstituted or substituted by 1 to 3 radicals from the group comprising (C₁-C₄)alkyl, (C₂-C₄)-alkenyl, (C₂-C₄)alkynyl, halogen, cyano, NO₂, (C₁-C₄)alkoxy, (C₂-C₄)alkenyloxy, (C₂-C₄)alkynyloxy, (C₁-C₄)alkylthio, mono-and di-(C₁-C₄)alkylamino, phenyloxy and benzyloxy,
d) a radical of the formula -NR'R' where R' is (C₁-C₄)alkyl,
e) pyridino, morpholino, dimethylmorpholino or hydrazino,
f) a radical of the formula 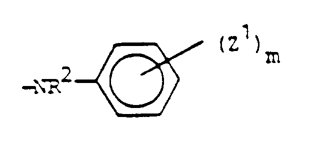 where R² is H or (C₁-C₄)alkyl, the radicals Z¹ independently of one another are halogen, nitro, (C₁-C₄)alkyl, (C₁-C₄)-alkoxy or phenoxy, and m is 0 to 3,
g) a radical of the formula -O-N=CR³R³ where the R³ radicals are (C₁-C₄)alkyl, in particular methyl, or, together with the carbon atom linking them, are cyclohexyl or cyclopentyl,
h) a radical of the formula -O-CR⁴R⁴-CO-R⁵ where the R⁴ radicals independently of one another are H, (C₁-C₄)alkyl, (C₂-C₄)-alkenyl, (C₂-C₄)alkynyl, phenyl, benzyl or (C₁-C₄)alkoxy and R⁵ is (C₁-C₄)alkyl, (C₂-C₄)alkenyl, (C₂-C₄)alkynyl, phenyl or benzyl,
i) a radical of the formula -NH-N=CR⁶R⁶ where the radicals R⁶ independently of one another are H, (C₁-C₄)alkyl or phenyl or, together with the carbon atom linking them, are cyclohexyl or cyclopentyl, or
k) a radical of the formula -O-CR⁷R⁷-CO-R⁸ where the radicals R⁷ independently of one another are H, (C₁-C₄)alkyl, (C₂-C₄)alkenyl, (C₂-C₄)alkynyl, phenyl, benzyl or (C₁-C₄)alkoxy and R⁸ has the meaning of R as mentioned above under a) to i), and
R¹ is (C₁-C₁₈)alkyl, (C₁-C₁₈)alkenyl, (C₁-C₁₈)alkynyl, cycloalkyl or cycloalkenyl, where the last 4 groups mentioned are unsubstituted or mono- or polysubstituted by radicals from the group comprising phenyl, (C₁-C₄)alkoxy, (C₁-C₄)alkenyloxy, (C₁-C₄)alkyl, (C₁-C₄)alkynyloxy, benzyloxy, phenyloxy, cycloalkyloxy, alkylthio, mono- and dialkylamino, cyano, halogen and NO₂.

3. An agent as claimed in claim 1 or 2, in which, in formula I,
R is (C₁-C₄)alkoxy, hydrazino, dimethylamino or benzylamino and
R¹ is (C₃-C₆)alkyl, cyclohexyl, halocyclohexyl, cyclooctyl, ethenyl, methylhydrazino or adamantyl.

4. An agent as claimed in one of claims 1 to 3, in which, in formula (I), X is an oxygen atom.

5. An agent as claimed in one of claims 1 to 4, which comprises additionally at least one herbicidal active substance.

6. An agent as claimed in claim 5, wherein the herbicidal active substances are from the class of the carbamates, thiocarbamates, haloacetanilides, substituted phenoxy-, naphthoxy- and phenoxyphenoxycarboxylic acid derivatives as well as heteroaryloxyphenoxyalkanecarboxylic acid derivatives, cyclohexanedione derivatives, imidazolinones and sulfonylureas.

7. A compound of the formula (I), as defined in one of claims 1 to 4, and salts thereof, with the exception of compounds of the formula (I) where
a) X is oxygen, X¹ is R¹, X² is COR and
- R is OCH₃ and R¹ is n-C₆H₁₃, n-C₈H₁₇ or n-C₁₂H₂₅ or
- R is OC₂H₅ and
R¹ is H, CH₃, n-C₄H₉, n-C₆H₁₃, CH=CH₂, CH₂CH=CH₂, C(CH₃)=CH₂ or C≡CH or
- R is OH and R¹ is H or CH₃ or
- R is phenylamino or p-chlorophenylamino and R¹ is n-C₁₂H₂₅
or where
b) X is oxygen, X¹ is COR, X² is R¹ and
- R is OCH₃ and R¹ is H, CH₃, C₂H₅ or t-C₄H₉ or
- R is OC₂H₅ and R¹ is (C₁-C₇)alkyl.

8. A process for the preparation of a compound as claimed in claim 7, which comprises a), if X¹ is R¹ and X² is COR, reacting a compound of the formula (II)
H₂C=CH-R¹ (II)
with a compound of the formula (III) 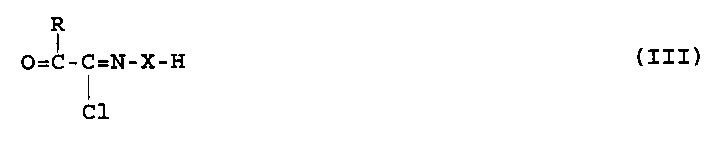 where, in formulae (II) and (III), X, R and R¹ are as defined in formula (I), and b), if X¹ is COR and X² is R¹, reacting a compound of the formula (IV) 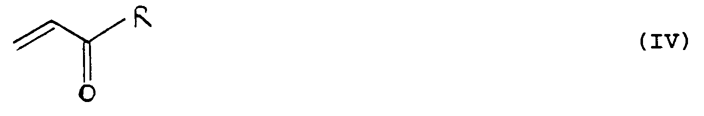 with a compound of the formula (V) 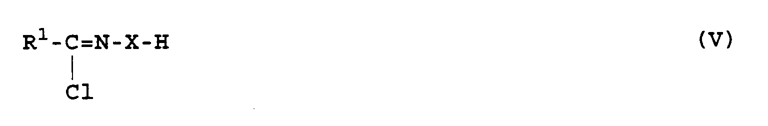 where, in formulae (IV) and (V), X, R and R¹ are as defined in formula (I), and optionally converting the compound obtained according to a) or b) into its salt.

9. A method for controlling undesired plants in crops of useful plants, which comprises applying at least one herbicidal active substance in combination with at least one compound of the formula I, as defined in one of claims 1 to 4, to plants, parts of plants, seeds of plants or to the area under cultivation.

10. The use of a compound of the formula (I), as defined in one of claims 1 to 4, for protecting crop plants against phytotoxic secondary effects of herbicides.

11. A process for the preparation of an agent as claimed in one of claims 1 to 6, which comprises bringing at least one compound of the formula (I), if appropriate together with at least one herbicidal active substance, and formulation auxiliaries analogously to customary methods into a use form suitable for crop protection.

## Claims (Claims for the following Contracting State(s): ES)

1. The use of a compound of the formula (I) or a salt thereof, 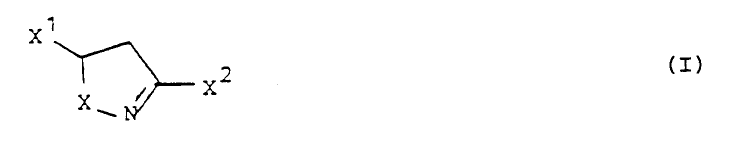 in which either
X¹ is R¹ and X² is CO-R or
X¹ is CO-R and X² is R¹,
where
X is an oxygen or sulfur atom, in particular an oxygen atom, and
R is
a) hydroxyl or mercapto,
b) alkoxy, alkenyloxy, alkynyloxy, alkylthio, alkenylthio, alkynylthio, cycloalkyloxy or cycloalkylthio, each of which is unsubstituted or monosubstituted or polysubstituted by radicals from the group comprising phenyl, alkoxy, alkenyloxy, alkynyloxy, benzyloxy, phenyloxy, cycloalkyloxy, alkylthio, mono- and dialkylamino, cyano, halogen and NO₂,
c) benzyloxy, phenyloxy, benzylthio or phenylthio, each of which is unsubstituted or monosubstituted or polysubstituted by radicals from the group comprising alkyl, alkenyl, alkynyl, halogen, cyano, nitro, alkoxy, alkenyloxy, alkynyloxy, alkylthio, mono- and dialkylamino, phenyloxy and benzyloxy,
d) trialkylsilylalkoxy, aryldialkylsilyloxy, aralkyldialkylsilyloxy, diarylalkylsilyloxy or diaralkylalkylsilyloxy,
e) a radical of the formula NR'R' where the radicals R' are identical or different radicals from the group comprising alkyl, alkenyl, alkynyl and cycloalkyl,
f) pyridino, morpholino, alkylmorpholino, dialkylmorpholino, hydrazino, alkylhydrazino or dialkylhydrazino,
g) a radical of the formula 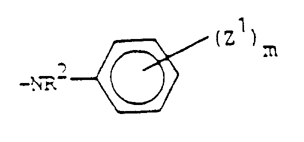 where R² is hydrogen, alkyl, alkenyl or alkynyl and the radicals Z¹ independently of one another are halogen, nitro, alkyl, alkenyl, alkoxy or phenoxy, and m is an integer from 0 to 5,
h) a radical of the formula 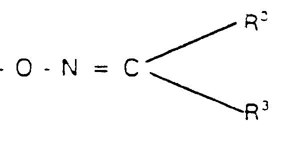 where the radicals R³ in each case independently of one another are alkyl or, together with the carbon atom linking them, are cycloalkyl,
i) a radical of the formula
-O-CR⁴R⁴-CO-R⁵
where the R⁴ radicals independently of one another are hydrogen, alkyl, alkenyl, alkynyl, aryl, benzyl, alkoxy, alkenyloxy, alkynyloxy or phenoxy and R⁵ is hydrogen, alkyl, alkenyl, alkynyl, aryl or benzyl,
k) a radical of the formula 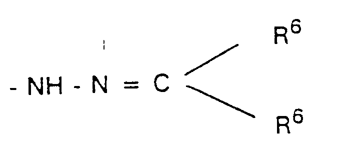 where the R⁶ radicals independently of one another are alkyl, hydrogen or aryl or, together with the carbon atom linking them, are cycloalkyl, or
l) a radical of the formula
-O-CR⁷R⁷-CO-R⁸
where the radicals R⁷ independently of one another are hydrogen, alkyl, alkenyl, alkynyl, aryl, benzyl, alkoxy, alkenyloxy, alkynyloxy or phenoxy and R⁸ has the meaning of R as mentioned above under a) to k), and
R¹ is hydrogen, alkyl, alkenyl, alkynyl, cycloalkyl or cycloalkenyl, where the last 4 groups mentioned are unsubstituted or mono- or polysubstituted by radicals from the group comprising phenyl, hydroxyl, alkoxy, alkenyloxy, alkynyloxy, benzyloxy, phenyloxy, cycloalkyloxy, alkylthio, mono- and dialkylamino, cyano, halogen and nitro, for protecting crop plants from phytotoxic side effects of herbicides.

2. The use as claimed in claim 1, in which, in formula (I),
R is
a) hydroxyl or mercapto,
b) (C₁-C₁₀)alkoxy, (C₂-C₄)alkenyloxy, (C₂-C₄)-alkynyloxy, (C₁-C₄)alkylthio, (C₂-C₄)-alkenylthio, (C₂-C₄)alkynylthio, (C₃-C₈)-cycloalkoxy or (C₃-C₈)cycloalkylthio, each of which is unsubstituted or mono-substituted or polysubstituted by radicals from the group comprising phenyl, (C₁-C₄)alkoxy, (C₂-C₄)alkenyloxy, (C₂-C₄)-alkynyloxy, benzyloxy, phenyloxy, (C₃-C₈)-cycloalkyloxy, (C₁-C₄)alkylthio, mono- and di(C₁-C₄)alkylamino, cyano, halogen and NO₂,
c) phenyloxy, phenylthio, benzyloxy or benzylthio, each of which is unsubstituted or substituted by 1 to 3 radicals from the group comprising (C₁-C₄)alkyl, (C₂-C₄)alkenyl, (C₂-C₄)alkynyl, halogen, cyano, NO₂, (C₁-C₄)alkoxy, (C₂-C₄)-alkenyloxy, (C₂-C₄)alkynyloxy, (C₁-C₄)-alkylthio, mono- and di-(C₁-C₄)alkylamino, phenyloxy and benzyloxy,
d) a radical of the formula -NR'R' where R' is (C₁-C₄)alkyl,
e) pyridino, morpholino, dimethylmorpholino or hydrazino,
f) a radical of the formula 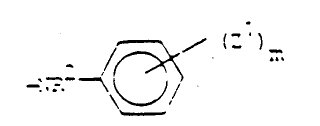 where R² is H or (C₁-C₄)alkyl, the radicals Z¹ independently of one another are halogen, nitro, (C₁-C₄)alkyl, (C₁-C₄)-alkoxy or phenoxy, and m is 0 to 3,
g) a radical of the formula -O-N=CR³R³ where the R³ radicals are (C₁-C₄)alkyl, in particular methyl, or, together with the carbon atom linking them, are cyclohexyl or cyclopentyl,
h) a radical of the formula -O-CR⁴R⁴-CO-R⁵ where the R⁴ radicals independently of one another are H, (C₁-C₄)alkyl, (C₂-C₄)-alkenyl, (C₂-C₄)alkynyl, phenyl, benzyl or (C₁-C₄)alkoxy and R⁵ is (C₁-C₄)alkyl, (C₂-C₄)alkenyl, (C₂-C₄)alkynyl, phenyl or benzyl,
i) a radical of the formula -NH-N=CR⁶R⁶ where the radicals R⁶ independently of one another are H, (C₁-C₄)alkyl or phenyl or, together with the carbon atom linking them, are cyclohexyl or cyclopentyl, or
k) a radical of the formula -O-CR⁷R⁷-CO-R⁸ where the radicals R⁷ independently of one another are H, (C₁-C₄)alkyl, (C₂-C₄)-alkenyl, (C₂-C₄)alkynyl, phenyl, benzyl or (C₁-C₄)alkoxy and R⁸ has the meaning of R as mentioned above under a) to i), and
R¹ is (C₁-C₁₈)alkyl, (C₁-C₁₈)alkenyl, (C₁-C₁₈)-alkynyl, cycloalkyl or cycloalkenyl, where the last 4 groups mentioned are unsubstituted or mono- or polysubstituted by radicals from the group comprising phenyl, (C₁-C₄)alkoxy, (C₁-C₄)alkenyloxy, (C₁-C₄)alkyl, (C₁-C₄)alkynyloxy, benzyloxy, phenyloxy, cycloalkyloxy, alkylthio, mono- and dialkylamino, cyano, halogen and NO₂.

3. The use as claimed in claim 1 or 2, in which, in formula I,
R is (C₁-C₄)alkoxy, hydrazino, dimethylamino or benzylamino and
R¹ is (C₃-C₆)alkyl, cyclohexyl, halocyclohexyl, cyclooctyl, ethenyl, methylhydrazino or adamantyl.

4. The use as claimed in one of claims 1 to 3, in which, in formula (I), X is an oxygen atom.

5. The use as claimed in one of claims 1 to 4, wherein the compound of the formula (I) is employed in a suitable use form together with a herbicide and customary formulation auxiliaries.

6. The use as claimed in claim 5, wherein the herbicidal active substances are from the class of the carbamates, thiocarbamates, haloacetanilides, substituted phenoxy-, naphthoxy- and phenoxyphenoxycarboxylic acid derivatives as well as heteroaryloxyphenoxyalkanecarboxylic acid derivatives, cyclohexanedione derivatives, imidazolinones and sulfonylureas.

7. A process for the preparation of a compound of the formula (I), as defined in one of claims 1 to 4, and salts thereof, with the exception of compounds of the formula (I) where
a) X is oxygen, X¹ is R¹, X² is COR and
- R is OCH₃ and R¹ is n-C₆H₁₃, n-C₈H₁₇ or n-C₁₂H₂₅ or
- R is OC₂H₅ and
R¹ is H, CH₃, n-C₄H₉, n-C₆H₁₃, CH=CH₂, CH₂CH=CH₂, C(CH₃)=CH₂ or C≡CH or
- R is OH and R¹ is H or CH₃ or
- R is phenylamino or p-chlorophenylamino and R¹ is n-C₁₂H₂₅
or where
b) X is oxygen, X¹ is COR, X² is R¹ and
- R is OCH₃ and R¹ is CH₃, C₂H₅ or t-C₄H₉ or
- R is OC₂H₅ and R¹ is (C₁-C₇)alkyl,
which comprises a), if X¹ is R¹ and X² is COR, reacting a compound of the formula (II)
H₂C=CH-R¹ (II)
with a compound of the formula (III) 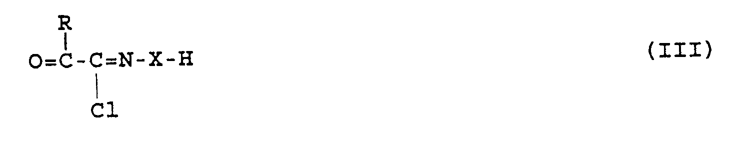 where, in formulae (II) and (III), X, R and R¹ are as defined in formula (I),
and b), if X¹ is COR and X² is R¹, reacting a compound of the formula (IV) 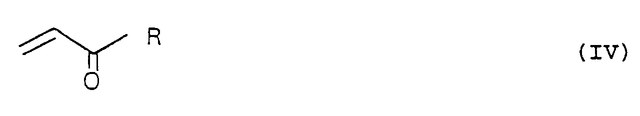 with a compound of the formula (V) 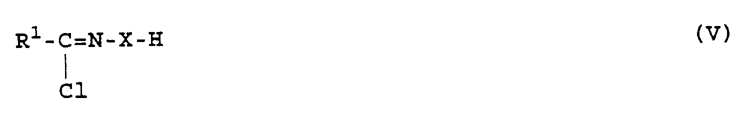 where, in formulae (IV) and (V), X, R and R¹ are as defined in formula (I),
and optionally converting the compound obtained according to a) or b) into its salt.

8. A method for controlling undesired plants in crops of useful plants, which comprises applying at least one herbicidal active substance in combination with at least one compound of the formula I, as defined in one of claims 1 to 4, to plants, parts of plants, seeds of plants or to the area under cultivation.

9. A process for the preparation of a crop-plant-protecting agent, which comprises bringing at least one compound of the formula (I), as defined in one of claims 1 to 4, if appropriate together with at least one herbicidal active substance, and formulation auxiliaries analogously to customary methods into a use form suitable for crop protection.

## Revendications (Revendications pour l'(les) Etat(s) contractant(s) suivant(s): DE, FR, GB, IT)

1. Agent protecteur des plantes de culture, qui contient au moins un composé de formule (I), ou l'un de ses sels 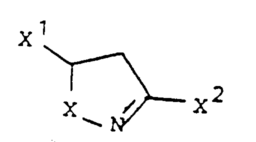 dans laquelle, ou bien
X¹ = R¹ et X² = CO-R, ou bien
X¹ = CO-R et X² = R¹,
où
X est un atome d'oxygène ou de soufre, en particulier un atome d'oxygène, et
R est :
a) un groupe hydroxy ou mercapto,
b) un groupe alcoxy, alcényloxy, alcynyloxy, alkylthio, alcénylthio, alcynylthio, cycloalkyloxy ou cycloalkylthio dont chacun est non substitué, ou est substitué une ou plusieurs fois par des substituants choisis dans le groupe comprenant les radicaux phényle, alcoxy, alcényloxy, alcynyloxy, benzyloxy, phényloxy, cycloalkyloxy, alkylthio, mono- et dialkylamino, cyano, halogéno et NO₂,
c) benzyloxy, phényloxy, benzylthio ou phénylthio, dont chacun est non substitué ou est une ou plusieurs fois substitué par des substituants choisis dans le groupe comprenant les radicaux alkyle, alcényle, alcynyle, halogéno, cyano, nitro, alcoxy, alcényloxy, alcynyloxy, alkylthio, mono- et dialkylamino, phényloxy et benzyloxy,
d) un radical trialkylsilylalcoxy, aryldialkylsilyloxy, aralkyldialkylsilyloxy, diarylalkylsilyloxy ou diaralkylalkylsilyloxy,
e) un radical de formule NR'R', où les radicaux R' représentent des radicaux identiques ou différents choisis parmi les groupes alkyle, alcényle, alcynyle et cycloalkyle,
f) un radical pyridino, morpholino, alkylmorpholino, dialkylmorpholino, hydrazino, alkylhydrazino ou dialkylhydrazino,
g) un radical de formule 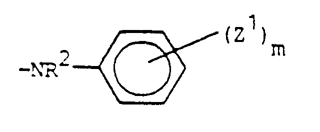 dans laquelle R² est un hydrogène ou un groupe alkyle, alcényle ou alcynyle, et les radicaux Z¹ sont, indépendamment les uns des autres, des groupes halogéno, nitro, alkyle, alcényle, alcoxy ou phénoxy et m est un nombre entier de 0 à 5,
h) un radical de formule 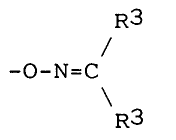 dans laquelle les radicaux R³ sont chacun indépendamment de l'autre des groupes alkyle, ou forment avec l'atome de carbone qui les relie un groupe cycloalkyle,
i) un radical de formule
-O-CR⁴R⁴-CO-R⁵
dans laquelle les radicaux R⁴, indépendamment l'un de l'autre, sont chacun des hydrogènes ou des groupes alkyle, alcényle, alcynyle, aryle, benzyle, alcoxy, alcényloxy, alcynyloxy ou phénoxy, et R⁵ est un hydrogène ou un groupe alkyle, alcényle, alcynyle, aryle ou benzyle,
k) un radical de formule 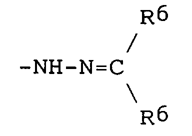 dans laquelle les radicaux R⁶, indépendamment l'un de l'autre, sont des groupes alkyle ou des hydrogènes, ou encore aryle, ou forment avec l'atome de carbone qui les relie un groupe cycloalkyle, ou bien
l) un radical de formule
-O-CR⁷R⁷-CO-R⁸
dans laquelle les radicaux R⁷, indépendamment l'un de l'autre, sont des hydrogènes ou des groupes alkyle, alcényle, alcynyle, aryle, benzyle, alcoxy, alcényloxy, alcynyloxy ou phénoxy, et R⁸ a les significations données ci-dessus, en a) à k), pour R, et
R¹ est un hydrogène ou un radical alkyle, alcényle, alcynyle, cycloalkyle, cycloalcényle, où ces 4 derniers groupes sont non substitués ou sont une ou plusieurs fois substitués par des substituants choisis dans le groupe comprenant les radicaux phényle, hydroxy, alcoxy, alcényloxy, alcynyloxy, benzyloxy, phényloxy, cycloalkyloxy, alkylthio, mono- et dialkylamino, cyano, halogéno et nitro,
et les auxiliaires usuels de formulation.

2. Agent selon la revendication 1, dans lequel, dans la formule (I)
R est
a) un groupe hydroxy ou mercapto,
b) un groupe alcoxy en C₁-C₁₀, alcényloxy en C₂-C₄, alcynyloxy en C₂-C₄, alkylthio en C₁-C₄, alcénylthio en C₂-C₄, alcynylthio en C₂-C₄, cycloalcoxy en C₃-C₈ ou cycloalkylthio en C₃-C₈, dont chacun est non substitué ou est une ou plusieurs fois substitué par des substituants du groupe comprenant les radicaux phényle, alcoxy en C₁-C₄, alcényloxy en C₂-C₄, alcynyloxy en C₂-C₄, benzyloxy, phényloxy, cycloalkyloxy en C₃-C₈, alkylthio en C₁-C₄, mono- et di-(alkyle en C₁-C₄)amino, cyano, halogéno et NO₂,
c) un groupe phényloxy, phénylthio, benzyloxy ou benzylthio, dont chacun est non substitué ou est substitué par 1 à 3 substituants choisis dans le groupe comprenant les radicaux alkyle en C₁-C₄, alcényle en C₂-C₄, alcynyle en C₂-C₄, halogéno, cyano, NO₂, alcoxy en C₁-C₄, alcényloxy en C₂-C₄, alcynyloxy en C₂-C₄, alkylthio en C₁-C₄, mono- et di-(alkyle en C₁-C₄)amino, phényloxy et benzyloxy,
d) un radical de formule -NR'R', où R' est un groupe alkyle en C₁-C₄,
e) un groupe pyridino, morpholino, diméthylmorpholino ou hydrazino,
f) un radical de formule 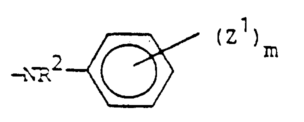 dans laquelle R² est H ou un groupe alkyle en C₁-C₄, les radicaux Z¹, indépendamment les uns des autres, sont des groupes halogéno, nitro, alkyle en C₁-C₄, alcoxy en C₁-C₄ ou phénoxy, et m vaut de 0 à 3,
g) un radical de formule -O-N=CR³R³, où les radicaux R³ sont des groupes alkyle en C₁-C₄, en particulier le groupe méthyle, ou encore, avec l'atome de carbone qui les relie, représentent le groupe cyclohexyle ou cyclopentyle,
h) un radical de formule -O-CR⁴R⁴-CO-R⁵, dans laquelle les radicaux R⁴, indépendamment l'un de l'autre, sont chacun H, ou des groupes alkyle en C₁-C₄, alcényle en C₂-C₄, alcynyle en C₂-C₄, phényle, benzyle ou alcoxy en C₁-C₄, et R⁵ est un groupe alkyle en C₁-C₄, alcényle en C₂-C₄, alcynyle en C₂-C₄, phényle ou benzyle,
i) un radical de formule -NH-N=CR⁶R⁶, dans laquelle les radicaux R⁶, indépendamment l'un de l'autre, sont chacun H, ou des radicaux alkyle en C₁-C₄ ou phényle, ou encore, avec l'atome de carbone qui les relie, représentent un groupe cyclohexyle ou cyclopentyle, ou bien
k) un radical de formule -O-CR⁷R⁷-CO-R⁸, dans laquelle les radicaux R⁷, indépendamment l'un de l'autre, sont H ou des groupes alkyle en C₁-C₄, alcényle en C₂-C₄, alcynyle en C₂-C₄, phényle, benzyle ou alcoxy en C₁-C₄, et R⁸ a les significations données ci-dessus en a) à i) pour R, et
R¹ est un groupe alkyle en C₁-C₁₈, alcényle en C₁-C₁₈, alcynyle en C₁-C₁₈, cycloalkyle ou cycloalcényle, où ces 4 derniers groupes sont non substitués ou sont une ou plusieurs fois substitués par des substituants choisis dans le groupe comprenant les radicaux phényle, alcoxy en C₁-C₄, alcényloxy en C₁-C₄, alkyle en C₁-C₄, alcynyloxy en C₁-C₄, benzyloxy, phényloxy, cycloalkyloxy, alkylthio, mono- et dialkylamino, cyano, halogéno et NO².

3. Agent selon la revendication 1 ou 2, dans lequel, dans la formule (I),
R est un radical alcoxy en C₁-C₄, hydrazino, diméthylamino ou benzylamino, et
R¹ est un radical alkyle en C₃-C₆, cyclohexyle, halogénocyclohexyle, cyclooctyle, éthényle, méthylhydrazino ou adamantyle.

4. Agent selon l'une des revendications 1 à 3, dans lequel dans la formule (I), X est un atome d'oxygène.

5. Agent selon l'une des revendications 1 à 4, caractérisé en ce qu'il contient en outre au moins une matière active herbicide.

6. Agent selon la revendication 5, caractérisé en ce que les matières actives herbicides appartiennent à la classe des carbamates, des thiocarbamates, des halogénacétanilides, des dérivés substitués des acides phénoxy-, naphtoxy- et phénoxy-phénoxycarboxyliques, ainsi que des dérivés des acides hétéroaryloxy-phénoxyalcane-carboxyliques, des dérivés de la cyclohexanedione, des imidazolinones et des sulfonylurées.

7. Composés de formule (I) tels que définis dans l'une des revendications 1 à 4, ainsi que leurs sels, à l'exception des composés de formule (I) dans laquelle :
a) X est un oxygène, X¹ = R¹, X² = COR et
R = OCH₃ et R¹ = n-C₆H₁₃, n-C₈H₁₇ ou n-C₁₂H₂₅ ou
R = OC₂H₅ et R¹ = H, CH₃, n-C₄H₉, n-C₆H₁₃, CH=CH₂, CH₂CH=CH₂, C(CH₃)=CH₂ ou C≡CH ou
R = OH et R¹ = H ou CH₃ ou
R = phénylamino ou p-chloro-phénylamino et R¹ = n-C₁₂H₂₅, ou encore dans laquelle
b) X est un oxygène, X¹ = COR, X² = R¹ et
R = OCH₃ et R¹ = H, CH₃, C₂H₅ ou t-C₄H₉ ou
R = OC₂H₅ et R¹ = alkyle en C₁-C₇.

8. Procédé de préparation d'un composé selon la revendication 7, caractérisé en ce que a) dans le cas dans lequel X¹ = R¹ et X² = COR, on fait réagir un composé de formule (II)
H₂C=CH-R¹ (II)
avec un composé de formule (III) 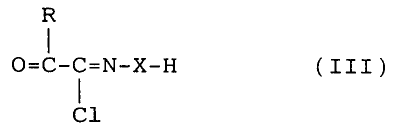 où, dans les formules (II) et (III), X , R et R¹ ont les significations données pour la formule (I), et en ce que b) dans le cas dans lequel X¹ = COR et X² = R¹, on fait réagir un composé de formule (IV) 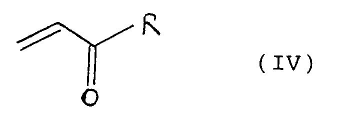 avec un composé de formule (V) 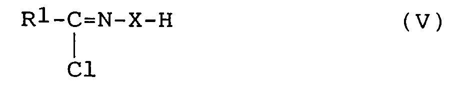 où dans les formules (IV) et (V), X, R, R¹ ont les significations données dans pour la formule (I), le composé obtenu en a) ou b) étant éventuellement converti en son sel.

9. Procédé pour maîtriser des végétaux indésirables dans des cultures de plantes utiles, caractérisé en ce qu'on applique sur les végétaux, les parties de végétaux, les semences ou la surface de culture, au moins une matière active herbicide en combinaison avec au moins un composé de formule (I) défini dans l'une des revendications 1 à 4.

10. Utilisation d'un composé de formule (I), défini selon l'une des revendications 1 à 4, pour protéger les plantes de culture contre les effets secondaires phytotoxiques des herbicides.

11. Procédé de préparation d'un agent selon l'une des revendications 1 à 6, caractérisé en ce qu'on convertit en une forme d'application convenant à la protection des végétaux un composé de formule (I), éventuellement en même temps qu'au moins une matière active herbicide, et des adjuvants de formulation, d'une manière analogue à des procédés usuels.

## Revendications (Revendications pour l'(les) Etat(s) contractant(s) suivant(s): ES)

1. Utilisation d'un composé de formule (I), ou d'un de ses sels 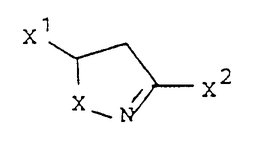 dans laquelle, ou bien
X¹ = R¹ et X² = CO-R, ou bien
X¹ = CO-R et X² = R¹,
où
X est un atome d'oxygène ou de soufre, en particulier un atome d'oxygène, et
R est :
a) un groupe hydroxy ou mercapto,
b) un groupe alcoxy, alcényloxy, alcynyloxy, alkylthio, alcénylthio, alcynylthio, cycloalkyloxy ou cycloalkylthio dont chacun est non substitué, ou est substitué une ou plusieurs fois par des substituants choisis dans le groupe comprenant les radicaux phényle, alcoxy, alcényloxy, alcynyloxy, benzyloxy, phényloxy, cycloalkyloxy, alkylthio, mono- et dialkylamino, cyano, halogéno et NO₂,
c) benzyloxy, phényloxy, benzylthio ou phénylthio, dont chacun est non substitué ou est une ou plusieurs fois substitué par des substituants choisis dans le groupe comprenant les radicaux alkyle, alcényle, alcynyle, halogéno, cyano, nitro, alcoxy, alcényloxy, alcynyloxy, alkylthio, mono- et dialkylamino, phényloxy et benzyloxy,
d) un radical trialkylsilylalcoxy, aryldialkylsilyloxy, aralkyldialkylsilyloxy, diarylalkylsilyloxy ou diaralkylalkylsilyloxy,
e) un radical de formule NR'R', où les radicaux R' représentent des radicaux identiques ou différents choisis parmi les groupes alkyle, alcényle, alcynyle et cycloalkyle,
f) un radical pyridino, morpholino, alkylmorpholino, dialkylmorpholino, hydrazino, alkylhydrazino ou dialkylhydrazino,
g) un radical de formule 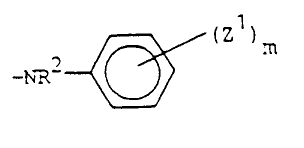 dans laquelle R² est un hydrogène ou un groupe alkyle, alcényle ou alcynyle, et les radicaux Z¹ sont, indépendamment les uns des autres, des groupes halogéno, nitro, alkyle, alcényle, alcoxy ou phénoxy et m est un nombre entier de 0 à 5,
h) un radical de formule 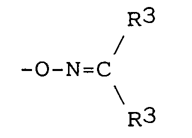 dans laquelle les radicaux R³ sont chacun indépendamment de l'autre des groupes alkyle, ou forment avec l'atome de carbone qui les relie un groupe cycloalkyle,
i) un radical de formule
-O-CR⁴R⁴-CO-R⁵
dans laquelle les radicaux R⁴, indépendamment l'un de l'autre, sont chacun des hydrogènes ou des groupes alkyle, alcényle, alcynyle, aryle, benzyle, alcoxy, alcényloxy, alcynyloxy ou phénoxy, et R⁵ est un hydrogène ou un groupe alkyle, alcényle, alcynyle, aryle ou benzyle,
k) un radical de formule 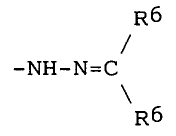 dans laquelle les radicaux R⁶, indépendamment l'un de l'autre, sont des groupes alkyle ou des hydrogènes, ou encore aryle, ou forment avec l'atome de carbone qui les relie un groupe cycloalkyle, ou bien
l) un radical de formule
-O-CR⁷R⁷-CO-R⁸
dans laquelle les radicaux R⁷, indépendamment l'un de l'autre, sont des hydrogènes ou des groupes alkyle, alcényle, alcynyle, aryle, benzyle, alcoxy, alcényloxy, alcynyloxy ou phénoxy, et R⁸ a les significations données ci-dessus, en a) à k), pour R, et
R¹ est un hydrogène ou un radical alkyle, alcényle, alcynyle, cycloalkyle, cycloalcényle, où ces 4 derniers groupes sont non substitués ou sont une ou plusieurs fois substitués par des substituants choisis dans le groupe comprenant les radicaux phényle, hydroxy, alcoxy, alcényloxy, alcynyloxy, benzyloxy, phényloxy, cycloalkyloxy, alkylthio, mono- et dialkylamino, cyano, halogéno et nitro,
et les auxiliaires usuels de formulation, pour protéger les plantes de culture contre les effets secondaires phytotoxiques des herbicides.

2. Utilisation selon la revendication 1, dans laquelle, dans la formule (I)
R est
a) un groupe hydroxy ou mercapto,
b) un groupe alcoxy en C₁-C₁₀, alcényloxy en C₂-C₄, alcynyloxy en C₂-C₄, alkylthio en C₁-C₄, alcénylthio en C₂-C₄, alcynylthio en C₂-C₄, cycloalcoxy en C₃-C₈ ou cycloalkylthio en C₃-C₈, dont chacun est non substitué ou est une ou plusieurs fois substitué par des substituants du groupe comprenant les radicaux phényle, alcoxy en C₁-C₄, alcényloxy en C₂-C₄, alcynyloxy en C₂-C₄, benzyloxy, phényloxy, cycloalkyloxy en C₃-C₈, alkylthio en C₁-C₄, mono- et di-(alkyle en C₁-C₄)amino, cyano, halogéno et NO₂,
c) un groupe phényloxy, phénylthio, benzyloxy ou benzylthio, dont chacun est non substitué ou est substitué par 1 à 3 substituants choisis dans le groupe comprenant les radicaux alkyle en C₁-C₄, alcényle en C₂-C₄, alcynyle en C₂-C₄, halogéno, cyano, NO₂, alcoxy en C₁-C₄, alcényloxy en C₂-C₄, alcynyloxy en C₂-C₄, alkylthio en C₁-C₄, mono- et di-(alkyle en C₁-C₄)amino, phényloxy et benzyloxy,
d) un radical de formule -NR'R', où R' est un groupe alkyle en C₁-C₄,
e) un radical pyridino, morpholino, diméthylmorpholino ou hydrazino,
f) un radical de formule 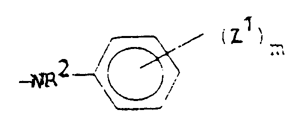 dans laquelle R² est H ou un groupe alkyle en C₁-C₄, les radicaux Z¹, indépendamment les uns des autres, sont des groupes halogéno, nitro, alkyle en C₁-C₄, alcoxy en C₁-C₄ ou phénoxy, et m vaut de 0 à 3,
g) un radical de formule -O-N=CR³R³, où les radicaux R³ sont des groupes alkyle en C₁-C₄, en particulier le groupe méthyle, ou encore, avec l'atome de carbone qui les relie, représentent le groupe cyclohexyle ou cyclopentyle,
h) un radical de formule -O-CR⁴R⁴-CO-R⁵, dans laquelle les radicaux R⁴, indépendamment l'un de l'autre, sont chacun H, ou des groupes alkyle en C₁-C₄, alcényle en C₂-C₄, alcynyle en C₂-C₄, phényle, benzyle ou alcoxy en C₁-C₄, et R⁵ est un groupe alkyle en C₁-C₄, alcényle en C₂-C₄, alcynyle en C₂-C₄, phényle ou benzyle,
i) un radical de formule -NH-N=CR⁶R⁶, dans laquelle les radicaux R⁶, indépendamment l'un de l'autre, sont chacun H, ou des radicaux alkyle en C₁-C₄ ou phényle, ou encore, avec l'atome de carbone qui les relie, représentent un groupe cyclohexyle ou cyclopentyle, ou bien
k) un radical de formule -O-CR⁷R⁷-CO-R⁸, dans laquelle les radicaux R⁷, indépendamment l'un de l'autre, sont H ou des groupes alkyle en C₁-C₄, alcényle en C₂-C₄, alcynyle en C₂-C₄, phényle, benzyle ou alcoxy en C₁-C₄, et R⁸ a les significations données ci-dessus en a) à i) pour R, et
R¹ est un groupe alkyle en C₁-C₁₈, alcényle en C₁-C₁₈, alcynyle en C₁-C₁₈, cycloalkyle ou cycloalcényle, où ces 4 derniers groupes sont non substitués ou sont une ou plusieurs fois substitués par des substituants choisis dans le groupe comprenant les radicaux phényle, alcoxy en C₁-C₄, alcényloxy en C₁-C₄, alkyle en C₁-C₄, alcynyloxy en C₁-C₄, benzyloxy, phényloxy, cycloalkyloxy, alkylthio, mono- et dialkylamino, cyano, halogéno et NO².

3. Utilisation selon la revendication 1 ou 2, dans laquelle, dans la formule (I),
R est un radical alcoxy en C₁-C₄, hydrazino, diméthylamino ou benzylamino, et
R¹ est un radical alkyle en C₃-C₆, cyclohexyle, halogénocyclohexyle, cyclooctyle, éthényle, méthylhydrazino ou adamantyle.

4. Utilisation selon l'une des revendications 1 à 3, dans laquelle dans la formule (I), X est un atome d'oxygène.

5. Utilisation selon l'une des revendications 1 à 4, caractérisée en ce qu'elle contient en outre au moins une matière active herbicide.

6. Utilisation selon la revendication 5, caractérisée en ce que les matières actives herbicides appartiennent à la classe des carbamates, des thiocarbamates, des halogén-acétanilides, des dérivés substitués des acides phénoxy-, naphtoxy- et phénoxy-phénoxycarboxyliques, ainsi que des dérivés des acides hétéroaryloxy-phénoxyalcane-carboxyliques, des dérivés de la cyclohexanedione, des imidazolinones et des sulfonylurées.

7. Procédé de préparation de composés de formule (I) tels que définis dans les revendications 1 à 4, ainsi que de leurs sels, à l'exception des composés de formule (I) dans laquelle
a) X est un oxygène, X¹ = R¹, X² = COR et
R = OCH₃ et R¹ = n-C₆H₁₃, n-C₈H₁₇ ou n-C₁₂H₂₅ ou
R = OC₂H₅ et R¹ = H, CH₃, n-C₄H₉, n-C₆H₁₃, CH=CH₂, CH₂CH=CH₂, C(CH₃)=CH₂ ou C≡CH ou
R = OH et R¹ = H ou CH₃ ou
R = phénylamino ou p-chloro-phénylamino et R¹ = n-C₁₂H₂₅, ou encore dans laquelle
b) X est un oxygène, X¹ = COR, X² = R¹ et
R = OCH₃ et R¹ = H, CH₃, C₂H₅ ou t-C₄H₉ ou
R = OC₂H₅ et R¹ = alkyle en C₁-C₇,
caractérisé en ce que a) dans le cas dans lequel X¹ = R¹ et X² = COR, on fait réagir un composé de formule (II)
H₂C=CH-R¹ (II)
avec un composé de formule (III) 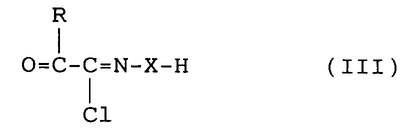 où, dans les formules (II) et (III), X , R et R¹ ont les significations données pour la formule (I), et en ce que b) dans le cas dans lequel X¹ = COR et X² = R¹, on fait réagir un composé de formule (IV) 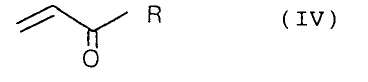 avec un composé de formule (V) 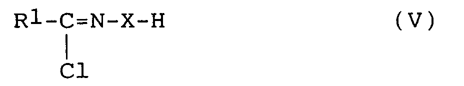 où dans les formules (IV) et (V), X, R, R¹ ont les significations données dans pour la formule (I), le composé obtenu en a) ou b) étant éventuellement converti en son sel.

8. Procédé pour maîtriser des végétaux indésirables dans des cultures de plantes utiles, caractérisé en ce qu'on applique sur les végétaux, les parties de végétaux, les semences ou la surface de culture, au moins une matière active herbicide en combinaison avec au moins un composé de formule (I) défini dans l'une des revendications 1 à 4.

9. Procédé de préparation d'un agent de protection des plantes de culture, caractérisé en ce qu'on convertit en une forme d'application convenant à la protection des végétaux, d'une manière analogue à des procédés usuels, au moins un composé de formule (I) tel que défini dans l'une des revendications 1 à 4, éventuellement en même temps qu'au moins une matière active herbicide, et des adjuvants de formulation.
